# EUROPEAN PATENT APPLICATION

(11) **EP 0 002 210 A1**
(43) Date of publication of application: **13.06.1979**
(21) Application number: 78101388.3
(22) Date of filing: 17.11.1978
(51) Int. Cl.: C07D 499/00, A61K 31/43, C07D 205/08, C07F 9/65

(54) **6- and 6,6- disubstituted-2-substituted-pen-2-em-3-carboxylic acids; processes for their preparation and pharmaceutical compositions containing such compounds**

(30) Priority: 17.11.1977 US 852427; 17.11.1977 US 852275; 17.11.1977 US 852278; 17.11.1977 US 852274; 10.10.1978 US 948711; 10.10.1978 US 948713; 10.10.1978 US 948712
(71) Applicant: Merck & Co., Inc., Rahway New Jersey 07065-0900 (US)
(72) Inventor: Christensen, Burton Grant, Metuchen N.J. 08840 (US); Di Ninno, Frank Paul, Old Bridge N.J. 08857 (US)
(74) Representative: Abitz, Walter, Dr.-Ing.

(57) **Abstract**

Disclosed are 6- and 6.6-disubstituted-2-substituted-pen-2-em-3-carboxylic acids of the following structure: 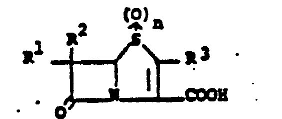 wherein: R¹ and R² are independently selected from hydrogen, -NHR^{1'} (R^{1'} is H or acyl), alkyl, alkoxyl. aralkyl, aryl. heterocyclyl and heterocycfyl-alkyl: R³ is selected from hydrogen, -R, -OR, -SR. -NR₂; R is substituted and unsubstituted alkyl, aryl, aralkyl, hetero-heterocyclyl, or heterocyclylalkyl; n is 0 or 1: when n=1, R³ is not -SR. Such compounds and their pharmaceutically acceptable salt, and ester derivatives are useful as antibiotics. Also disclosed are processes for the preparation of such compounds, pharmaceutical compositions comprising such compounds and methods of treatment comprising administering such compounds and compositions when an antibiotic effect is indicated.

## Description

### BACKGROUND OF THE INVENTION

This invention relates to 6- and 6,6-disubstituted-2-substituted-pen-2-em-3-carboxylic acids and their pharmaceutically acceptable salts and esters, which compounds are useful as antibiotics and which may be represented by the following generic structural formula (I): 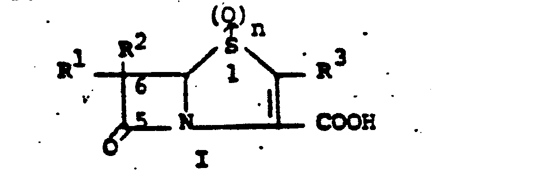 wherein: R¹ and R² are, inter alia, independently selected from hydrogen, substituted and unsubstituted: alkyl, alkoxyl, -NHR^{1'} (R^{1'} is Y or acyl), aryl, aralkyl, heterocyclyl and heterocyclylalkyl; and ^{R3} is, inter alia, selected from hydrogen, -R, -OR, -SR, -NR₂; R is substituted and unsubstituted: alkyl, aryl, aralkyl, heterocyclyl and heterocyclylalkylt n is 0 or 1; when n-1, R3 is not -SR; wherein the substituents on R¹, R² or R³ is not -SR; wherein the substituents on R¹ , R or R are selected from amino, mono-, di- and trialkylamino, hydroxyl, alkoxyl, mercapto, alkylthio, arylthio, such as phenylthio, fulfanoyl, amidino, guanidino, nitro, chloro, bromo, flyoro, cyano and carboxyl.

This invention also relates to processes for the preparation of such compounds (1); pharmaceutical compositions comprising such compounds; and to methods of treatment comprising administering such compounds and compositions when an antibiotic effect is indicated.

There is a continuing need for new antibiotics. For unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly, the search for new antibiotics continues.

Thus, it is an object of the present invention to provide a novel class of antibiotics which are useful in animal and human therapy and in inanimate systems. These antibiotics are active against a broad range of pathogens which representatively include both gram positive bacteria such as S, aureus, Strop. pyogenes, and B. subtilis, aud gram negative bacteria such as E. coli, Pseudomonas, Proteus morganii, Serratia and Klebsiella. Further objects of this invention are to provide chemical processes for the preparation of such antibiotics and their non-toxic pharmaceutically acceptable salts; pharmaceutical compositions comprising such antibiotics; and to provide mathods of treatment comprising administering such antibiotics and compositions when an antibiotic effect is indicated.

### DETAILED DESCRIPTION OF THE INVENTION

The compounds of the present invention (I, above) are conveniently prepared by the following schemes:

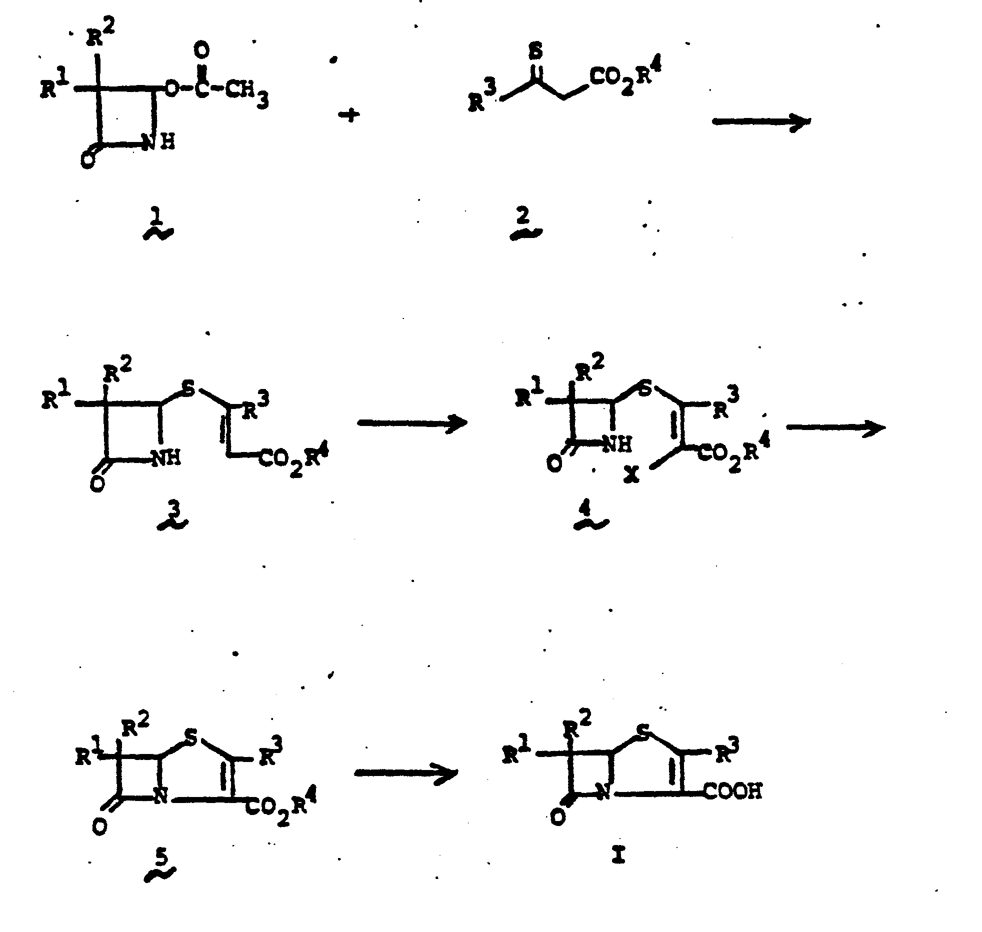

In words relative to the above diagram, the starting azetidinone 1 is treated with a 3-thionopropionic acid ester derivative (2) in the presence of base such as NaOCH₃, aluminum isopropbxide, Al[OCH (CH₃)₂]₃, triethglamine and the like in a solvent such as methanol, tetrahydrofuran (THF), CH₂Cl₂ and the like at a tamperature of from 0 to 22°C for from 1 hour to overnight to provide the seco- lactam 3. R is a readily removable carboxyl blocking group such as p-nitrobenzyl, o-aitrobenzyl, 2,2,2-trichloroethyl, t-butyldimathylsilyl or the like. Halogenation of 3 yields 4 wherein X is halo, such as chloro or bromo. Suitable halogenating agents include N-chlorosuccinimide, N-bromosuccinimide, N-bromoacetamide and the like: and the reaction 3→4 is conducted in the presence of a halogenating agent of choice in a solvent such as THF, benzene, benzene/ether and the like at a temperature of from -78° to 60°C for from 0.5 to 2 hours. Cyclization of 4 to provide 5 is accomplished by treating 4 with a strong base such as lithium diisopropylamide, lithium hexamethylsilazide, lithium tetramethylpiperidide or the like in a solvent such as THF, hexamethylphosphoramide, dimethoxyethane or the like in the presence of a metal complex coupling agent at a temperature of from -78 to 22°C for from 0.5 to 18 hours; suitable metals include . Cu (I), the platinum metals such as Pd°, Ni°(II) and the like: representative complsxes include cuprous iodide, cuprous bromide-dimethyl sulfide complex, cuprous iodide-tri-n-butylphosphine complex tetrakistriphenylphosphine Pd (0), tetrakistriphenylphosphine Ni(0), bis- cyclooctadienyl Ni(0), bis (diphenylphosphino)ethane Ni(II)· - C1₂ or the like at a temperature of from -78° to 22°C for from 0.5 to 18 hours. The carboxyl protected intermediate 5 is deblocked to provide I. When the preferred blocking groups, R⁴ are employed such as p-nitrobenzyl or trichloroethyl, the deblocking reaction may be accomplished by hydrOgenation or zinc mediated reduction according to well-known procedures. A representative deblocking procedure comprises treating 5 in a solvent such as ethylacetate under hydrogen (1-40 atmospheres) at a temperature of 0° to 25°C for from 0.25 to 2 hours in the presence of a hydrogenation catalyst such as 10% Pd/C, 5%/Pd BaC0₃₁ 5% Pt/C or the like.

The starting azetidinone material 1 may conveniently be prepared by the following scheme: 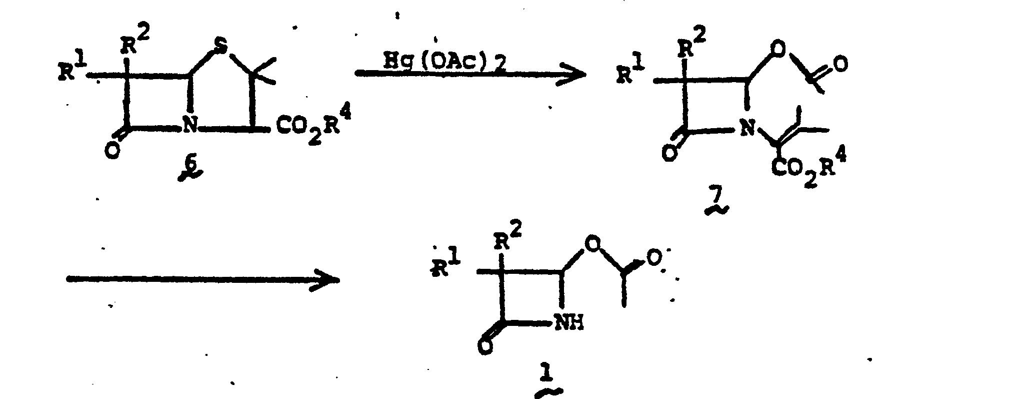 wherein R¹ and _{R}2 are as defined above and _{R}⁴ is any well-known carboxyl protecting group such as benzyl, methyl, trichloroethyl or the like; the preparation of starting material 6 is given below in the Examples Section.

In words relative to the above diagram, starting material 6 is cleaved with mercuric acetate (Hg(CAc)₂) in acetic acid solution at a temperature of from 22°C to 110°C for from 0.25 to 8 hours to provide the acetoxy lactam 7. Relative to these reactions, R⁴ is a readily removable carboxyl protecting group such as methyl, benzyl, trichloroethyl or the like. Removal of the isopropylidene ester function is accomplished by treating 7 with potassium permanganate, osmium tetroxide or the like in a solvent such as aqueous pyridine, aqueous acetone or the like at a temperature of 0° to 22°C for from 0.25 to 2 hours, to provide azetidinone 1. Analogous procedures are known in the literature; see, for example: E. G. Brain, et al., J. Chem. Soc., Perkin I, 447 (1976); R. J..Stoodley and N. R. Whitehouse, Ibid., 32 (1973).

Starting material 2 in the above-described synthesis, may conveniently be prepared in a variety of ways. One preferred method, when the ultimate 2-substituent, R³, (Structure I) is -SR, is shown in the following scheme: 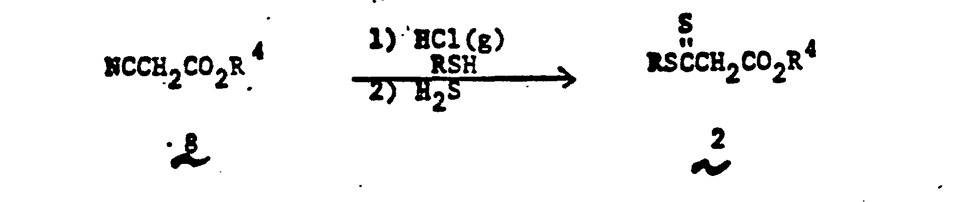 In words relative to the above diagram, the cyanoacetic acid ester 8 is treated with gaseous hydrogen chloride in a solvent such as benzene, diethylether, tetrahydrofuran or the like in the presence of a mercaptan (RSH) at a temperature of from 0°C to 25°C for from 0.25 to 1 hour. The resulting mixture is stirred at a temperature of from 0° to 80°C for from 8 to 96 hours. The precipitate formed is collected by filtration and is dissolved in anhydrous dimethylsulfoxide or dimethylformamide or the like. The mixture is treated with gaseous hydrogen sulfide at a temperature of from 0°C to 25°C for from 10 min. to 24 hours and is stirred further at a temperature of from 0°C to 25°C for from 2 to 24 hours to provide 2. Relative to these reactions R and R ² are as previously defined.

The following list representatively illustrates suitable starting materials 2. Such reagents are employed as described in the above procedure to provide species bearing a preferred substituent at the 2-position.

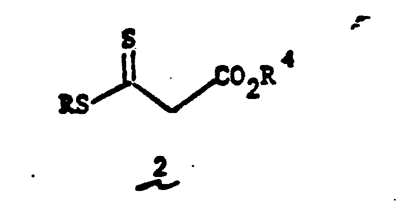

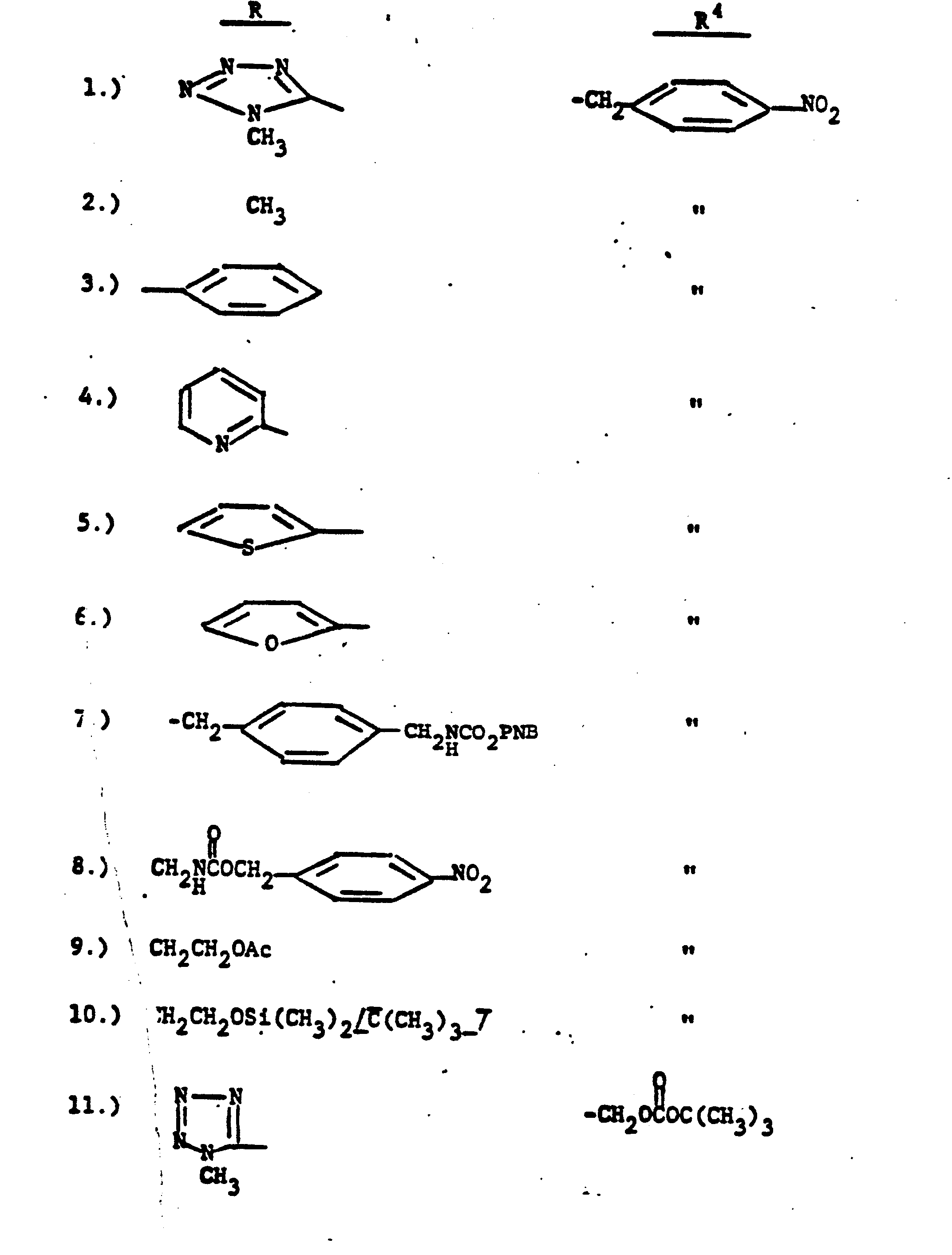

When starting material 2, in the above-described synthesis, provides the 2-substituent as -R³, it may be prepared by known literature procedures: F. Duus, et al., Ark. Kemi, 29, 191 (1968); Chem. Abs., 69:31404p (1968). A schematic summary of such a convenient process is given below: 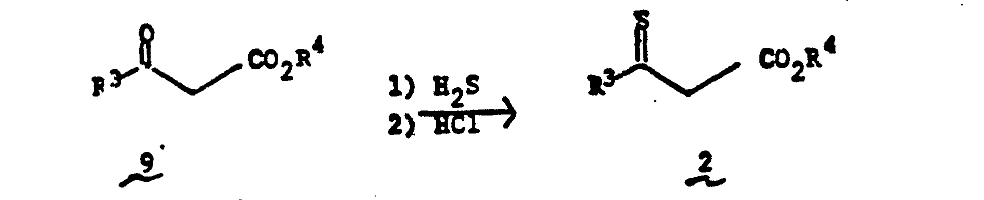 In words relative to the above diagram, the readily available substituted 3-ketoproprionic acid ester derivatives 9 is treated with hydrogen sulfide gas for from 1 to 3 hours in a solvent such as acetonitrile, dimethylformamide, dimethylsulfoxide or the like at a temperature of from -60° to 25°C. The mixture is then treated with gaseous hydrogen chloride for from 0.5 to 2 hours at a temperature of from -60° to 25°C to provide starting material 2. Relative to this reaction, R³ and R⁴ are as previously described.

The following list representatively illustrates suitable starting materials 2. Such reagents are employed as described in the above procedure to provide species bearing a preferred substituent, R³, at the 2-position.

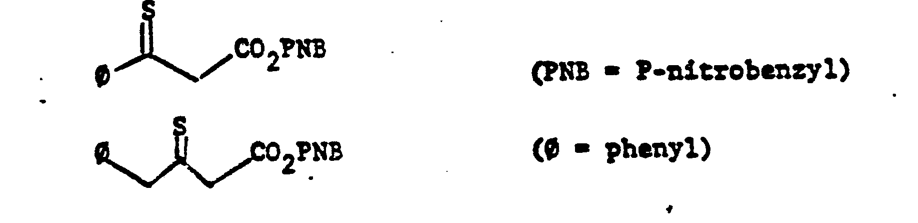

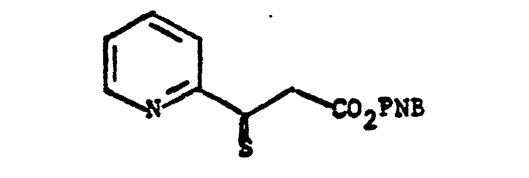

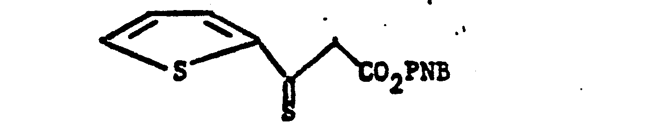

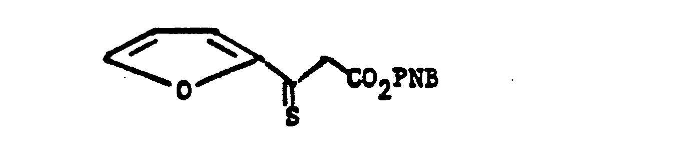

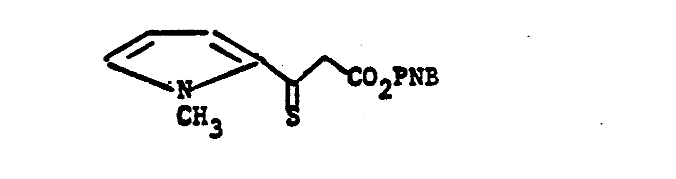

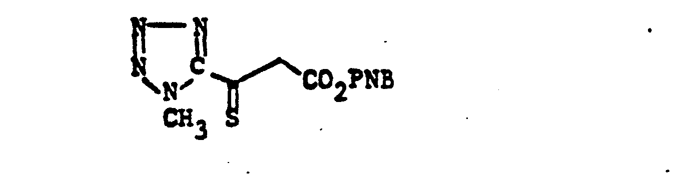

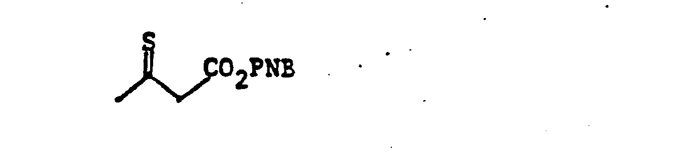

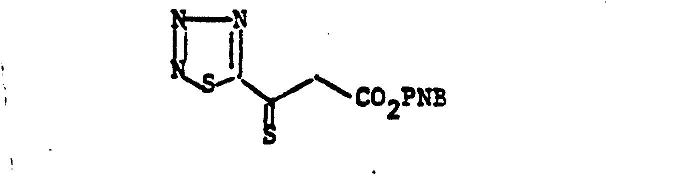

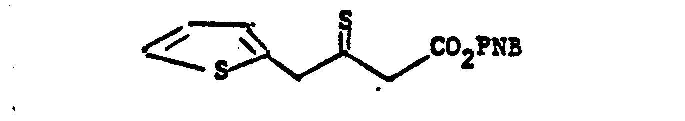

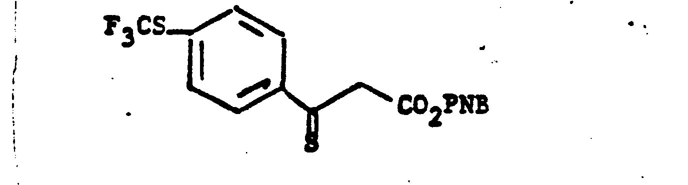

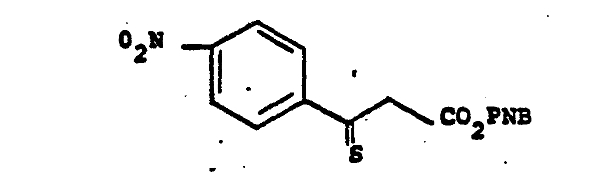

The compounds of the present invention (I, above) when R¹ is -NHR^{1'} are conveniently prepared by the following scheme:

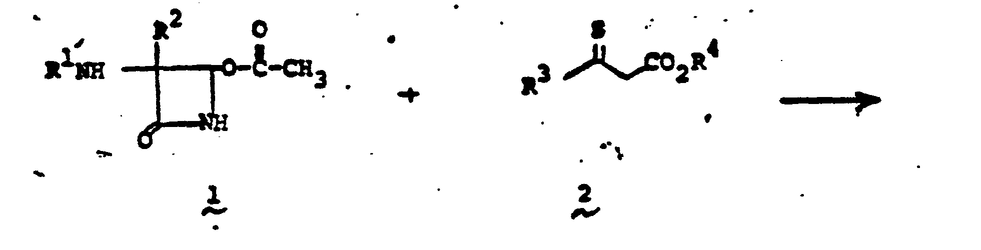

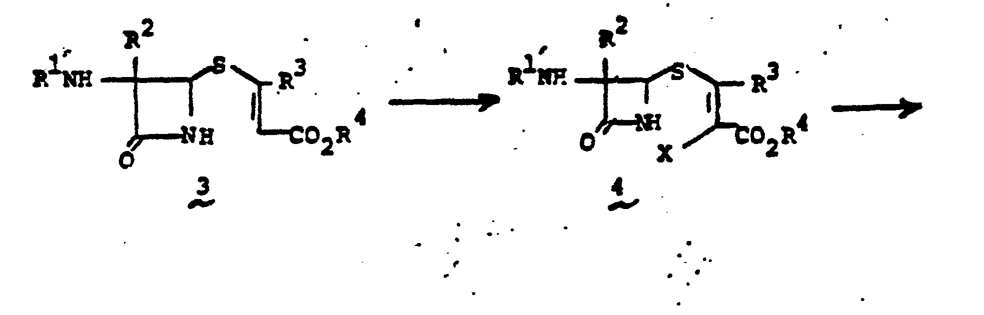

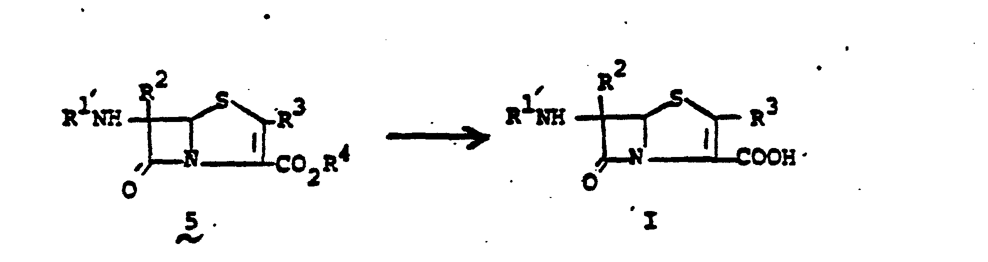

In words relative to the above diagram, the starting azetidinone 1 is treated with.a 3-thionopropionic acid ester derivative (2) in the presence of a base such as NaOCH₃,'Al[OCH(CH₃)₂]₃, (CH₃CH₂)₃N and the like in a solvent such as methanol, tetrahydrofuran (THF), CH₂C1₂ and the.like at a tamperature of from, 0° to 22°C for from 1 hour to overnight to provide the seco-lactam (3). Relative to these reactions, R⁴ is a readily removable carboxyl blocking group such as p-nitrobenzyl, t-butyl, trichloroethyl or the like; and R¹. R and R are as defined above. Halogenation of 3 yields 4 wherein X is halo, such as chloro or bromo. Suitable halogenating agents include N-chlorosuccinimide, N-bromosuccinimide, N-bromoacetamide and the like; and the reaction 3→4 is conducted in the presence of the balogenating agent of choice in a solvent such as THF, benzene, benzene-ether and the like at a temperature of from -78° to 60°C. for from 0.5 to 2 hours. Cyclisation of 4 to provide 5 is accomplished by treatiag 4 with a strong base such as lithiumdiisopropylamide, lithiumhexamethyldisilamide, lithiumtetramethylpiperidide or the like in a solvent such as tetrahydrofuran, hexamethylphosphoramide, dimethoxyethane or the like in the presence of a metal complex coupling agent at a temperature of from -78 to 22°C for from 0.5 to 18 hours; suitable metals include Cu (I), the platinum metals such as Pd°; Ni°, Ni(II) and the like: representative complexes include cuprous iodide, cuprous bromide-dimethylsulfide complex, cuprous iodide-tri-n-butylphosphine complex, tetrakistriphenyl phosphine Pd(O), tetrakistriphenyl phosphine Ni(O), bis- cyclooctadienyl Ni(O), bis(digherrylphosphino)-ethane Ni(II)·Cl₂ or the like. The fully protected intermediate 5 is deblocked to provide I. When the preferzed blocking groups are employed, that is, R⁴ is p-nitrobenzyl or trichloroethyl, the deblocking reaction may be accomplished by hydrogenation or zinc mediated reduction according to well-known procedures. A representative deblocking procedure comprises trsating 5 in a solvent such a. ethylacetate under hydrogen (1-40 atmospheres) at a temperature of 0° to 22°C for from 0.25 to 2 hours in the presence of a hydrogenation catalyst such as 10%Pd/C, 5%Pd/BaCo₃, 5%Pt/C or the like.

The starting azetidinone material 1 may conveniently be prepared by the following scheme: 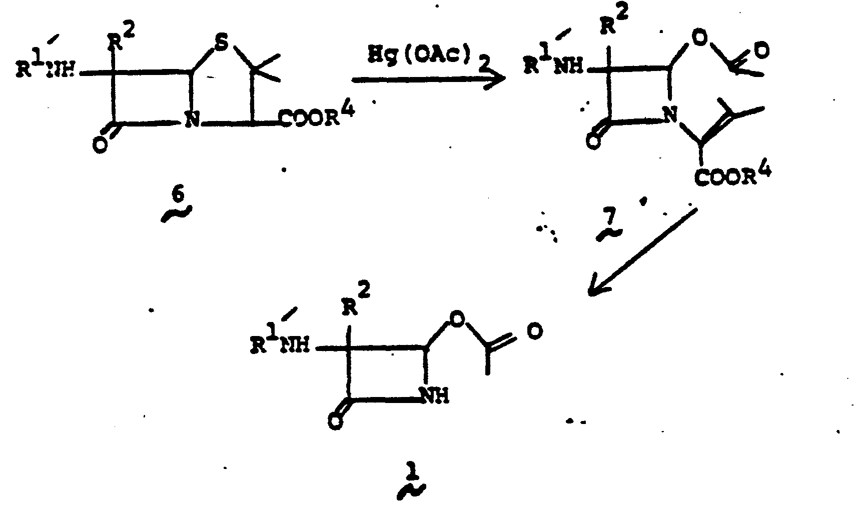 wherein _{R}¹, R² and _{R}⁴ are as defined above.

In words relative to the above diagram, starting material 6 is cleaved with mercuric acetate in acetic acid solution at a temperature of from 22°C to 110°C for from 0.25 hours to 8 hours to provide the acetoxy lactam 7. Relative to these reactioas, R⁴ is a selected carboxyl protecting group such as methyl, benzyl, trichloroethyl or the like. Removal of the isopropylidene ester function is accomplished by treating 7 with potassium permanganate, osmium tetroxide or the like in a solvent such as aqueous pyridine, aqueous acetone or the like at a temperature of 0° to 22°C for from 0.25 to 2 hours, to provide azetidinone 1. Analgous procedures are known in the literature; see, for example: E.G. Brain; et al., J. Chem. Soc., Perkin I, 447 (1976); R.J. Stoodley and N.R. Whitehouse, ibid., 32 (1973).

An alternative procedure, which is especially suitable for embodiments wherein R³ is H or R is ilₗus- trated below: 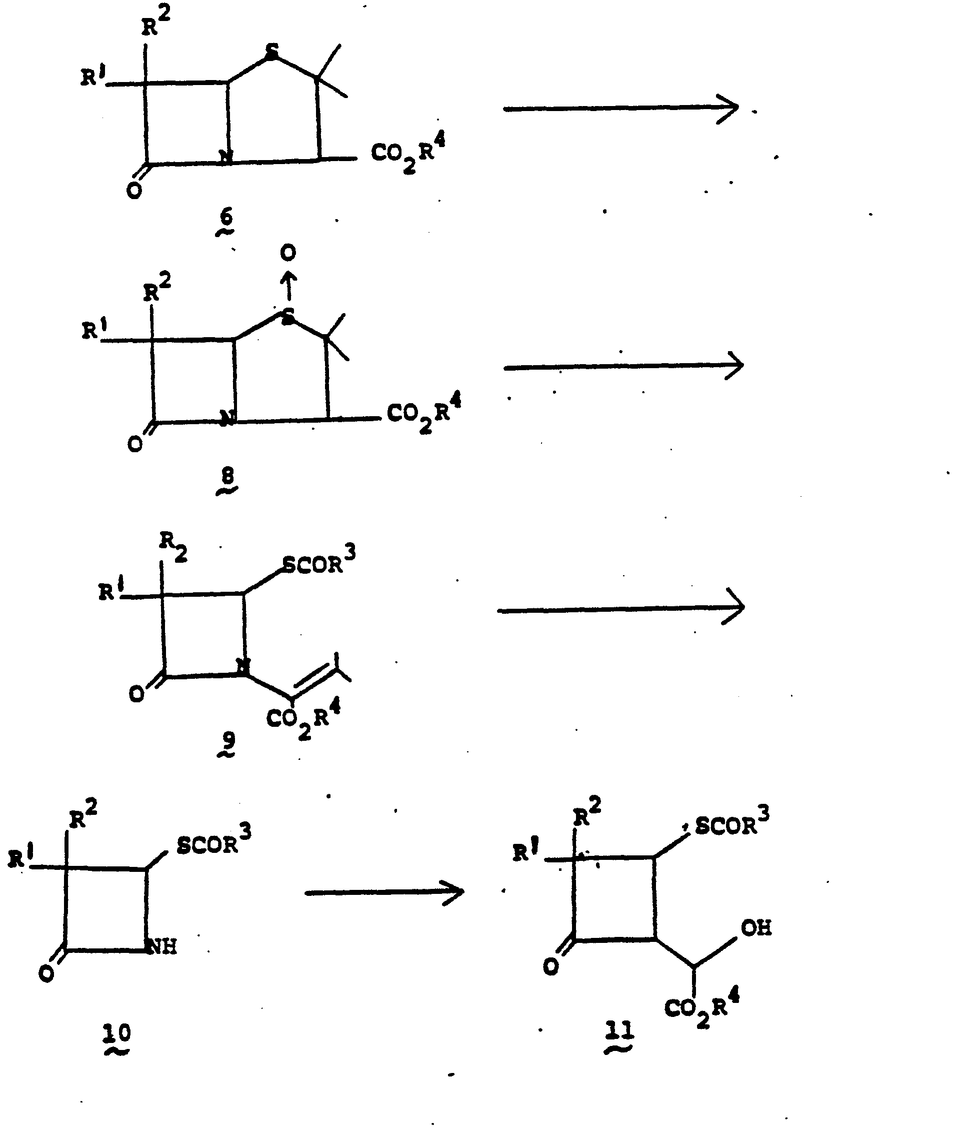 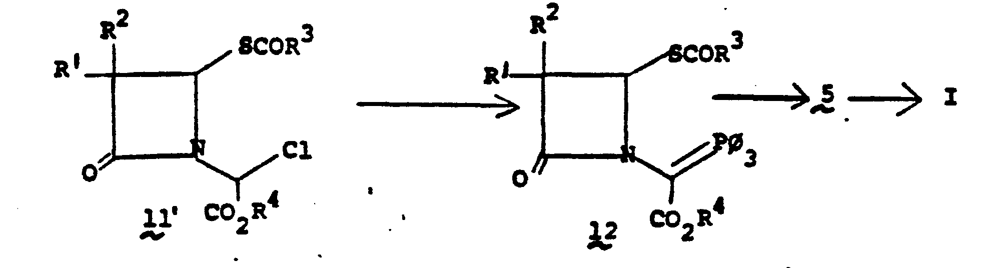 wherein all symbolism is as previously defined; R⁴ is aryl such as phenyl and substituted phenyl; Ø is phenyl.

In word relative to the above reaction diagram, above-defined starting material 6 is taken through the scheme 6→ →12 whereupon 12 is converted to above-defined species 5 which is deblocked as previously described to yield I.. The oxidation reaction 6→8 is known and is conveniently accomplished by treating 6 in a solvent such as methylsne chloride, chloroform, benzene, tetrahydrofuran or the like at a temperature of from 0° - 25°C with a stoichiosnetric amount of an oxidizing agent such as m-chloroperbenzoic acid, perbenzoic acid, H₂0₂, peracetic acid or the like. The resulting sulfoxide 8, after isolation by conventional work-up, in a solvent such as benzene, toluene, dioxane, or the like is treated with a stoichiometric to slight excess of tri-n-butylphosphine and a stoichiometric to ten-fold excess of the anhydride (R³CO)₂0 (for example, substituted or unsubstituted benzoic anhydride). The mixture is typically heated under nitrogen for from 0.5 to 10 hours at a temperature of from 50°C to reflux to yield 9, which may be isolated by conventional work-up. In cases where the isomerization of the β,Y-unsaturated ester to the α,β-unsaturated ester does not occur under the reaction conditions or during work-up, the product is conveniently isomerized to the latter (as in 9) with triethylamine. Cleavage of 9 to provide 10 is typically accomplished by treating 9, is a solvent such as 8:1 acetone-water, aqueous pyridine, or the like with an equivalent to three-fold excess amount if an oxidizing agent such as potassium permanganate, OsO₄, sodium periodate or the like at a temperature of from 0° to 25^{*}C for from 5 min. to 2.0 hr., after which the reaction is quenched and 10 isolated by conventional work-up. The reaction 10→ 11 is accomplished by treating 10 with an excess of glyoxalate ester such as p-nitrobenzylglyoxalate hydrate. The reaction is conveniently carried out in a solvent such as benzene, toluene, xylene or the like at a temperature of from about 25°C to reflux under a nitrogen atmosphere for from about 2 to 10 hours. The reaction 11→11'→12 may be conducted stepwise.

The halogenation reaction 11→ 11' may be conducted by any of a variety of well-known halogenation means. Suitable reagents include: SOCl₂, POCl₃, oxalyl chloride and the like. A preferred means of chlorination involves treating 2 in a solvent such aa tetrahydrofuran (THF), ether, CH₂Cl₂ and the like with thionylchloride in the presence of 1 to 2 equivalents (relative to the thionylchloride) of a base such as pyridine, triethylamine, quinoline and the like. Typically, the reaction is conducted at a temperature of from -30 to 25°C for from 0.5 to 1 hr. The resulting species, 11', is isolated, if desired, by conventional procedures for later reaction, 11→ 12 The intermediate 12 is prepared from 11' by treating 11' in a solvent such as dimethylformamide (DMF), dimethylsulfoxide (DMSO), THF, dimethoxyethane (DME) and the like with 1 to 1.5 equivalents of a phosphine such as triphenylphosphine, tributylphosphine, triethylphosphine, tris-(2-cyanoethyl)-phosphine or the like. Typically, the reaction is conducted under a nitrogen atmosphere at a temperature of from -20 to 25°C, for from 0.5 to 2 hrs. The closure reaction 12→ 5 is accomplished by heating the phosphoraneazetidinone 12 in a solvent such as benzene, toluene droxane, xylene, DMF or the like at a temperature of from 20 to 100°C for from 0.2.5 hrs. to 4 days. The resulting penem is deblocked to yield I according to the previously described procedure.

In the generic representation of the compounds of the present invention (I): 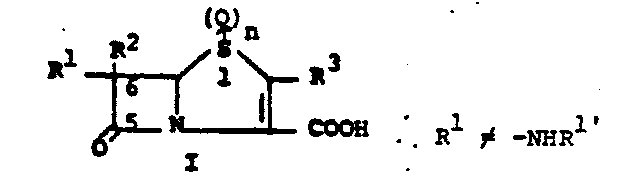 The preferred values for R are: hydrogen; substituted and unsubstituted: -OR, -SR, -NR₂ (R is defined herein), alkyl having 1-6 carbon atoms, aryl such as phenyl, aralkyl wherein the aryl moiety is preferably phenyl and the alkyl has 1-6 carbon atoms such as benzyl,phenethyl and the like, heterocycyl or heterocyclyalkyl wherein the alkyl has 1-3 carbon atoms and the heterocyclic ring comprises 4-6 atoms, up to 4 of which may be selected from oxygen, sulfur and nitrogen; and wherein the chain or nuclear substituent on R is selected from: amino, mono-, di- and trialkylamino, hydroxyl, alkoxyl, mercapto, alkylthio, arylthio such as phenylthio, sulfamoyl, amidino, guanidino, nitro, chloro, bromo, fluoro, cyano and carboxyl; the alkyl moieties of the above-recited substituents have 1-6 carbon atoms; n=0 is preferred.

The preferred ester moieties, R⁴, (see 5, above) used as carboxyl protecting groups are those selected from benzyl, p-nitrobenzyl, o-nitrobenzyl, t-butyl, bromo-t-butyl, t-butyl-dimethylsilyl, trimethylsilyl, trichloroethyl: R⁴ may also represent, in addition to hydrogen, pharmaceutically acceptable ester moieties such as pivaloyloxymethyl, allyl, methallyl, (2-methylthi_{Q})-ethyl, or 3-buten-1-yl; and pharmaceutically acceptable salt cations.

R¹ and R are independently selected from the group consisting of hydrogen, substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the linear chain has 1-6 carbon atoms; heteroaryl, heteroaralkyl, heterocyclyl and heterocyclylalkyl wherein the substituent or substituents relative to the above-named radicals are selected from the group consisting of: amino, mono-, di- and trialkylamino, hydroxyl, alkoxyl, mercapto, alkylthio, arylthio such as phenylthio, sulfamoyl, amidino, guanidino, nitro, chloro, bromo, fluoro, cyano and carboxy, and wherein the hetero atom or atoms in the above-named heterocyclic moieties are selected from the group consisting of 1-4 oxygen, nitrogen or sulphur atoms; and wherein the alkyl moieties of the above-recited substituents have 1-6 carbon atoms.

The most preferred embodiments of the present invention (I) are those wherein R¹ is hydrogen and R² is hydroxymethyl, 1'-hydroxy-3-phenyl-propyl, l'-hydroxy-3-phenyl-3-carboxypropyl, 1'-hydroxy-2',2',2'-trifluoroethyl; R¹ is methoxyl, and R² is all of above; R¹ and _{R}² are hydrogen; R³ is hydrogen, phenyl, methyl, 2'-thienyl, 2'-pyridyl, benzyl, p-methoxyphenyl, p-aminomethylphenyl or SR wherein R is as described above and is especially: aminomethyl and aminoethyl.

For 6-amido embodiments of _{I} (R¹ = NHR^{1'}) : 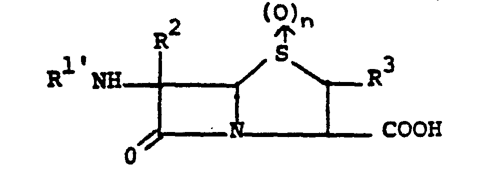 the values for R², n and R³ are as defined above; and the acyl radical represented by R¹ can be substituted or unsubstituted aliphatic, aromatic or heterocyclic, araliphatic or heterocyclylaliphatic carboxylic acid radical, a substituted or unsubstituted carbamyl radical or a carbothioic acid radical. One group of acyl radicals can be represented by the general formula: 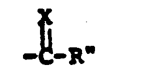 wherein X is O or S and R" represents hydrogen; amino; substituted amino such as alkyl- and dialkylamino wherein the alkyl radical comprises 1 to about 6 carbon atoms; substituted or unsubstituted: straight or branched chain alkyl wherein the alkyl radical comprises 1 to about 6 carbon atoms; mercapto such as alkylthio, typically comprising 1 to 6 carbon atoms; arylthio, typically comprising 6 to 10 carbon atoms; hydroxy such as alkoxy, typically comprising 1 to 6 carbon atoms; aryloxy, typically comprising 6 to 10 carbon atoms; alkenyl, or alkynyl groups typically comprising 2 to 6 carbon atoms; aryl such as phenyl; aralkyl such as benzyl; cycloalkyl, typically comprising 3 to 6 carbon atoms; or a heteroaryl or heteroaralkyl group (mono- and bicyclic) wherein the alkyl moiety typically comprises 1 to 3 carbon atoms and the heterocyclic ring comprises typically 4 to 10 atoms and the hetero atom or atoms are selected from 0, N and S; such above-listed groups can be unsubstituted or can be substituted by radicals such as OH, SH, SR (R is loweralkyl or aryl such as phenyl), alkyl or alkoxy groups having 1 to about 6 carbon atoms, halo, such as Cl, Br, F and I, cyano, carboxy, sulfamino, carbamoyl, sulfonyl, azido, amino, substituted amino such as alkylamino including quaternary ammonium wherein the alkyl group comprises 1 to 6 carbon atoms, haloalkyl such as trifluoromethyl, carboxyalkyl, carbamoylalkyl, N-substituted carbamoylalkyl, wherein the alkyl moiety of the foregoing four radicals comprises 1 to about 6 carbon atoms, amidino, guanidino, N-substitutad guanidino, guanidino lower alkyl and the like. Representative examples of such acyl groups that might be mentioned are those wherein R" is benzyl, p-hydroxybenzyl, 4-amino-4-carboxybutyl, methyl, cyanomethyl, 2-pentenyl, n-amyl, n-heptyl, ethyl, 3- or 4-nitrobenzyl, phenethyl, β,β- diphenylethyl, methyldiphenylmethyl, triphenylmethyl, 2-methoxyphenyl, 2,6-dimethoxyphenyl, 2,4,6-trimethoxyphenyl, 3,5-dimethyl-4-isoxazolyl, 3-butyl-S-methyl-4-isoxazolyl, 5-methyl-3-phenyl-4-isoxazolyl, 3-(2-chlorophenyl)-5-methyl-4-isoxazolyl, 3-(2,6-dichlorophenyl)5-methyl-4-isoxazolyl, D-4-amino-4-carboxybutyl, D-4-N-benzoylamiao-4-carboxy-n-butyl, p-aminobenzyl, o-aminobenzyl, m-aminobenzyl, p-dimethylaminobenzyl, (3-pyridyl)methyl, 2-ethoxy-1-napthyl, 3-carboxy-2-quinoxalinyl, 3-(2,6-dichloroptienyl)-5-(2-furyl)-4-isoxazolyl, 3-phenyl-4-isoxazolyl, 5-methyl-3-(4-guanidinophenyl)-4-isoxasolyl, 4-guanidinomethyl- phenyl, 4-guanidinomethylbenzyl, 4-guanidinobenzyl, 4- guanidinophenyl, 2,6-dimethoxy-4-guanidino, o-sulfobenzyl, p-carboxymethylbenzyl, p-carbamoylmethylbenzyl, m-fluorobenzyl, m-bromobenzyl, p-chlosobenzyl, p-methoxybenzyl, 1-napthylmethyl, 3-isothiazolylmethyl, 4-isothiazolylmethyl, 5-isothiazolylmethyl, guanyl- thiomethyl, 4-pyridylmethyl, 5-isoxazoiylmethyl, 4-methoxy-5-isoxazolylmethyl, 4-methyl-5-isoxazolylmethyl, 1-imidazolylmethyl, 2-benzofuranylmethyl,-2-indolylmethyl, 2-phenylvinyl, 2-phenylethynyl, 1-aminocyolo- hexyl, 2- and 3-thienylaminomethyl, 2-(5-nitrofuranyl) vinyl, phenyl, o-methoxyphenyl, o-chlorophenyl, o-phenylphenyl, p-aminomethylbsnzyl, 1-(5-cyanotrizolyl) methyl, difluoromethyl, dichloromethyl, dibromomethyl, 1-(3-methylimidazolyl)methyl, 2- or 3-(5-carboxymethyl- thianyl)methyl, 2- or 3-(4-carbamoylthienyl)-methyl, 2- or 3-(5-methylthienyl)methyl, 2- or 3-(methoxy- thienyl)methyl, 2- or 3-(4-chlorothienyl)-methyl, 2-or 3-(5-sulfothienyl)methyl, 2- or 3-(5-carboxythienyl) methyl, 3-(1,2,5-thiadiazolyl)methyl, 3-(4-methoxy-1,2,5-thiadiazolyl)methyl, 2-furylmethyl, -2-(5-nitrofuryl) methyl, 3-furylmethyl, 2-theinylmethyl, 3-thienylmethyl, tetrazolylmethyl, benzamidinomethyl and cyclohexylamidino- methyl.

The acyl group can also be a radical of the formula: 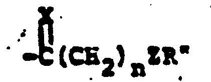 wherein X is 0 or S and n is 0-4, Z represents oxygen, sulfur, carbonyl or nitrogen and R" is defined as above. Representative members of the substituent 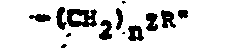 that might be mentioned are allylthiomethyl, phenylthiomethyl, butylmercaptomethyl, a-chlorocrotylmercaptomethyl, phenoxymethyl, phenoxyethyl, phenoxybutyl, phenoxybenzyl, diphenoxymethyl, dimethylmethoxymethyl, dimethylbutoxy- methyl, dimethylphenoxymethyl, 4-guanidinophenoxymethyl, 4-pyridylthiomethyl, p-(carboxymethyl)phenoxymethyl, p-(carboxymethyl)-phenylthiomethyl, 2-thiazolylthiomethyl, p-(sulfo)phenoxymethyl, p-(carboxymethyl)phenylthiomethyl, 2-pyrimidinylthiomethyl, phenethylthiomethyl, 1-(5,6,7,8-tetrahydronaphthyl)oxomethyl, N-methyl-4-pyridylthio, benzyloxy, methoxy, ethoxy, phenoxy, phenylthio, amino, methylamino, dimethylamino, pyridinium methyl, trimethylammonium-methyl, cyanomethylthiomethyl, trifluoromethylthiomethyl, 4-pyridylethyl, 4-pyridylpropyl, 4-pyridyl- butyl, 3-imidazolylethyl, 3-imidazolylpropyl, 3-imida- zolylbutyl, 1-pyrroloethyl, 1-pyrrolopropyl and 1-pyrrolobutyl.

Alternatively, the acyl group can be a radical of the formula: 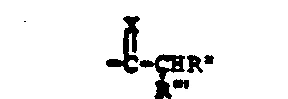 wherein R" is defined as above and R"' is a radical such as amino, hydroxy, azido, carbamoyl, guanidino, amidino, acyloxy, halo, such as Cl, F, Br, I, sulfamino, tetrazolyl, sulfo, carboxy, carbalkoxy, phosphono and the like. Representative members of the substituent 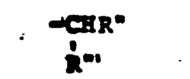 that might be mentioned are a-aminobenzyl, a-amino-(2-thienyl)methyl, a-(methylamino)benzyl, a-amino-methylmer- captopropyl, a-amino-3- or 4-chlorobenzyl, a-amino-3-or 4-hydroxybenzyl, a-amino-2,4-dichlorobenzyl, a-amino-3,4-dichlorobenzyl, D(-)-a-hydroxybenzyl, a-carboxybenzyl, a-amino-(3-thienyl)methyl D-(-)-a-amino-3-chloro-4-hydroxybenzyl, a-amino(cyclohexyl)methyl, a-(5-tetrazolyl)-benzyl, 2-thienyl-carboxymethyl, 3-thienyl-carboxymethyl, 2-furyl-carboxymethyl, 3-furyl-carboxymethyl, a-sulfaminobenzyl, 3-thienyl-sulfaminomethyl, a-(N-methylaulfamino)-benzyl, D(-)-2-thienyl-guanidinomethyl, D(-)-a-guanidinobenzyl, a-guanylureidobenzyl, a-hydroxybenzyl, a-azidobenzyl, a-fluorobenzyl, 4-(5-methoxy-1,3-oxadiazolyl)-aminomethyl, 4-(5-methoxy-l, 3-oxadiazolyl)-hydroxymethyl, 4-(5-methoxy-1,3-sulfadia- zolyl)-hydroxymethyl, 4-(5-chlorothienyl)-aminomethyl, 2-(5-chlorothienyl)-hydroxymethyl, 2-(5-chlorothienyl)-carboxy-methyl, 3-(1,2-thiazolyl)-aminomethyl, 3-(1,2-thiazolyl)-hydroxymethyl, 3-(1,2-thiazolyl)-carboxymethyl, 2-(1,4-thiazolyl)-aminomethyl, 2-(1,4-thiazolyl)-hydroxymethyl, 2-(1,4-thiazolyl)-carboxymethyl, 2-benzothienyl- aminomethyl, 2-benzothienylhydroxymethyl, 2-benzothienyl- carboxymethyl, a-sulfobenzyl, a-phosphonobensyl, a-diethylphosphono and a-monoethylphosphono. Further acyl radicals of interest in this class when X = oxygen are: 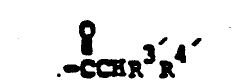 wherein R³ and R⁴ are as defined below. R³ repreaents hydrogen, halo, such as chloro, fluoro, bromo, iodo, amino, guanidino, phosphono, hydroxy, tetrazolyl, carboxy, sulfo, or sulfamino and R represents phenyl, substituted phenyl, a mono- or bicyclic heterocyclyl containing one or more oxygen, sulfur or nitrogen atoms in the ring, such as furyl, quioxalyl, thiehyl, quinolyl, quinazolyl, thiazolyl, isothiazolyl, tetrazolyl, oxadiazolyl, thiadiazolyl, and the like substituted heterocyeles, phenylthio, phenyloxy, lower alkyl of 1-6 carbon atoms, heterocyclic or substituted heterocyclic thio groups; or cyano.

The substituents on the moieties, R and R⁴, can be halo, carboxymethyl, guanidino, guanidinomethyl, carboxamido- methyl, aminoethyl, nitro, methoxy or methyl. When R³ is selected from the group consisting of hydrogen, hydroxy, amino or carboxy and R⁴ is selected from the group consisting of phenyl, or a 5- or 6-membered heterocyclic ring having one or two sulfur, oxygen or nitrogen hetero atom such as tetrazolyl, thienyl, furyl and phenyl, the following acyl radicals are representative: phenylacetyl, 3-bromophenylacetyl, p-aminomethylphenylacetyl, 4-carboxymethylphenylacetyl, 4-carboxyamidomethylphenyl- acetyl, 2-furylacetyl, 5-nitro-2-furylacetyl, 3-furylacetyl, 2-thienylacetyl, 5-chloro-2-thienylacetyl, 5- methoxy-2-thienylacetyl, a-guanidino-2-thienylacetyl, 3-thienylacetyl, 2-(4-methylthienyl)acetyl, 3-isothiazolyl- acetyl, 4-methoxy-3-isothiazolylacetyl, 4-isothiazolyl- acetyl, 3-methyl-4-isothiazolylacetyl, 5-isothiazolyl- acetyl, 3-chloro-5-isothiazolylacetyl, 3-methyl-1,2,5-oxadiazolylacetyl, 1,2,5-thiadiazolyl-4-acetyl, 3-methyl-1,2,5-thiadiazolyl-acetyl, 3-chlbro-l,2,5-thiadiazolyl- acetyl, 3-methoxy-1,2,5-thiadiazolylacetyl, phenylthioacetyl, 4-pyridylthioacstyl, cyanoacetyl, 1-tetrazolylacetyl, a-fluorophenylacetyl, D-phenylglycyl, 4-hydroxy-D-phenylglycyl, 2-thienylglycyl, 3-thienylglycyl, phenylmalonyl, 3-chloraphenylmalonyl, 2-thienylmalonyl, 3-thienylmalonyl, a-phosphonophenylacetyl, a-amino cyclo- hexadienylacetyl, a-sulfaminophenylacetyl, a-hydroxyphenylacetyl, a-tetrazolylphenylacetyl and a-sulfophenyl- acetyl.

Especially preferred embodiments of the present invention are those, as defined above, except that any unsubstituted amino group borne on radical R³ of Structure I is derivatized according to the teachings of Belgium Patent 848,545 (issued May 20, 1977); the resulting amino group being represented thusly (partial structure) : 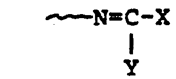 wherein X and Y are defined by the publication; species wherein X is H or lower alkyl and Y is NH₂ are especially preferred.

The products of this invention (I) form a wide variety of pharmacologically acceptable sats with inorganic and organic bases; these include, for example, metal salts derived from alkali metal or alkaline earth metal hydroxides, carbonates or bicarbonates and salts derived from primary, secondary or tertiary amines such as monoalkylamines, di-alkylamines, trialkylamines, loweralkanolamines, di- loweralkanolnmines, lower alkylenediamines, N,N-diaralkyl lower alkylenediamines, aralkylamines, amino substituted lower alkanols, N,N-diloweralkalamino substituted lower alkamols, amino-, polyamino and guanidino-substituted lower alkanoic acids and nitrogen containing heterocyclic amines. Representative examples include salts derived from sodium hydroxide, sodium carbonate,'sodium bicarbonate, potassium carbonate, potassium hydroxide, calcium carbonate, trimethylamine, triethylamine, piperidine, morpholine, quinine, lysine, protamine, arginine, procaine, ethanolamine, morphine, benzylamine, ehtylenediamine, N,N'- dibenzylethylenediamine, diethanolzmine, piperazine, dimethylaminoethanol, 2-amino-2-methyl-1-propanol, theophylline, N-methylglucamine and the like.

Salts of the amino group carried of I are also contemplated. Such pharmaceutically acceptable acid addition salts are derived from organic and inorganic acids such as HC1, HBr, citric, tartaric and the like.

The salts can be mono-salts such as the mono- sodium salt obtained by treating one equivalent of sodium hydroxide with one equivalent of the product (I), also mixed di-salts. Such salts may be obtained by treating one equivalent of a base having a divalent cation, such as calcium hydroxide, with one equivalent of the product (I). The salts of this invention are pharmaoologically acceptable nontoxic derivatives which can be used as the active ingredient in suitable unit-dosage pharmaceutical forms. Also, they may be combined with other drugs to provide compositions having a broad spectrum of activity.

The novel 6- and 6,6-disubstituted-2-aubstituted- pen-2-em-3-carboxylic acids of the present invention are valuable antimicrobial substances which are active against various gram-positive and gram-negative pathogens. Thus, the free acid and especially the salts thereof such as amino and metal salts, particularly the alkali metal and alkaline earth metal salts, are useful bactericides and can be used for removing susceptible pathogens from dental and medical equipment, for separating microorganisms, and for therapeutic use in humans and animals. For this latter purpose pharmacologically acceptable salts with inorganic and organic bases such as those known in the art and used for the administration of penicillins and cephalosporins can be utilized. For example, salts such as alkali metal and alkaline earth metal salts, and primary, secondary and tertiary amine salts can be used for this purpose. These salts can be combined with pharmaceutically acceptable liquid and solid vehicles to form suitable dosage unit forms such as pills, tablets, capsules, suppositories, syrups, elixirs and the like which can be prepared in accordance with procedures well known in this art.

The novel compounds are valuable antibiotics active against various gram-positive and gram-negative bacteria and, accordingly, find utility in hyman and veterinary medicine. The compounds of this invention can therefore be used as antibacterial drugs for treating infections caused by gram-positive or gram-negative bacteria, for example against Staphylococcus aureus, Escherichia coli, Klebsiella pneumoniae, Bacillus subtilis, Salmonella typhosa, Pseudomonas and Bacterium proteus. The antibacterials of the invention may further be utilized as additives to animal feedingstuffs, for preserving foodstuffs and as disinfectants. For example, they may be employed in aqueous compositions in concentrations ranging from 0.1 to 100 parts of antibiotic per million parts of solution in order to destroy and inhibit the growth of harmful bacteria on medical and dental equipment and as bactericides in industrial applications, for example in waterbased paints and in the white water of paper mills to inhibit the growth of harmful bacteria.

The products of this invention may be used alone or in combination as an active ingredient in any one of a variety of pharmaceutical preparations. These antibiotics and their corresponding salts may be employed in capsule form or as tablets, powders or liquid solutions or as suspensions or elixirs. They may be administered orally, intravenously or intramuscularly.

The compositions are;preferably presented in a form suitable for absorption by the gastro-intestinal tract. Tablets and capsules for oral administration may be in unit dose presentation form, and may contain conventional excipients such as binding agents, for example, syrup, acacia, gelatin, sorbitol, tragacanth, or polyvinylpyrrolidone; fillers for example, lactose, sugar, maize- starch, calcium phosphate, sorbitol or glycine: lubricants, for example, magnesium stearate, talc, polyethylene glycol, silica; disintegrants, for example, potato starch or acceptable wetting agents such as sodium lauryl sulphate. The tablets may be coated according to methods well known in the art. Oral liquid preparations may be in the form of aqueous or oily suspension, solution, emulsions, syrups, elixirs, etc. or may be presented as a dry product, for reconstitution with water or other suitable vehicles before use. Such liquid preparations may contain conven tional additives such as suspending agents, for example, sorbitol syrup, methyl cellulose, glucose/sugar syrup, gelatin, hydroxyethylcellulose, carboxymethyl cellulose, aluminum stearate gel or hydrogenated edible oils, for example almond oil, fractionated coconut oil, oily esters, propylene glycol, or ethyl alcohol; preservatives, for example methyl or propyl p-hydroxybenzoates or sorbic acid. Suppositories will contain cdnventional suppository bases, elgl, cocoa butter or other glyceride.

Compositions for injection may be presented in unit dose :form in ampules, or in multidose containers with an added preservative. The compositions may take such forms as suspensions, solutions, or emulsions in oily or aqueous vehicles, and may contain formulatory agents such as suspending, stabilizing and/or dispersing agents. Alternatively, the active ingredient may be in powder form for reconstitution with a suitable vehicle, e.g., sterile, pyrogen-free water, before use.

The compositions may also be prepared in suitable forms for absorption through the mucous membranes of the nose and throat or bronchial tissues and may conveniently take the form of powder or liquid sprays or inhalants, lozenges, throat paints, etc. For medication of the eyes or ears, the preparations may be presented as individual capsules, in liquid or semi-solid form, or may be used as drops etc. Topical applications may be formulated in hydrophobic or hydrophilic bases as ointments, creams, lotions, paints, powders, etc.

Also, in addition to a carrier, the instant compositions may include other ingredients such as stabilizers, binders, antioxidants, preservatives, lubricators, suspending agents, vixcosity agents or flavoring agents and the like. In addition, there may also be included in the composition other active ingredients to provide a broader spectrum of antibiotic activity.

For veterinary medicine the composition may, for example, be formulated as an intramammary preparation in either long acting or quick-release bases.

The dosage to be administered depends to a large extent upon the condition of the subject being treated and the weight of the host, the route and frequency of administration, the parenteral route being preferred for generalized infections and the oral route f_{b}r intestinal infections. In general, a daily oral dosag consists of from about 15 to about 600 mg. of active ingredient per kg. of body weight of the subject in one or more applications per day. A preferred daily dosage for adult humans lies in the range of from about 80 to 120 mg. of active ingredient per kg. of body weight.

The instant compositions may be administered in several unit dosage forms as, for example, in solid or liquid orally ingestible dosage form. The compositions per unit dosage, whether liquid or solid may contain from 0.1% to 99% of active material, the preferred range being from about 10-60%. The composition will generally contain from 15 mg. to about 1500 mg. of the active ingredient; however, in general, it is preferable to employ a dosage amount in the range of from about 250 mg. to 1000 mg. In parenteral administration the unit dosage is usually the pure compound in a slightly acidified sterile water solution or in the form of a soluble powder intended for solution.

The following examples illustrate but do not limit the product, process, compositional or method of treatment aspects of the present invention. All temperatures are in °C.

### SECTION A

### EXAMPLE 1

### Preparation of Azetidinone 1

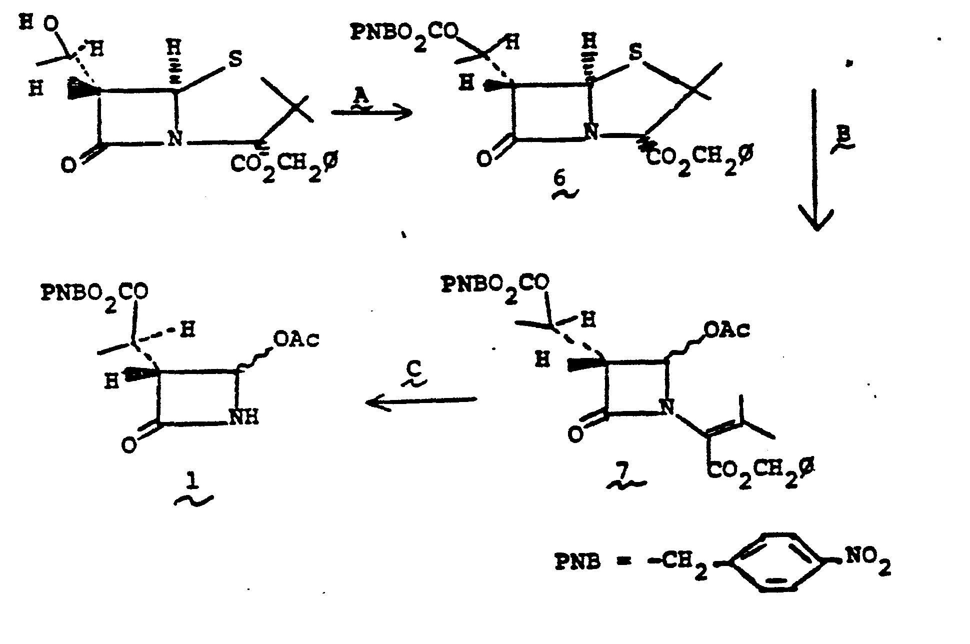

### Step A

### Preparation of 6

To a stirred solution of 215 mg. (0.6 mmol) of benzyl-6α-[(R)-1'-hydroxyethyl]penicillanate and 276 mg (0.13 mmol) p-nitrobenzyl-chloroformate in 5 ml dry methylene chloride at 0°C is added in one portion solid 4-dimethylaminopyridine (156 mg., 0.13 mmole). The mixture is stirred at 0°C under a nitrogen atmosphere for 2 min. and allowed to warm over 28 min. The mixture is poured into ice-H₂0 and extracted with CH₂C1₂. The organic phase is separated and washed successively with cold, dilute, aqueous HCl and saturated NaCl (aq.). After drying with MgSO₄, the filtered solution is evaporated and dried in vacuo to give 420 mg of residue. Purification by plate layer chromatography [one development C₆H₆-EtOAc(9:1)] provides 278.2 mg. (84%) product 6; ir (CHCl₃) 1770, 1740 cm⁻¹; nmr (CDCl₃)δ 1.4, 3H(s); 1.48, 3H(d, J=6Hz); 1.61, 3H(s); 3.46, 1H(dd, J-2, 7Hz); 4.5, 1H(s); 5.2, 2H(s); 5.23, 1H(m); 5.27, 2H(s); 5.31, 1H(d, J=_{2H}z); 7.37, 5H(s); 7.53, 2H(d, J=8Hz); 8.23, 2H(d, J=8Hz).

### Step

### Preparation of 7

A stirred mixture of 186.8 mg (0.36 mmol) of 6 and 232 mg. (0.73 mmol) Hg(OAc)₂ (mercuric acetate) in 7 ml glacial HOAc (acetic acid) is heated at 90°C. for 1.5 hour under a nitrogen atmosphere. The cooled mixture is filtered through supercel, washing thoroughly with CH₂C1₂. The filtrate is diluted with H₂0 and is neutralized with solid NaHCO₃. The mixture is extracted thoroughly with CH₂C1₂ and the combined extracts are washed successively with saturated NaHCO₃ (aq.) and saturated NaCl (aq.). After drying with MgSO₄, the filtered solution is evaporated and the residue so obtained is purified by plate layer chromatography [one developmetn CHCl₃-EtOAc(20:1)] to provide 138.6 mg (71%) of approximately a 1:1 mixture of cis and trans- acetoxyazetidinones 7; ir (CHCl₃) 1770, 1750, 1731 cm⁻¹; nmr (CDCl₃)δ1.4 δ 1.53, 3H(d's, J's=6Hz and 7Hz); 1.86, 1.96 & 2.0 6H(s); 2.23 3H(s); 3.4 & 3.58, 1H(dr's, J's= 2,7Hz & 4,10Hz); 5.2, 1H(m); 5.2, 2H(s); 5.23, 2H(s); 6.23 & 6.3, 1H(d's, J's=2 & 4Hz); 7.3, 5H(s); 7.53 2H(d, J=8Hz); 8.2, 2A(J=8Hz); mass spectrum m/e 540 (M⁺) 498, 390, 346, 301, 222, 210, 193, 136, 91.

### Step C

### Preparation of 1

To a stirred solution of 138.6 mg (0.26 mmol) of azetidinones 7 in 3.5 ml of 8:1(CH₃)₂CO-H₂O and 1 drop of . pH 7 O.lN phosphate buffer at room temperature (25°C) is added 40.6 mg (0.26 mmol) of solid RMnO₄. The mixture is stirred under a nitrogen atmosphere at 25°C for 8 min. After this time, 40.6 mg (0.26 mmol) of additional KMnO₄ is added and the mixture is stirred further for 45 min. The reaction mixture is diluted with EtOAc (ethylacetate) and treated with cold, aqueous Na₂S₂0₃ until the violet coloration of KMnO₄ is no longer apparent. The mixture is filtered through celite and is washed well with EtOAc. The filtrate is washed with saturated NaCl (aq.), dried (MgSO₄), filtered, and evaporated. Purification of the residue by plate layer chromatography [one development CHCl₃-EtOAc(3:1)] gives 65.5 mg. (72%) of the azetidinone mixture 1; ir (CHCl₃) 3300, 1785, 1750 cm⁻¹; nmr (CDCl₃)δ, 1.5 & 1.53, 3H(d's, J's-7Hz); 1.98 & 2.12, 2H(s); 2.43, (dd, J-2, 6Hz) & 2.65, (dq, J-1.5, 4.0, 9Hz) [lH]; 5.28, 2H(S); 5.3, 1H(m); 5.88 & 5.95, 1A(d's, J's=1.5 & 4.0Hz); ₆.₉₃, 1H(bs); ₇.₅₇, _{2H}(d, J=8Hz); 8.25, 2H(d, J=8Hz); mass spectrum m/e 309, 292, 249, 181, 154, 136.

### EXAMPLE 2

### Preparation of 6-α-[(R)-1'hydroxyethyl]-2-aminoethylthio-Pen-2-em-3-carboxylic acid (I)

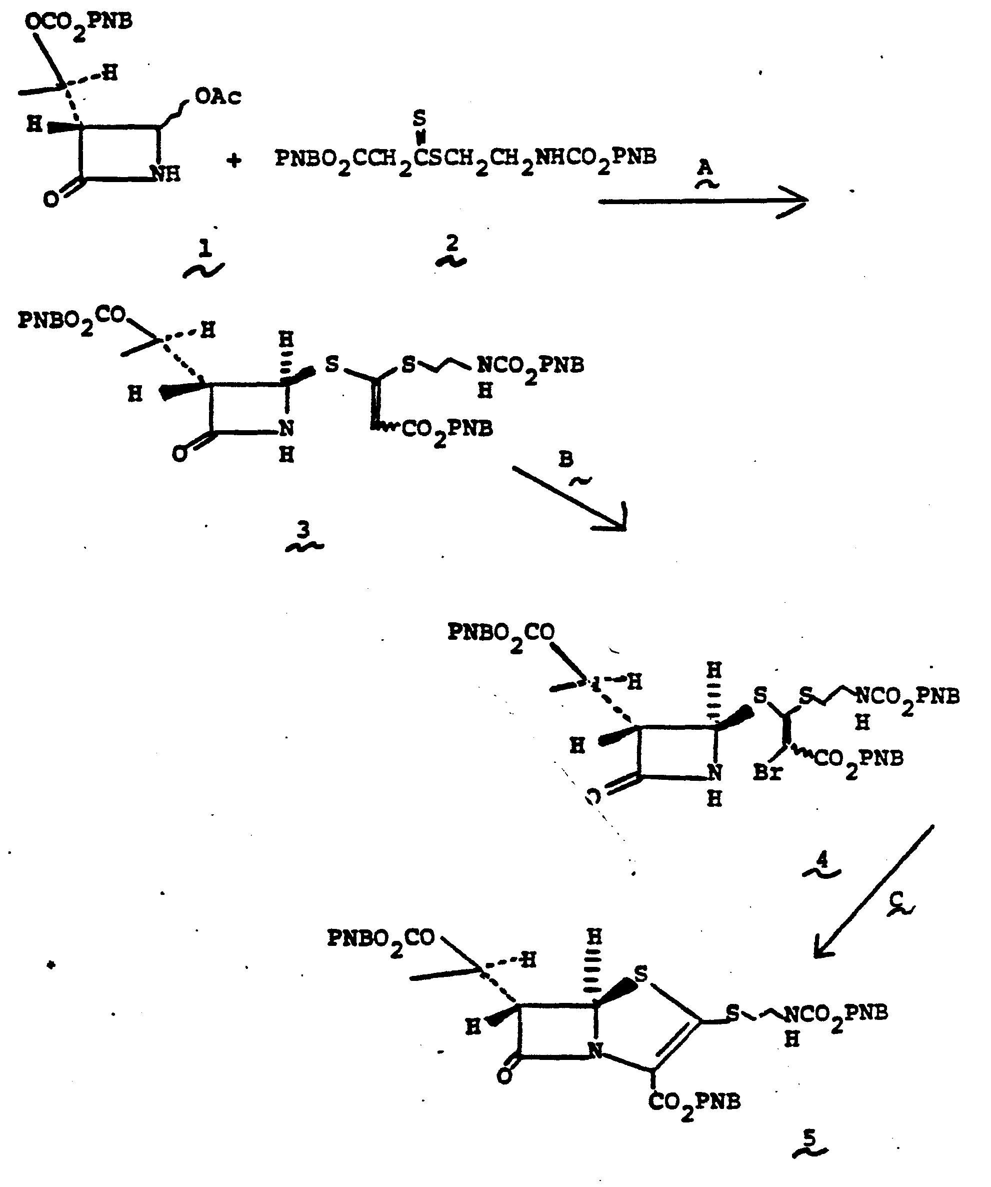

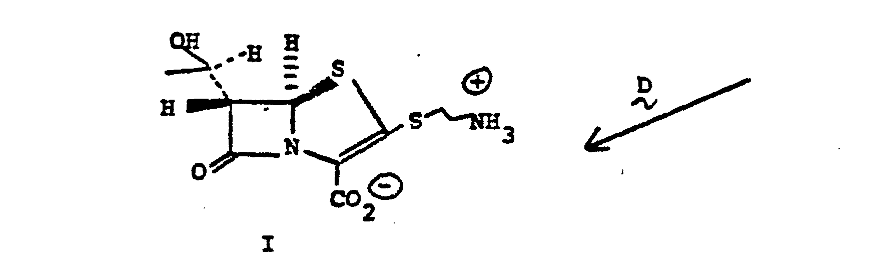

### Step A

### Preparation of 2

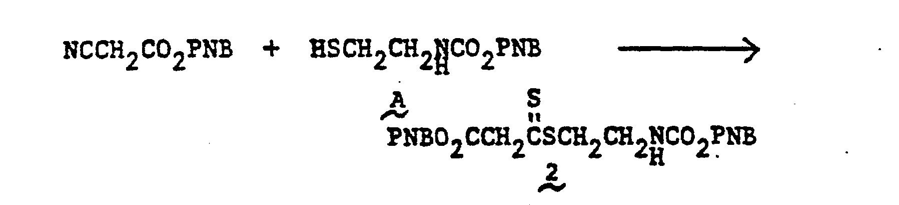

To a stirred solution of 3.77 g (17.2 mmol) of p-nitrobenzylcyanoacetate and 4.0 g (15.6 mmol) of N-p-r.itro-Cbz-cysteamine (A) in 40 ml dry benzene at 25°C under a nitrogen atmosphere is introduced a stream of HC1(g) for 7 min. The reaction mixture is cooled on an ice water bath and the introduction of HCl(g) continued for 3 min. The ice water bath is removed and the mixture is stirred magnetically for 24 hrs. After this time, the solution is removed from the separated solid with the aid of a filter stick and the solid is washed with dry benzene (2 x 40 ml) in the same manner. The solid is dried in vacuo in the reaction flask, then dissolved in dry dimethylsulfoxide (DMSO) at 25°C. To the stirred solution is added an excess of H₂S(g) under nitrogen atmosphere for 10 min. and the mixture is stirred at 25°C. for 1.0 hr. The resulting orange colored solution containing some suspended material is poured into 200 ml cold H₂0 and 200 ml Et₂0. The organic phase is separated and washed with H₂0 (4x) and saturated NaCl(aq) solution, then dried over MgSO₄, filtered and evaporated to give 3.4 g of residue. Purification is accomplished by chromatography on silica gel eluting with benzene:ethylacetate (4:1) to give 2.1 g (67% based on 59% conversion) of 2 as a yellow-orange oil which slowly solidifies; IR(CHCl₃) 3400, 1720 (sh), 1₇10, 160₁, 1520 cm⁻¹; _{NMR} (CDCl₃)δ: 3.45 (m, 4_{H}); 4.08 (s, 2H); 5.1 (bs, 1H):5.17 (s, 2H); 5.27 (s, 2H); 7.42 (d, J-8Hz, 4H); 8.12 (d, J=8Hz, 4H); mass spectrum m/e no 493(M⁺) 446, 256, 239, 209, 195, 165, 153, 136, 120.

### Step B

### Preparation of Seco-Lactam 3

To a stirred mixture of 62.2 mg (0.18 mmol) of azetidinones 1 and 87.1 mg (0.18 mmol) of dithioate 2 in 1.5 ml of dry THF at 25°C is added in one portion 50.5 mg (0.25 mmol) of solid aluminum isoproproxide [Al(O-<)₃]. The mixture is stirred at 25°C under N₂ for 6.0 hr. and is then partitioned between EtOAc and a cold, aqueous solution of dilute HC1-tartaric acid mixture. The EtOAc phase is separated and is washed with saturated NaCl(aq.), dried (MgSO₄), filtered, and evaporated. Purification by plate layer chromatography [2 developments CHCl₃-EtOAc (3:1)7 provides 66.4 mg. (48%) of white foam 3, ir (CHC1₃) 1770, 1725, 1690, 1600 cm⁻¹; nmr (CDCl₃)δ1.48, 3H(d, J=6Hz); 3.2, 2H(m); 3.5, 3H(m).; 5.24, 8H(m); 6.1, 1H(s); 6.83. 1H(bs); 7.56, 6H(m); 8.24, 6H (apparent doublet, J=8Hz); 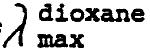 263 nm (E% = 427);α_{D} = +49.7°.

### Step C

### Preparation of Bromide

To a stirred solution of lactam 3 (66.4 mg, 0.085 mmol) and 15.1 mg (0.05 mmol) HMPA (hexamethylphosphoramide in 2.0 ml dry THF (tetrahydrofuran) at 25°C is added in one portion 16.5 mg (0.093 mmol) solid NBS (N-bromosuccinimide). The mixture is stirred at 25°C under N₂ for 0.5 hr. and evaporated. The residue is partitioned between EtOAc and H₂0 and the EtOAc phase is separated. It is further washed with H₂0 (2x) and saturated NaCl (aq.), then dried (MgSO₄), filtered, and evaporated. Purification by repetitive plate layer chromatography [2 developments CHCl₃-EtOAc (3:1)] affords 54.7 mg (75%) of material after the first chromatogram and 35.7 mg (49%) of white foam 4 after the second chromatogram; ir (CHC13) 3375, 1775, 1720, 1600 cm⁻¹; nmr (CDCl₃)δ1.48, 3H (d, J=6Hz); 2.96, 2H(m); 3.37, 2H(m); 3.46, lH(dd, J-2.5, 7Hz); 5.18, 2H(s): 5.1-5.4, 2H(m); 5.24, 2H(s), 5.38; 2H(s); 5.7, 1H(m); 6.72, 1H(bs); 7.56, 6H(m); 8.25, 6H(m); _{D} = +48.5°; 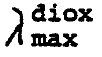 264 nm (E% - 394).

### Step D

### Treparation of penem

To a stirred mixture of 35.7 mg (0.04 mmol) of bromide 4 and 28 mg (0.14 mmol) of CuBr·S(CH₃)₂ in 5 ml dry THF at -78°C under N₂ is added 2 ml of a cold, freshly prepared solution of lithium diisopropylamide, LiN(-<)₂, [generated at 0°/20 min. with 5 mg (0.05 mmol) HN(-<)₂ (diisopropylamide) and 19 ul of 2.4 M buLi (butyllithium)7. The mixture is stirred over the following temperature ranges for the times indicated: -78° to -74° (40 min.); -74° to -68° (30 min.); -68° to -57° (30 min.); -57° to -41° (20 min.); -41° to -26° (20 min.); -26° to -20° (28 min.); -20° to -13° (38 min.); -15° to -9° (53 min.); -10° to -5° (18 min.); and -5° to 0° (18 min.). The mixture is then treated at 0° with 1 ml of saturated NH₄Cl (aq.) and diluted with Et₂O/H₂O. The organic phase is separated and washed further with aqueous NH₄Cl and saturated NaCl (aq.). The organic layer is dried (MgSO₄), filtered, and evaporated. The residue is purified by plate layer chromatography [two developments in CHC1₃-EtOAc (3:1)7 to afford 18.6 mg (58%) of penem 5 as an off-white foam; ir (CHC1₃) 3430, 1799, 1730, 1704, 1601, 1520 cm⁻¹; nmr(CDCl₃)δ: 1.40, 3H(d, J=5.5Hz); 3.09, 2H(m); 3.54, 2H(m); 3.63, lH(dd, J=2.5, 6.0 Hz); 5.22, 9H(m); 7.58, 6_{H}(m); 8.26, 6H(m); 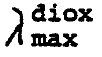 265 nm (_{E%} 292), 315 nm (E% 91.5);α_{D} = 41.3°.

### Step E

### Preparation of Penem I

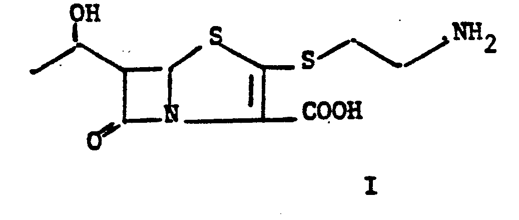

To 5.2 mg 5(Step D, above) is added 0.60 ml dioxane, 0.05 ml ethanol, 0.35 ml deionized water and 0.01 ml of 1.0m R₂HPO₄. To the resultant clear solution is added 5 mg of 10% Pd/C. The suspension is flushed with N₂, then 5-6 times alternately with 50 psi H₂ and vacuum. Finally, it is shaken under a 50 psi H₂ atmosphere for 30-40 min. After centrifugation, the Pd/C is washed and centrifuged 2-3X with 0.5 ml portions of deionized water. The combined centrifugates are extracted 5x1-2 ml ether. Residual ether is removed under vacuum and theaqueous solution applied to an XAD-2 column (20x140 mm). Fractions of 100 drops (6-7ml) are collected, with continuous UV monitoring, by elution with deionized water. The chosen fractions are combined and lyophilized to provide I.

### SECTION B

### EXAMPLE 2

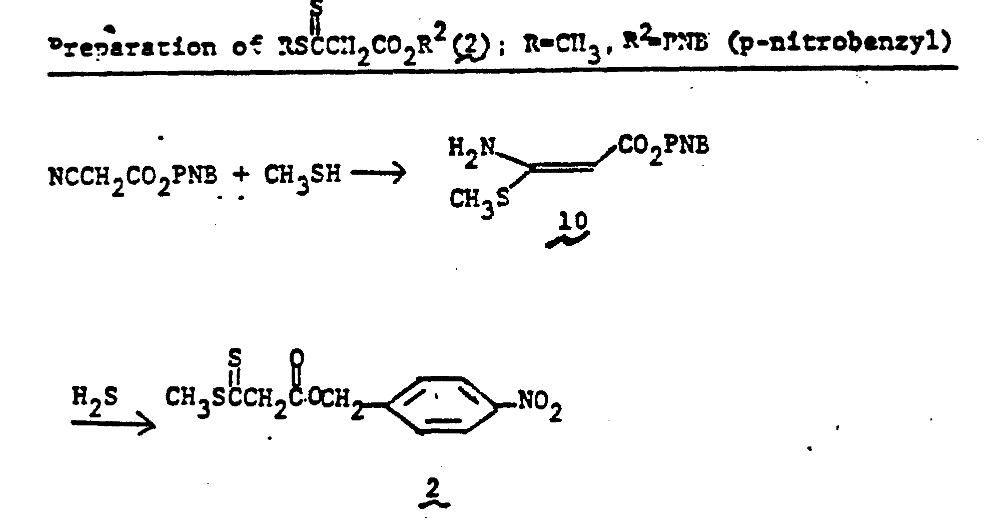

To a stirred mixture of 11.0 g (.05mol) of p-nitrobenzylcyanoacetate and 3.72 g (.078 mol) methyl- mercepten in 200 ml of sodium dried benzene at 25°C is introduced a stream of HCl(g) until 1.85 g (.05 mmol) is added. The mixture is then stirred at 25°C under nitrogen atmosphere for 74.5 hrs. The solution is deCanted away from the separated solid and the moist precipitate is dissolved in 50 ml of dry pyridine. (Alternatively the precipitate is dissolved in dry dimethyl- fulfoxide and treated with H₂S(g) as described below to afford 2 directly.) The pyridine solution is treated with excess H₂S(g) and is stirred at 25°C under nitrogen atmosphere for 2 hrs. The reaction mixture is poured into ice/water and extracted with ether. The separated extract is washed twice with water and twice with Erine solution then dried over MgSO₄, filtered, and evaporated to give a crystalline mass. Recrystallization from ether gives 5.0 g of the enamide 10; ir(CHC1₃), 3636, 3322, 1664, ₁₅₉₂, ₁₅₁₇ cm⁻¹; NMR(CDCl₃)δ: 2.4(s, 3_{H}), 4.68 (s, 1H); 5.17 (s, 2H); 6.43 (bs, 2H); 7.43 (d, J=8Hz, 2H)= 8.13 (d, J=8Hz, 28); mass epectrum m/e 268 (M⁺), 221, 175, 136. Purification of the mother liquors from above by column chromatography on silica gel eluting with CHCl₃-pet-ether (1:1) provides 940 mg of 2 as an orange oil: ir (CHCl₃) 1742, 1613, 1522; NMR(CDCl₃)δ2.67 (s, 3H); 4.13 (s, 2H); 5.23 (s, 2H); 7.47 (d, J=9Hz, 2H); 8.17 (d, J=9Hz, 2H); mass spectrum m/e 285 (M⁺), 238, 136, 106. The enamide 10 is converted to 2 by the following procedure.

The crude enamide 10 7.9 g (29 mmol) is dissolved in 40 ml of dry DMF (dimethylformamide) at 25°C and is treated successively with 3.7 g (32 mmol) of trifluoroacetic acid and excess hydrogen sulfide. The resulting mixture is stirred magnetically at 25°C under CaCl₂ drying tube protection for 5 hrs. The mixture is poured into 75 ml ice-H₂O and extracted with 100 ml Et₂0 (diethylether). The separated etheral extract is washed twice with H₂0 and saturated NaCl(aq) solution, then dried with MgSO₄, filtered, and evaporated. Purification by column chromatography on silica gel eluting with CHC1₅ petroleum ether (1:1) gives 6.55 g (74%) of 2.

### EXAMPLE 3

Preparation of dithioate derivative 2,

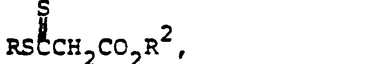

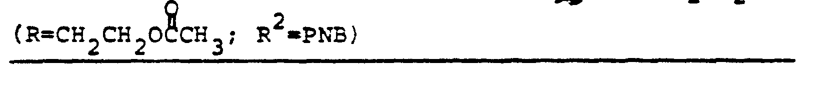

To a stirred mixture of 6.78 g (5.6 mmol) of 2-acetoxyethyl mercaptan and 11.0 g (5 mmol) p-nitro- benzylcyanoacetate in 100 ml of dry benzene is introduced 3.0 g (8.1 mmol) lf hydrogen chloride gas. The mixture is stirred under N₂ at RT (25°C) for 42 hours after which time the solution is decanted away from the oily mass. The mass is washed twice with dry benzene by decantation. The oil is dissolved in 20 ml of dry dimethylformamide and is treated with a vigorous stream of hydrogen sulfide (g) for 5 min. The mixture is stirred at 25°C for 5 hours and then is poured into ice cold water and is extracted with ether. The ether extract is washed well with water, then brine, and is dried over MgS0₄, filtered, and evaporated. Purification by column chromatography on silica gel provides the dithioate derivative 2 as an orange solid; ir (CHCl₃) 1770, 1600, 1538 cm⁻¹; nmr (CDCl₃)δ: 2.03, 3H(s); 3.53, 2H(t, J-6Hz); 4.1, 2H(s); 4.27, 2H(t,J=6Hz); 5.25, 2H(s); 7.46, 2H(d,J=9Hz); 8.2, 2H(d,J-9Hz); mass spectrum m/e 357 (M⁺), 297, 271, 206, 169, 136.

### EXAMPLE 4

### Preparation of Benzyl-thiobenzoylacetate 2,

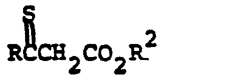

### (R - phenyl; R² = benzyl):

Into a stirred solution of 19.2 g of benzylaceto- acetate in 150 ml of acetonitrile at -60°C is passed a stream of hydrogen sulfide gas for 1.5 hours, followed by dry HC1(g) for 40 min. The reaction solution is then let warm to -20°C and is poured into ice water. The product 2 is extracted into benzene and purified by vacuum distillation to provide the known benzyl-thio- benzo^{y}lacetate, bp 98° (0.05 mm).

### EXAMPLE 5

Following the procedure described in the foregoing text and Examples, the following starting reagents 2 necessary for the preparation of the compounds of the present invention are representatively obtained by analogy.

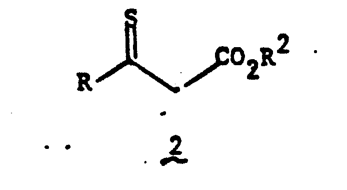

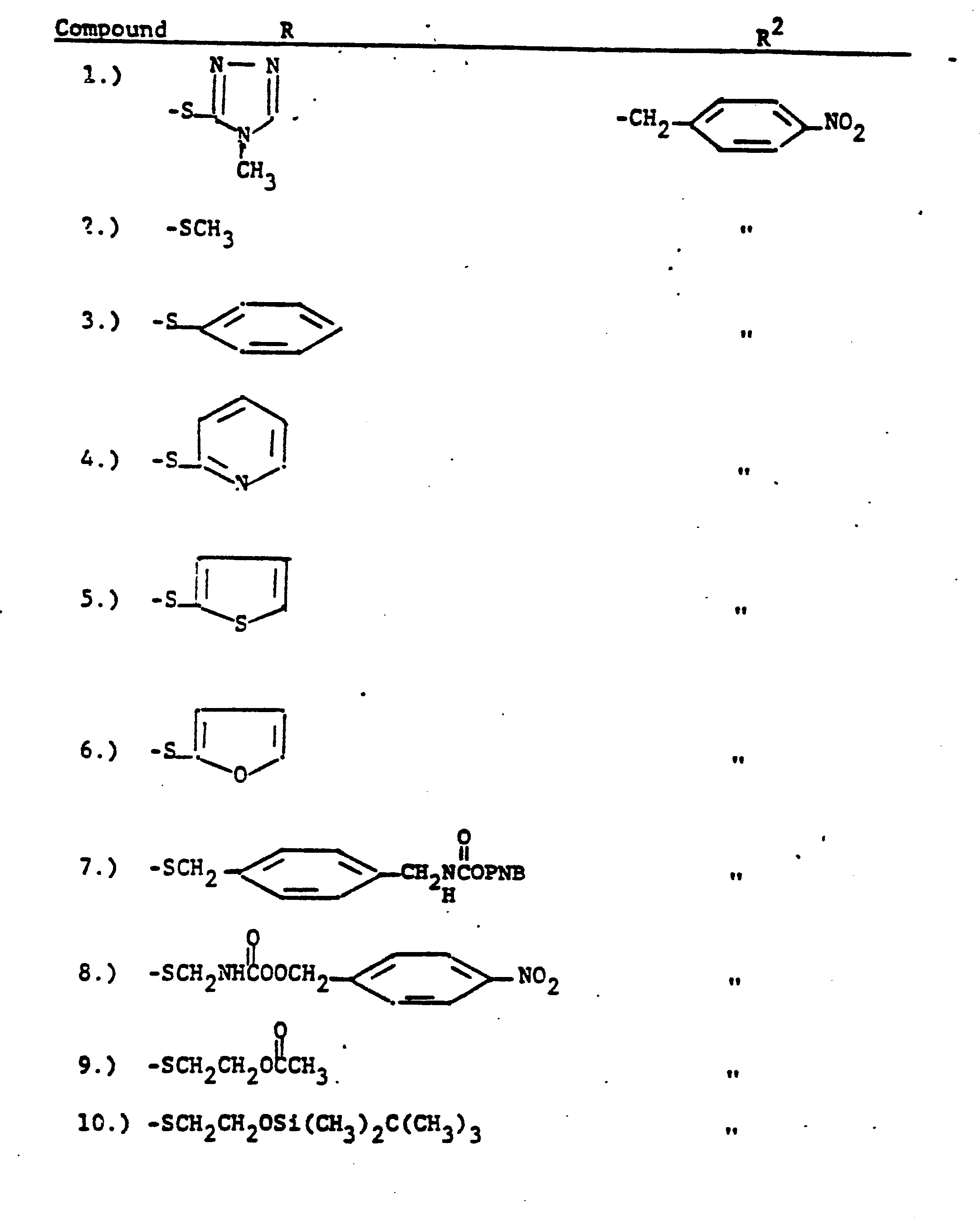

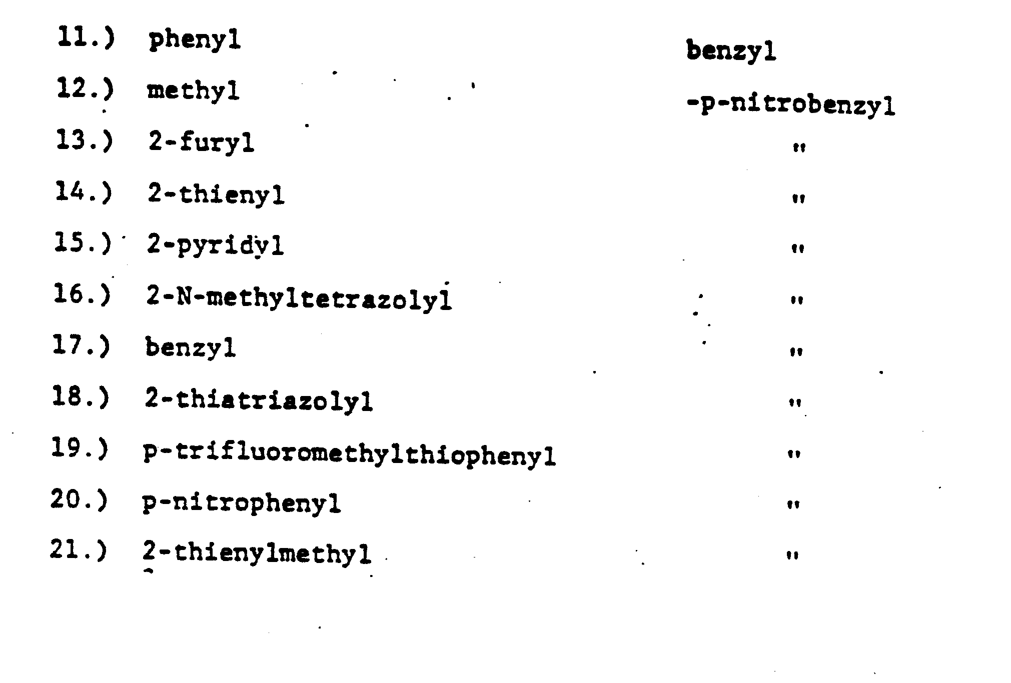

### EXAMPLE 6

### Preparation of secolactam 3(R₁=R₂=PNB, R=SCH₃)

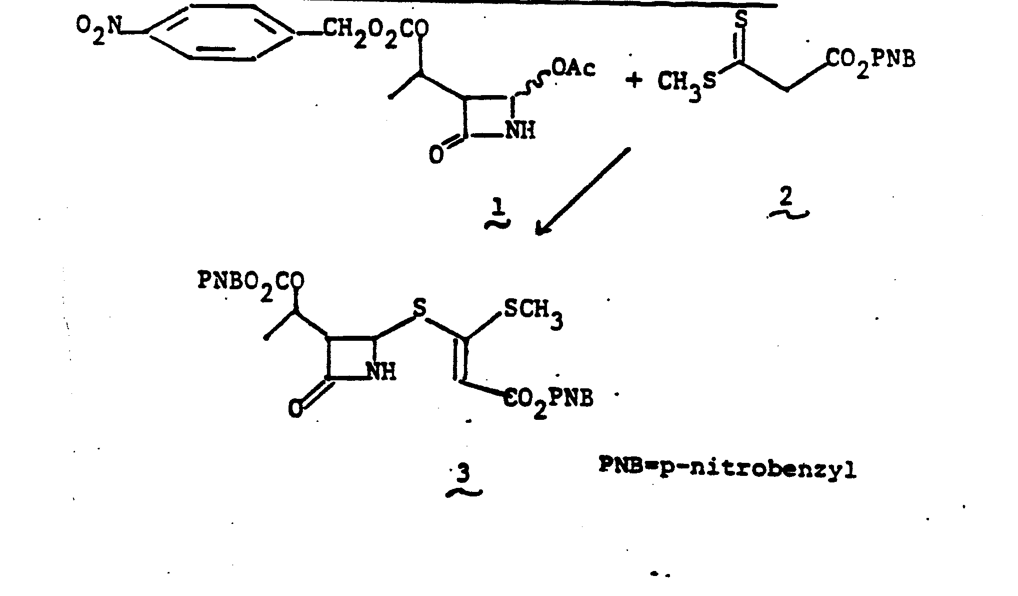

To a stirred mixture of 70.4 mg (0.2 mmol) of azetidinones 1 and 57.0 mg (o.2 mmol) of dithiomalonate derivative 2 in 2 ml of dry tetrahydrofuran (THF) at 25°C is added 57.2 mg (0.28 mmol) of solid aluminum isopropoxide. The mixture is stirred at 25°C under nitrogen atmosphere for 24 hours and is then partitioned between ethylacetate and cold, dilute aqueous HCl. The EtOAc phase is separated, washed with brine, dried with MgSO₄, filtered, and evaporated. Purification by plate layer chromatography provides seco-laetam 3.

### EXAMPLE 7

Following the procedures described in the foregoing Examples and text, the following secolactams 3 are prepared by analogy.

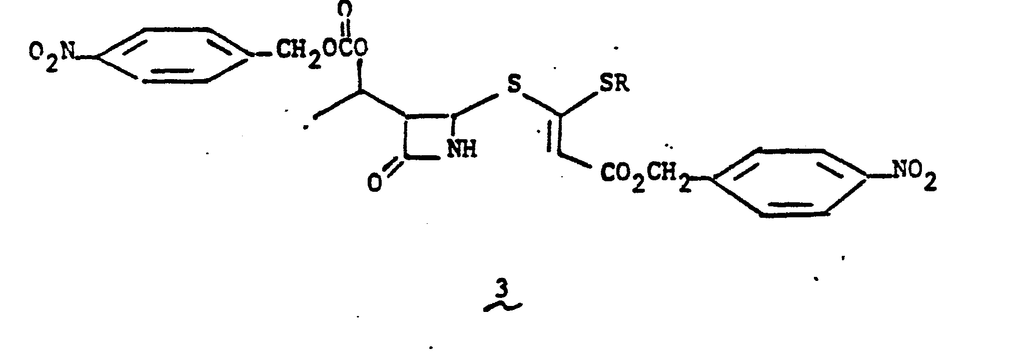

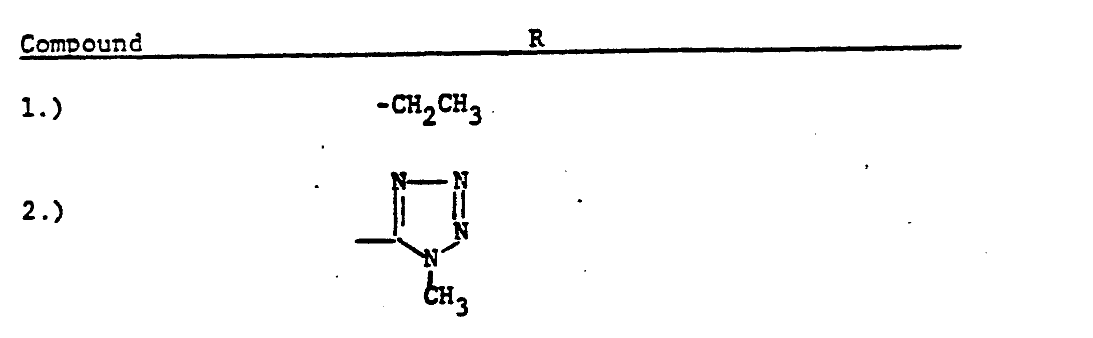

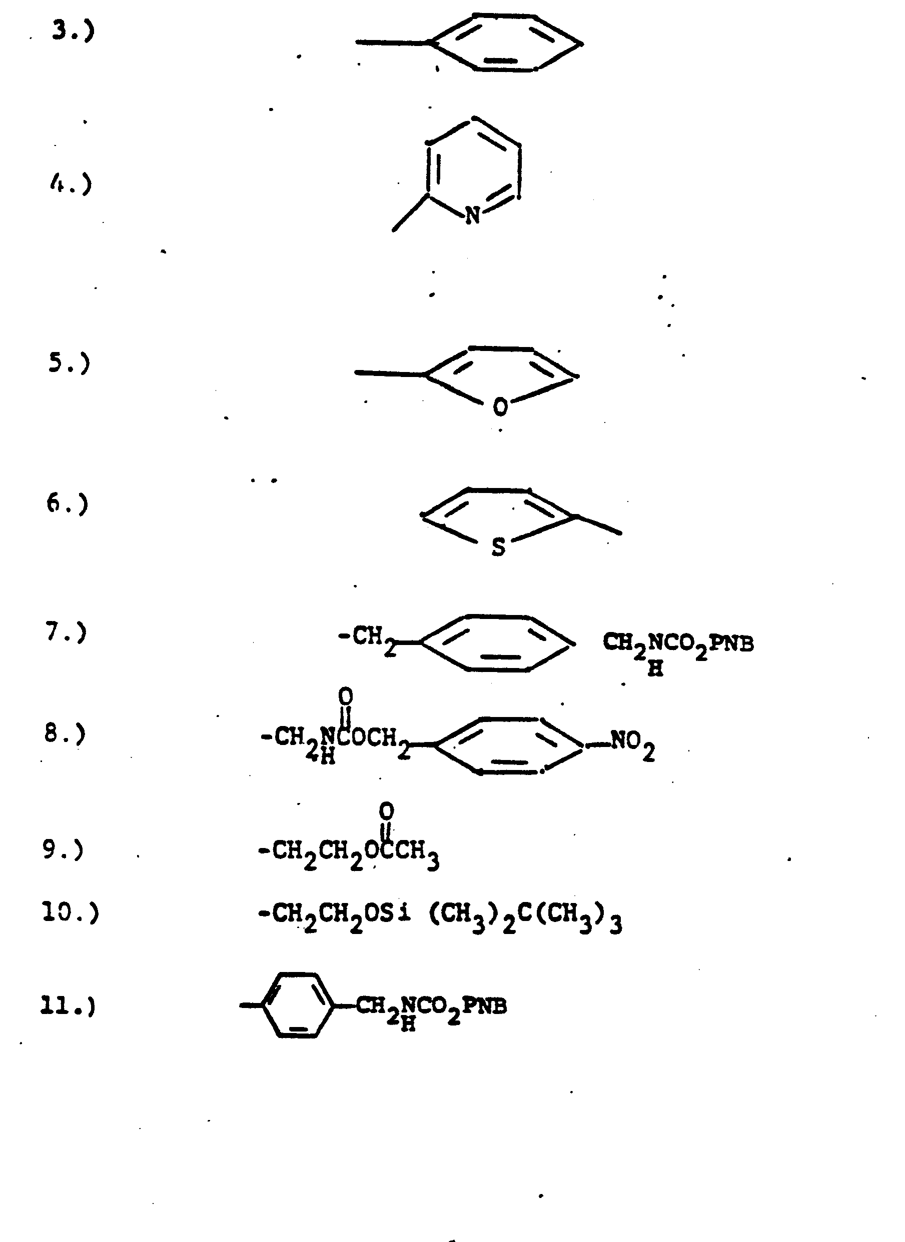

### EXAMPLE 8

### Preparation of secolactam 3

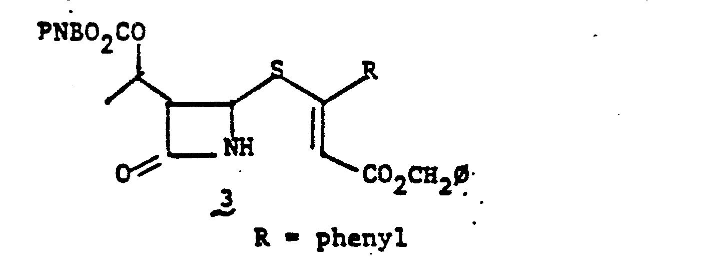

To a stirred mixture of 35.2 mg (0.1 mmol) of azetidinones 1 and 27 mg (0.1 mmnol) of benzylthiobenzoyl- acetate in 2.0 ml methanol at 25°C is added 59.4 mg (0.11 mmol) of sodium methoxide. The mixture is stirred at 25°C under a nitrogen atmosphere for 24 hours. The solvent is removed by evaporation and the residue is partitioned between ethyl acetate and cold water. The EtOAc phase is separated, washed with brine, dried with MgSO₄, filtered, and evaporated. The secolactam 3 is obtained by plate layer chromatography.

### EXAMPLE 9

Employing the procedures described in the foregoing text and Examples, the following azetidinones 3 are prepared by analogy:

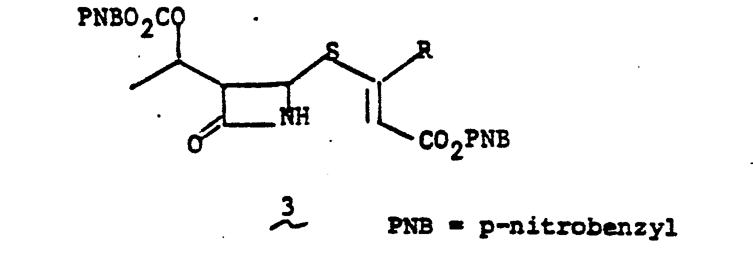

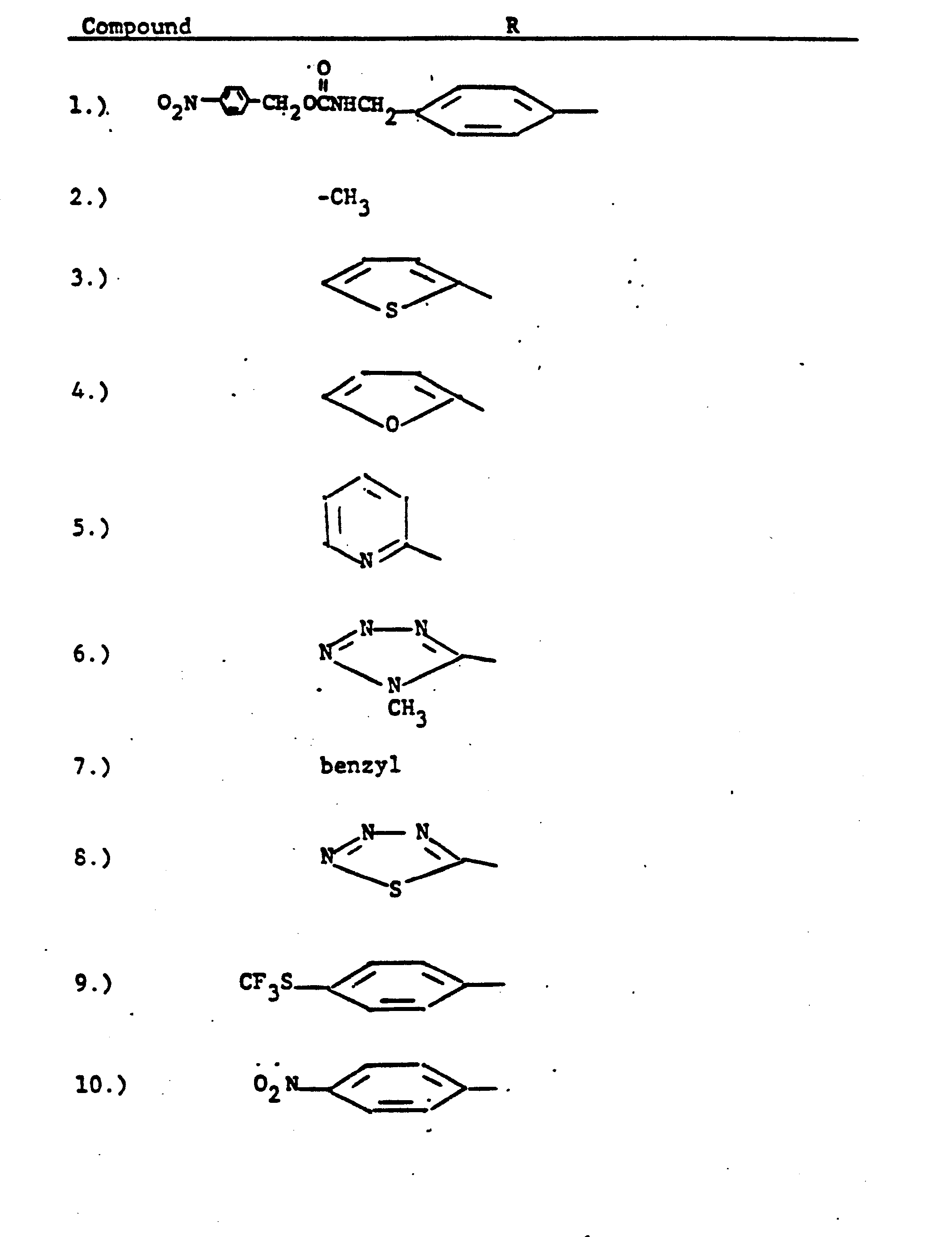

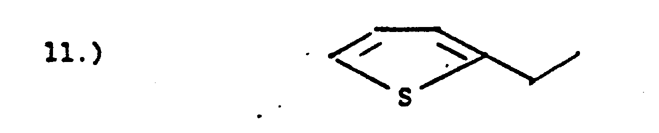

### EXAMPLE 10

### Preparation of bromide 4

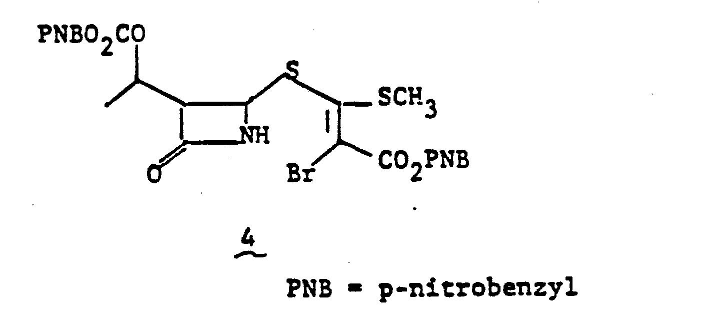

To a stirred solution of the secolactam 3 prepared in Example 6 (57.7 mg 0.1 mmol) and 17.9 mg (0.1 mmol) of hexamethylphosphoramide in 2 ml dry tetrahydrofuran at 25°C is added 17.8 mg (0.1 mmol) of N-bromosuecinimide. The mixture is stirred at 25°C under N₂ for 0.5 hr. and the solvent is removed under reduced pressure. Purification by repetitive plate layer chromatography affords bromide 4.

### EXAMPLE 11

Following the procedures described in the foregoing text and Examples, the following intermediate species 4 are representatively prepared by analogy.

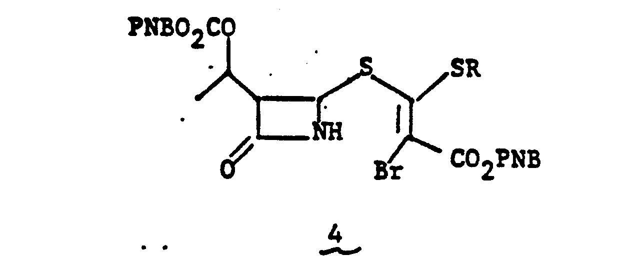

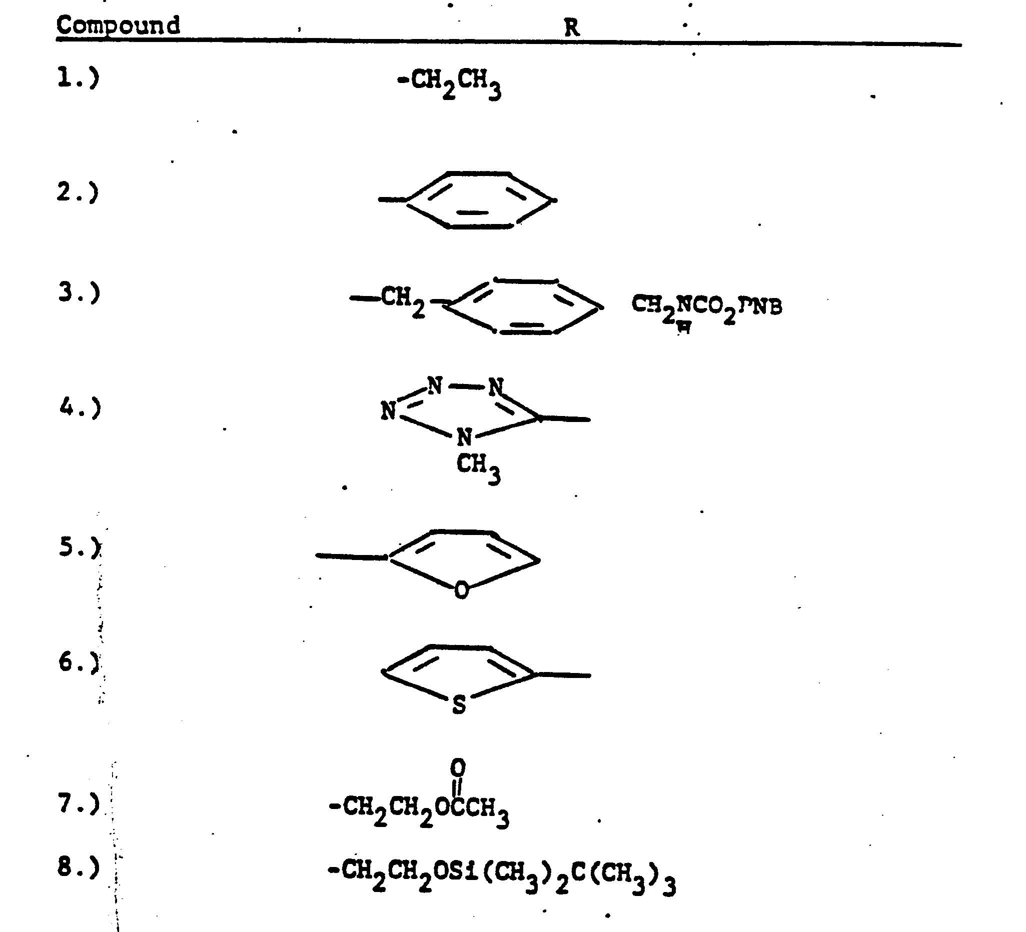

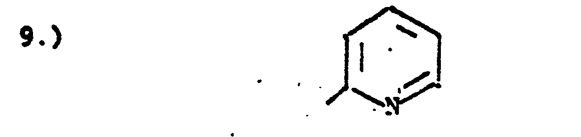

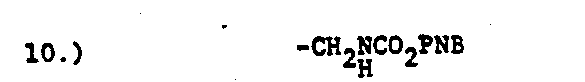

### EXAMPLE 12

### Preparation of bromide 4

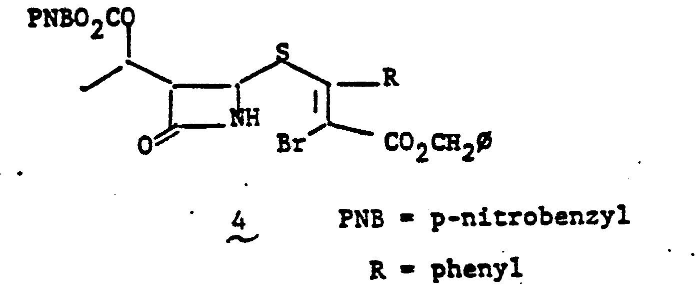

To a stirred mixture of the seeolactam derivative 3 of Example 8 (56.2 mg, 0.1 mmol) and 17.9 mg (0.1 mmol) of hexamethylphosphoramide in 2 ml dry tetrahydrofuran at 25°C is added 17.8 mg (0.1 mmol) of N-bromosuccinimide. The mixture is stirred at 25°C under N₂ for 1.0 hour and the solvent removed under reduced pressure. Purification by repetitive plate layer chromatography provides bromide 4.

### EXAMPLE 13

Following the procedure described in the foregoing text and Examples, the following bromides 4 are representatively obtained:

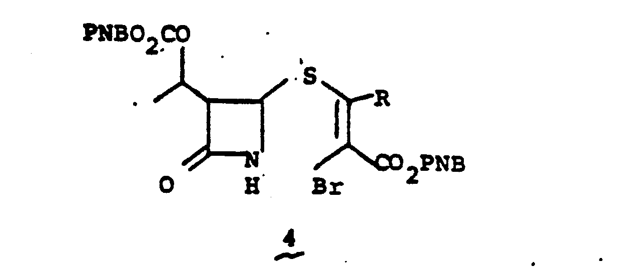

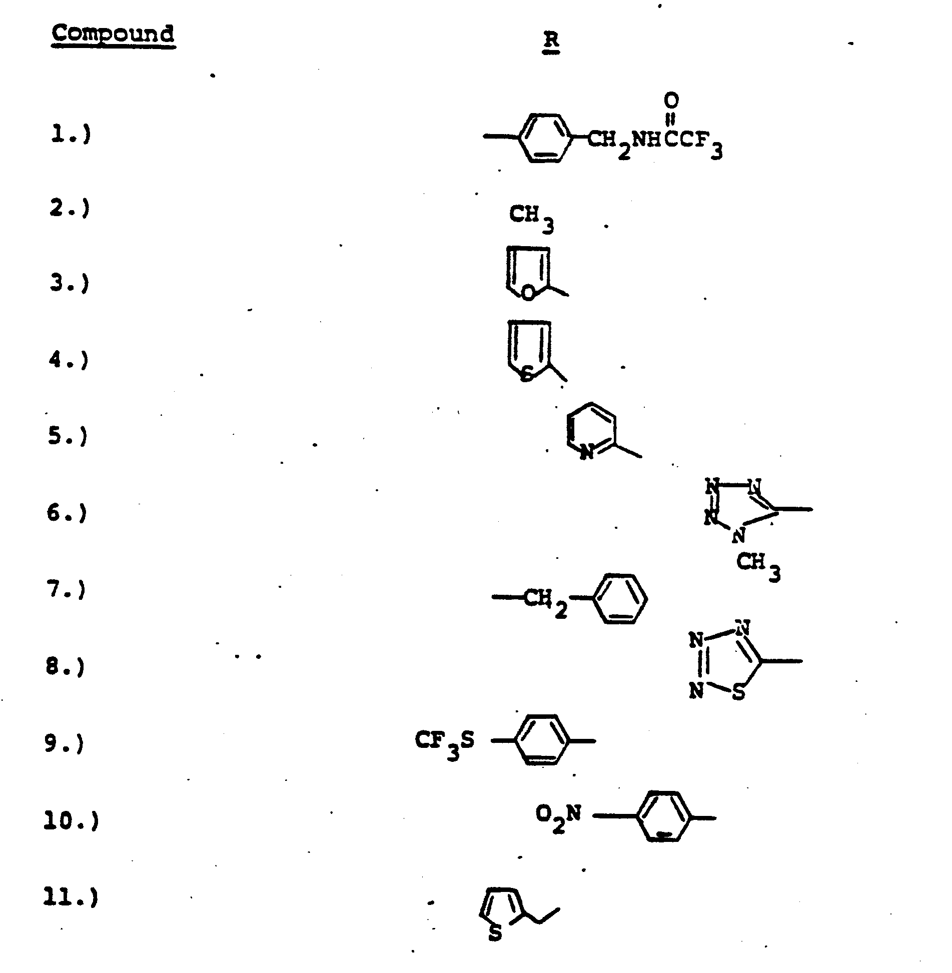

### EXAMPLE 14

### Preparation of Penem 5

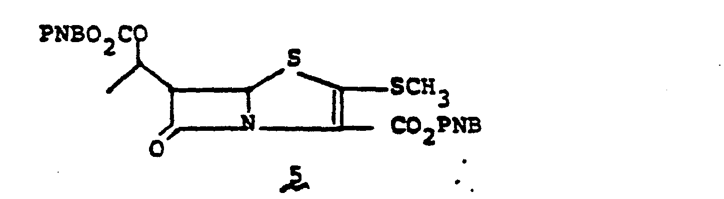

To a stirred mixture of the seco lactam broaaide 4 of Example 10 (65.6 mg, 0.1 mmol) and cuprous bromide-dimethylsulfide complex (67.7 mg, 0.33 mmol) in 10 ml dry tetrahydrofuran at -78°C under a nitrogen atmosphere is added a cold, freshly prepared solution of lithium diisopropylamide (o.ll mmol) in 4 ml dry tetrahydrofuran. The mixture is stirred at -78°C for 40 minutes and is allowed to warm to 0°C over a period of 5 hours. At 0°C, 2 ml of saturated ammonium chloride (aq.) solution is added and the mixture is partitioned between ether and water. The organic phase is separated and washed further with saturated ammonium chloride solution and brine. The organic phse is separated, dried over MgSO₄, filtered, and evaporated. Purification is accomplished by plate layer chromatography to yield penem 5.

### EXAMPLE 15

Following the procedure described in the foregoing Examples and text, there is obtained the following representative penems 5 by analogy:

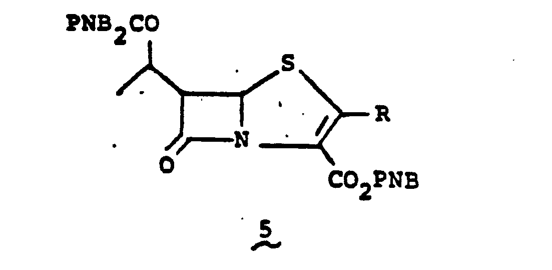

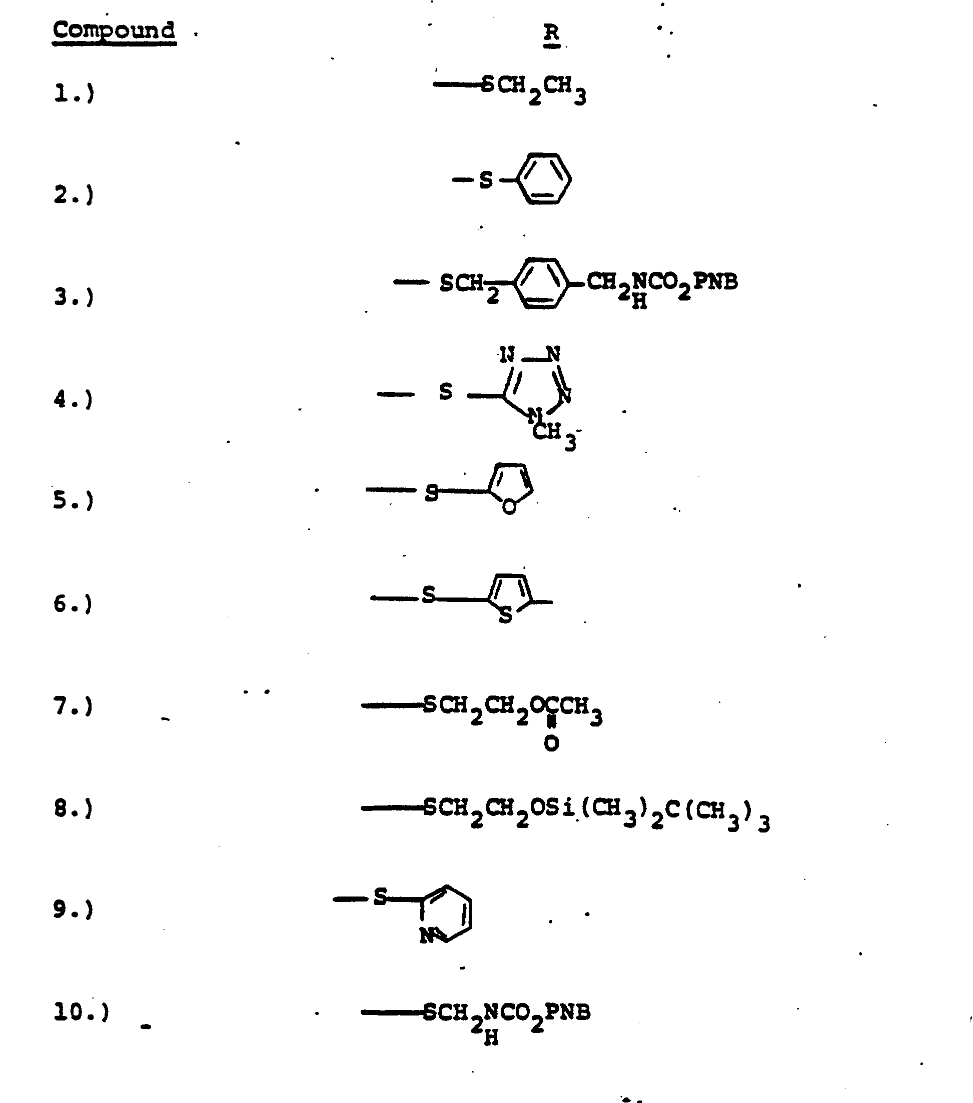

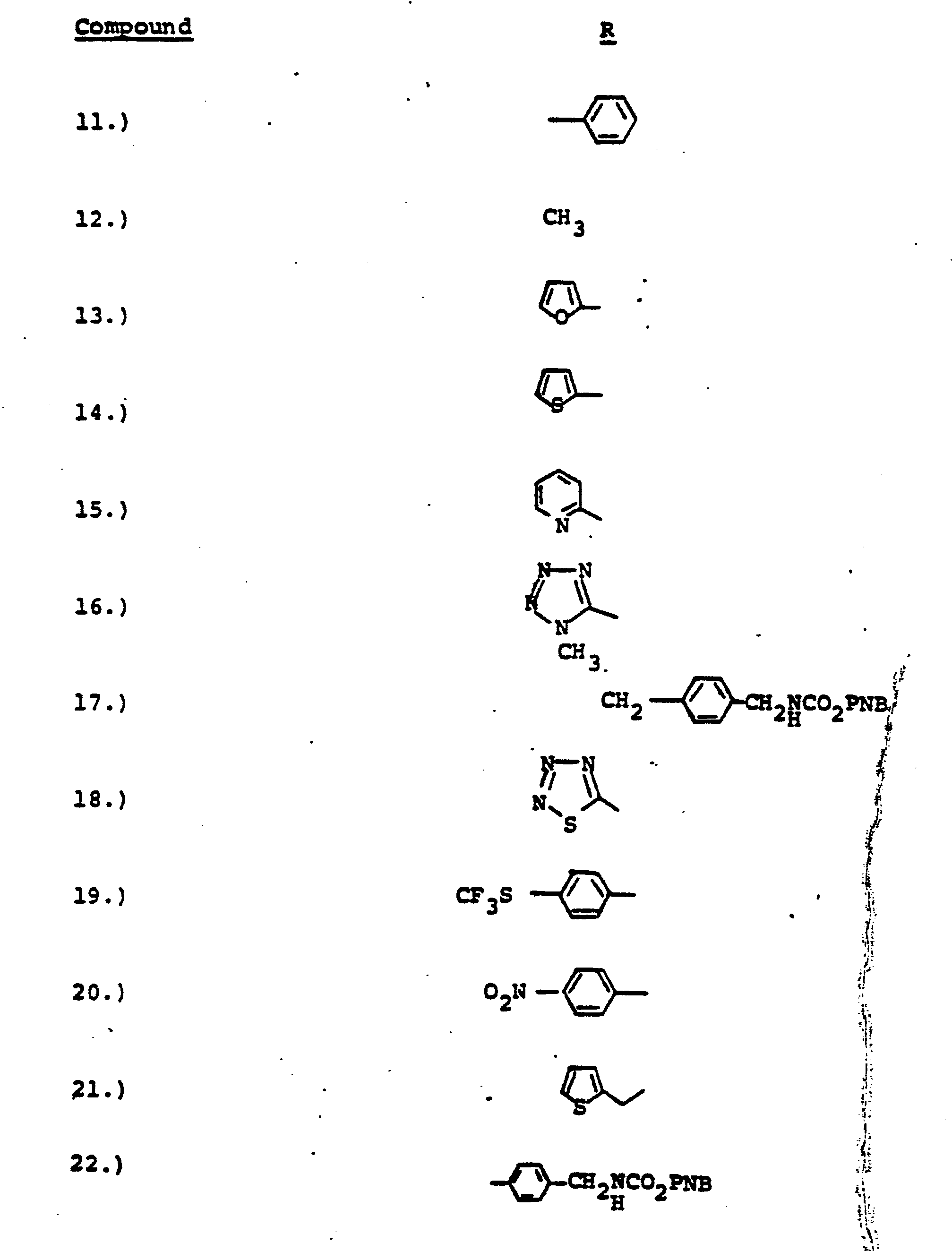

### EXAMPLE 16

### Preparation of penem I.

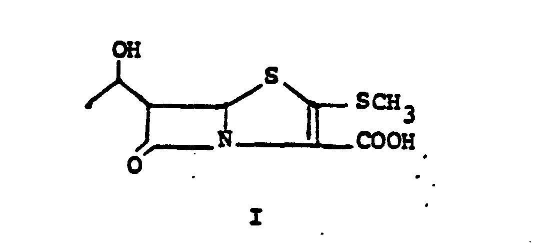

A mixture of 10 mg of 10% palladium on carbon, 5 mg. (0.0086 mmol) of the penem of Example 14 and 1 mg. sodium bicarbonate in 0.8 ml ehtylacetate, 0.2 ml isopropanol, and 0.5 ml of 0.5M pH 7 phosphate buffer is hydrogenated at 40 psi at 25°C in a Parr shaker for 48 minutes. The catalyst is removed by filtration through supercel and is washed thoroughly with deionized water. The cold filtrate is extracted with ethylacetate and the separated aqueous phase is acidified at 0°C with 0.1M pH 2 phosphate buffer to pH 2.5. The aqueous phase is extracted thoroughly with EtOAc. The extracts are combined, dried over anhydrous odium sulfate, filtered and evaporated to provide penem I. The free acid is converted to the corresponding sodium salt by treatment with an aquivalent amount of an aqueous cetone solution of sodium bicarbonate, followed by removal f the acetone under reduced pressure and lyophilization of he aqueous solution.

In cases where the deblocking procedure results in the frmation of zwitterionic penems I, the above acidification in performed as described but is stopped at pH 7. The swtterionic product is then obtained after chromatography on -AD-2 resin.

### EXAMPLE 17

### Preparation of Sodio-6-[1'-Hydroxyethyl]-2-pen-2-em-3-carboxylate

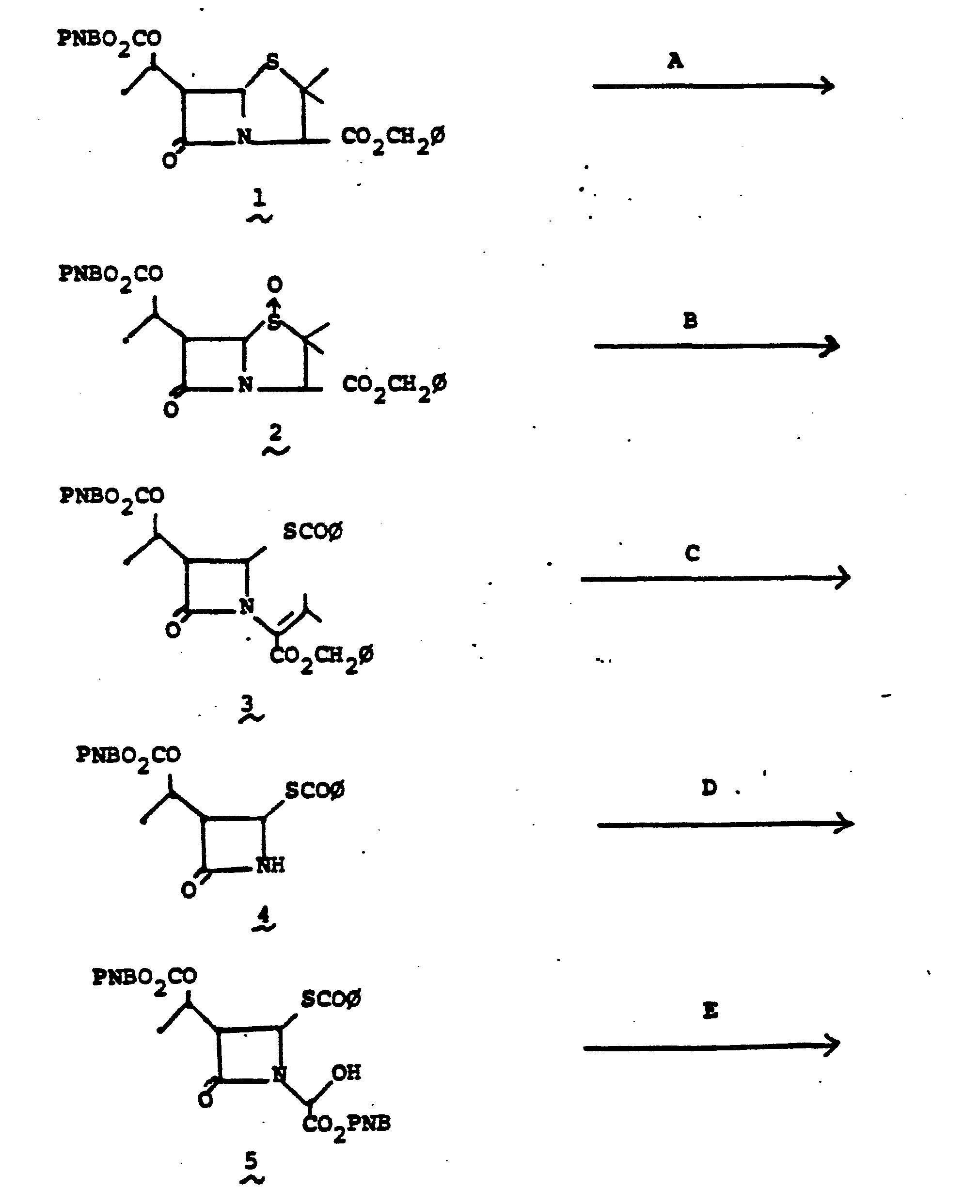

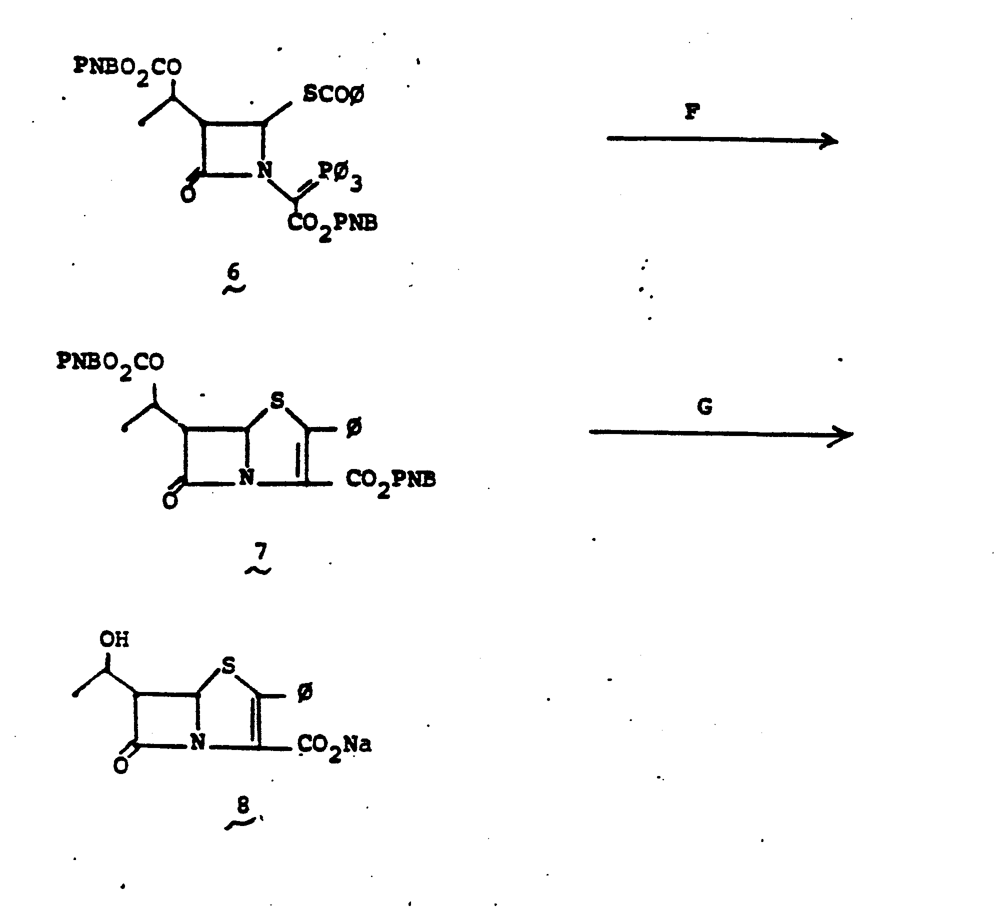

### step A:

To a stirred solution of benzyl-6α-[1'-p-nitrobanzyloxycarbanylaxyethyl]penicillanate (643 mg, 1.25 mmoles) in 15 ml. methylene chloride at 25°C is added all at once 253.8 mg (1.25 mmolee) of 85% m-chloroperbenzoic acid. The mixture is stirred under an atmosphere of nitrogen at 25°C for 15 min and is then partitioned bstraeen ethyl acetate and ice cold, aqueous sodium bicarbonate solution. The orgaaic phase is separated, washed further with saturated, aqueous eodium chloride solution, dried (MgSO₄), filtered, and evaporated. The residue is purified by plate layer chromatography (PLC) [development CH₂Cl₂-EtOAc (9:1)) to give 326.3 mg (49%) of the desired product as a colorless oil: IR(CHCl₃) 1790 and 1750 cm⁻¹, NMR(CDCl₃) δ:1.1 (S, 3H), 1.53 (d, J=6Hz, 3H), 1.65 (5,3H), 3.78 (dd, J=2 and 7Hz, 1H), 4.53 (s,1H), 4.97 (d, J=2Hz, 1H) , 5.24 (m,5H), 7.37 (S,5H), 7.57 (d, J=8Hz, 2H), and 8.25 (d, J=8Hz, 2H).

### Step B

A stirred mixture of 107.2 mg (0.2 mmoles) of the sulfoxide obtained in Step A, 49 mg (0.24 mmoles) of tri-n-butylphosphine, and 229 mg (1.0 mmolee) of benzoic anhydride in 3.0 al of benzene is refluxed at 90°C under a nitrogen atmosphere for 7.0 hours.. The cooled mixture is partitioned between ethyl acetate and cold, aqueous sodium bicarboaate and the orgaaic phase is separated. The organic phase is washed with saturated, aqueous sodium chloride, dried (MgSO₄), filtered, and evaporated to give a dark, semi-solid residue. Purification by PLC [1 development CB₂Cl₂-EtOAc (30:1)] provides 57.1 mg (41%) of seco-laetam 3, as·a ligba yellow oil ; IR(CHCl₃) 1760 (broad), 1715 (sh), and 1670 C⁻¹; NMR (CDCl₃) δ:1.44 (d, J=6Hz,, 3H), 1.98 (S, 3H) , 2 2, (S, 3H) , 3.56 (dd, J=2.2, 7Hz, 1H) , 5.2 (m, 1H) , 5. (dd, J=14Hz, 2H) , 5.3 (dd, J=13Hz, 2H), 5.92 (d, J= 2Hz) 7.26 (_{S}, 5H) , 7:46 (m, 5H) , 7.86 (d, J=z) , and 8.16 (d, 3=8Hz); mass spectrum (FD) m/e 618(M⁺).

### Step C:

To tirred solutiou of 22.3 mg (0.036 mmoles) of Metidinon in 0.75 ml. of 8:1 acetone-water and two dropts 1 0.1N pH 7 phosphate buffer at 25°C is added all aence 5.7 mg (0.036 mmoles) of potassium permanganate. mixture is stirred at 25°C under nitrogen for 8 in and then an additional 5.7 mg of potassium petman state is added. The mixture is stirred further for 50 mi and is then cooled on an ice-water bath, diluted witl ethyl acetate, and is treated with 5% aqueous sodium ioaulfate. The mixture is filtered through celite and washed well with ethyl acetate. The filtrate is part bned between ethyl acetate and cold, aqueous brine ution and the organic phase is separated, dried (Mg fiitered, and evaporated. The residue is Purif [1 developoeat CHCl₃-EtOAc (3:1)] t tive 3 mg (55%) of azetidinone 4 as a colorlua fo IR(Ch 3375, 1760 (br), and 1660 CM⁻¹; NMH (CDCl₃) δ:1 (d J=6Hz, 3H), ₃.52 (dd, J=2.2 17 Hz), 5.3 ; 5.3 (_{d}d, J-13Hz, 2H), 5.5 ( 2.2Nz) 6.42 7.51 (appt, J=8.0Hz, 2H) , 7 (d, J=9Hz, 2H) , 6, (appt., J=8.0Hz, 1H) , 7,2 (d,J=8.5Hz), and (d, J-9Hz, 2H).

### Step:D

: A stiirsd mixture of 11.8 mg (0.027 amoles) of azetidinoae 4 (Step C) and a large excess of p-nitrobensylglyoualate hydrate in 1 ml of toluene and 0.5 ml of dimethylformamide containing 500 mg of ground 3A molecular sieves is heated at 80°C under a nitrogen atmosphere for 5.0 hr. The cooled mixture is diluted with ethyl acatats and is filtered. The filtrate is eoncentrstsd on a rotatory evaporator and the concentrate is partitioned between diethyl ether and cold water. The organic phase is separatad, washed further with water, dried (MgSO₄) , filtered, and evaporated. The residue is purified by PLC [1 development CHCl₃-EtOAc (3:1)] to give 5.0 mg (29%) of product 5; IR(CHCl₃): 3500, 1775, 1750, and 1665 cm⁻¹.

### Step

To a stirred solution of 12.8 mg (0.02 mmoles) of laetam 5 from Step D in 1.0 ml anhydrous tetrahydrofuran at 0°C under a nitrogen atmosphere is added in rapid successioa 2.4 mg (0.02 asaoles) of neat thionyl chloride and 2.0 mg (0.02 mmolee) of neat triethylamine. The mixture is stirred at 0°C for 2.0 hours and is then partitioned between diethyl ether and cold, aqueous brine. The organic phase is separated, washed with saturated, aqueous sodium chloride, dried (HgSO₄), filtered, and evaporated. The residue so obtained is dissolved in 0.5 ml dry tetrahydrofuran and is stirred with 10.0 mg (0.04 mmoles) of triphenyl- phosphine at 50°C under a nitrogen atmosphere for 18.0 hrs. The cooled sixture is partitioned between sthyl acetats and aqueous sodium bicarbonate sointion. The organie phase is ssparatsd, dried (Na₂SO₄), filtered, and evaporated. Purification of the residue by P_{L}C affords the phosphorane derivative

### step F

A stirred solution of 88.3 mg (0.1 mmole) of phosphoraneazetidinone (Step B) in.80 ml of toluene is heated at 85°C under an atmosphere of nitrogen for 3.5 days. The toluene is zemoved under reduced pressure and the residue is purified by plate layer chromatography to provide penem 7.

### Step G

A mixture of 60.5 mg (0.1 mmoles) of penem 7, 150 mg of 10% palladium-on-charcoal, and dilute, aqueous sodium bicarbonate in 5 ml. of ethyl acetate is hydrogenated at 25°C and atmospheric pressure for 30 minutes. After this time 25 mg of fresh catalyst is added and the mixture is hydrogenated for an additional 5 minutes. The catalyst is removed by filtration and washed with dilute, cold aqueous sodium bicarbonate and ethyl acetate. The organic phase is separated and the aqueous phase acidified with dilute, cold aqueous citric acid. Thorough extraction of the acidified aqueous phase with methylene chloride, followed by drying tNa₂SO₄) and evaporation, provides the penem carboxylic acid 8, which is immediately converted to its sodium salt by treatment with a stoichiometric amount of aqueous sodium bicarbonate in acetone solution, evaporation of acetone, and lyopholization of the aqueous solution.

### EXAMPLE 17A

### Preparations of:

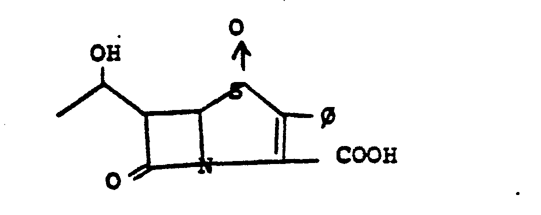

To a stirred solution of 60.5 mg (0.1 mmoles) of penem 7 from Example 17, step F in 2 ml of methylene chloride at 25°C is added 20.3 mg (0.1mmoles) of 85% m-chloroperbenzoic acid. The mixture is stirred at 25°C under a nitrogen atmosphere for 0.5 hours and is then partitioned between methylene chloride and cold, dilute aqueous sodium bicarbonate. The organic phase is separated, dried (Na₂S0₄), filtered and evaporated to yield the desired sulfoxide.

The above illustrated carboxylic acid is obtained as described in Step G of Exaample 17 when the indicated substitution is nade by catalytic hydrogenation of the sulfoxide prepared above.

### EXAMPLE 18

Following the procedures described in the foregoing Examples and text, there is obtained the following species of the present invention by analogy:

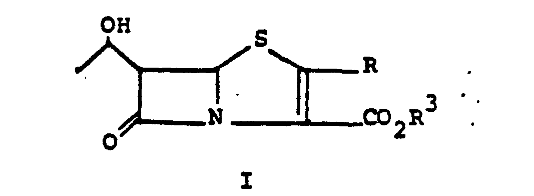

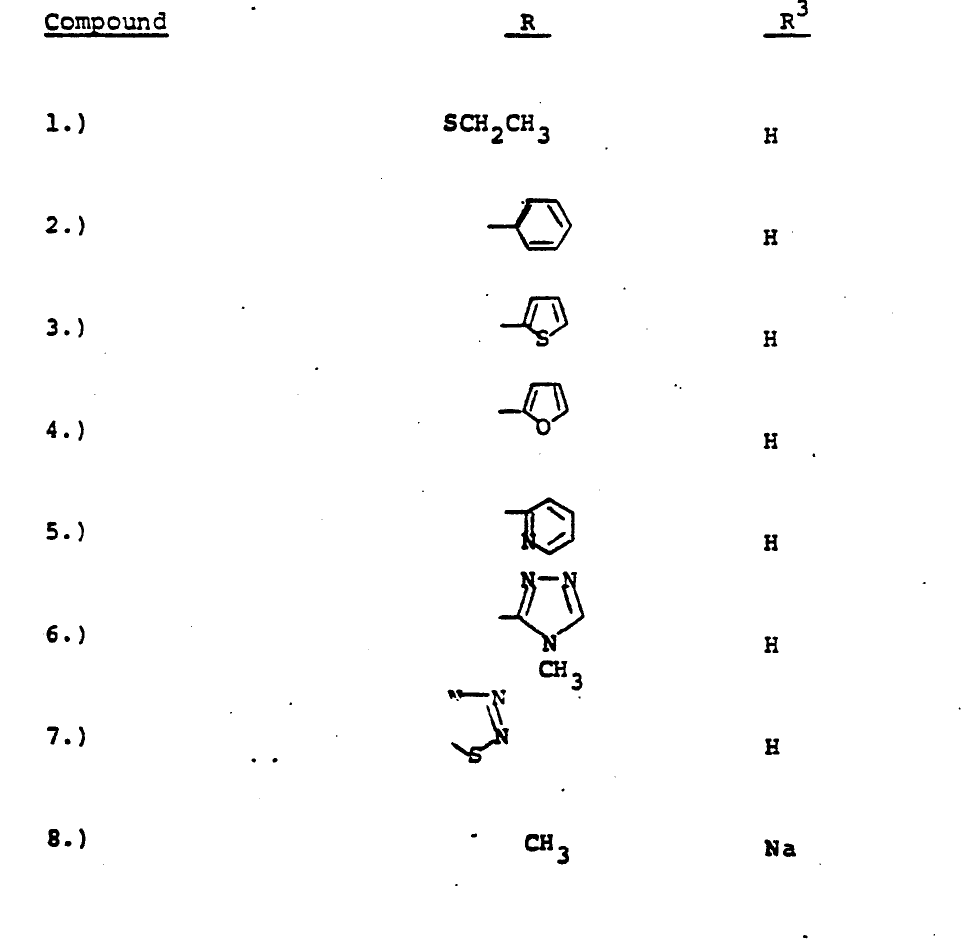

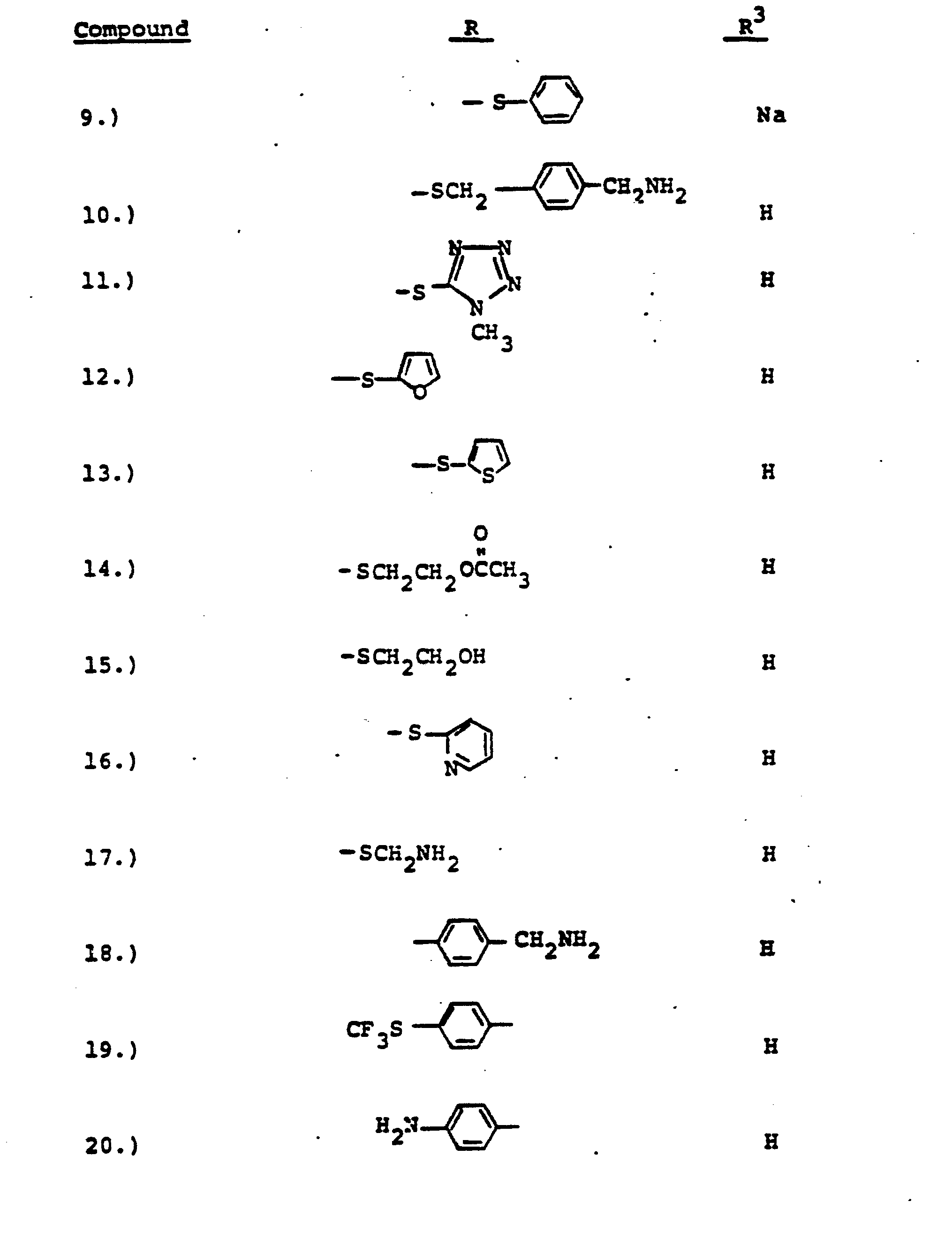

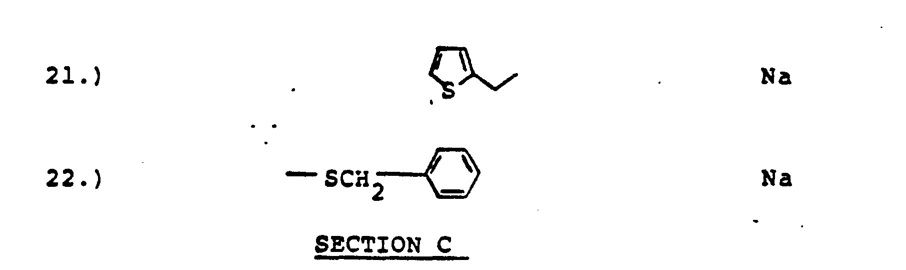

### EXAMPLE 1

Preparation of 4-acetoxy-1-(1-benxyloxycarboayl-2-methyl- prop-1-enyl)-3-methoxy-3-phenylacetamidoaxetidin-2-one (7)

A stirred mixture of 454 mg (1 mmol) of benzyl-6-methoxy-6-phenylacetamido-penicillinate [L.D. Cama, W.J. Leanza and T.R. Beattie, and B.G. Christensen, JACS, 95, 1408 (1972)] and 637.4 mg (2.0 mmol) of mercuric acetate in 15 ml of glacial acetic acid is heated at 95°C for 30 minutes. The cooled mixture is filtered and the filtrate is diluted with water. The mixture is neutralized with solid sodium bicarbonate and thoroughly extracted with chloroform. The combined chloroform extracts are washed successively with dilute aquopaus sodium bicarbonate and brine. The chloroform solution is then dried (MgSO₄) , filtered, and evaporated. Purificaison by plate layer chromatography provides 4-acetoxy-1-(1-Zinzyloxycarbonyl-2-methylpzop-1-enyl)-3-methoxy-3-phenylacetaidoazetidin- ₂-one (7).

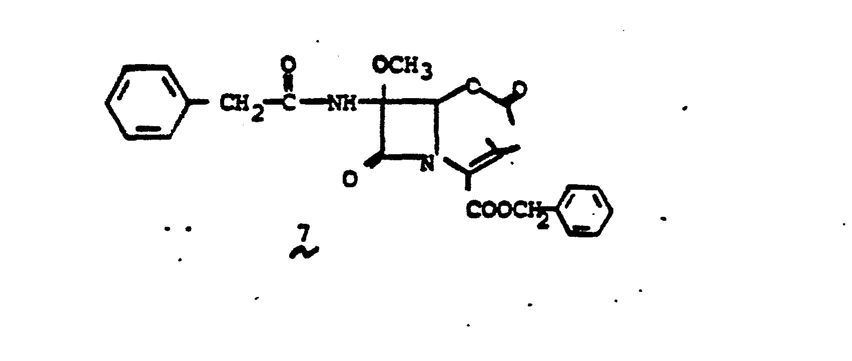

### EXAMPLE 2

### Preparation of 4-acetoxy-3-methoxy-2-phenylacetamidoazeti- din-2-one (1)

To a stirred solution of 240 mg (0.5 mmol) of azetidinone prepared in Example 1 in 8 ml of 8:1 acetone-water and 2 drops of pH 7 0.1N phosphate buffer at 25°C is added 79 mg (0.5 mmol) of solid KMnO₄. The mixture is stirred under N₂ at 25° for 8 minutes and an additional 79 mg (0.5 mmol) of KMnO₄ is then added. The mixture is stirred further for 45 minutes and diluted with EtOAc. The mixture is treated with sufficient aqueous sodium thiosulfate to destroy any remaining permanganate. The mixture is then filtered through celite and washed thoroughly with ethyl acetate. The filtrate is partitioned between EtOAc/brine and the organic phase is separated, dried (MgSO₄), filtered and evaporated. Purification of the residue by plate layer chromatography affords 4-acetoxy-3-methoxy-3-phenylacetamido-azetidin-2-one (1).

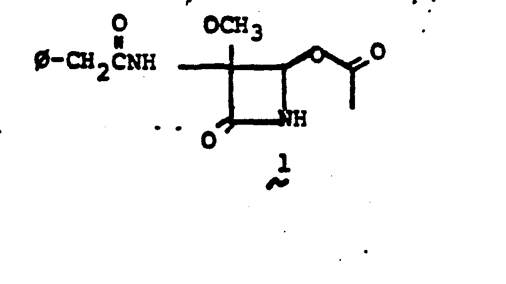

### EXAMPLE 3

### Preparation of 4-acetoxy-3-phenylacetamidoazetidin-2- one (1)

To a stirred solution of 4-acetoxy-1-(1-methoxycarbonyl-2-methylprop-1-enyl)-3-phenylacetamidoaze- tidin-2-one (709 mg, 1.9 mmol (R.J. Stoodley and N.R. Whitehouse, J. Chem. Soc., Perkin I, 32(1973).] in 5 ml dimethylformamide, 5 ml pyridine and 1 ml water at 0°C in an ice-water bath is added 497 mg (3.15 mmol) solid potaasium permanganate. The mixture is stirred at 0°C under a nitrogen atmosphere for 1.0 hour. The mixture is treated with sufficient aqueous sodium thiosulfate to destroy the excess permanganate and is diluted with ethylacetate. The mixture is filtered and the filtrate is partitioned between ethylacetate and dilute aqneous hydrochloric acid. The organic phase is separated and is washed successively with dilute aqueous sodium bicarbonate and brine. The organic phase is dried (MgSO₄), filtered, and evaporated. The residue is purified by plate layer chromatography [1 development OH-EtOAc (1:1)] to give 95.1 mg (19%) of 1: nmr (CDC1₃) 2.03, 3H(s); 3.5, 28(s); 4.67; 1H(dd, J=18z,78z); 5.73, 18(d, J=1Hz); 7.17, 6H(s) ; 7.53, 1H(bs).

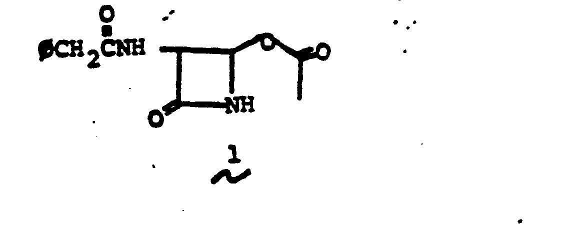

### EXAMPLE 4

Following the procedures in the text and in Examples 1, 2, 3, the following 4-acetoxyazetidinones are obtained by analogy:

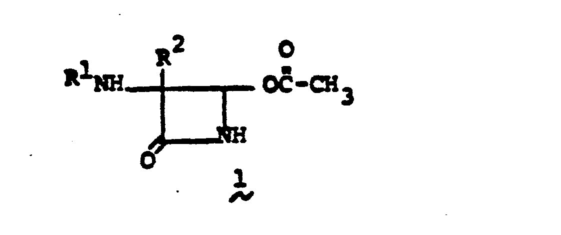

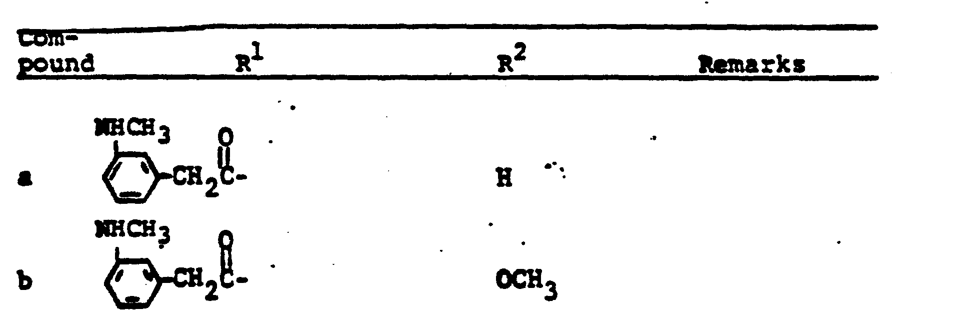

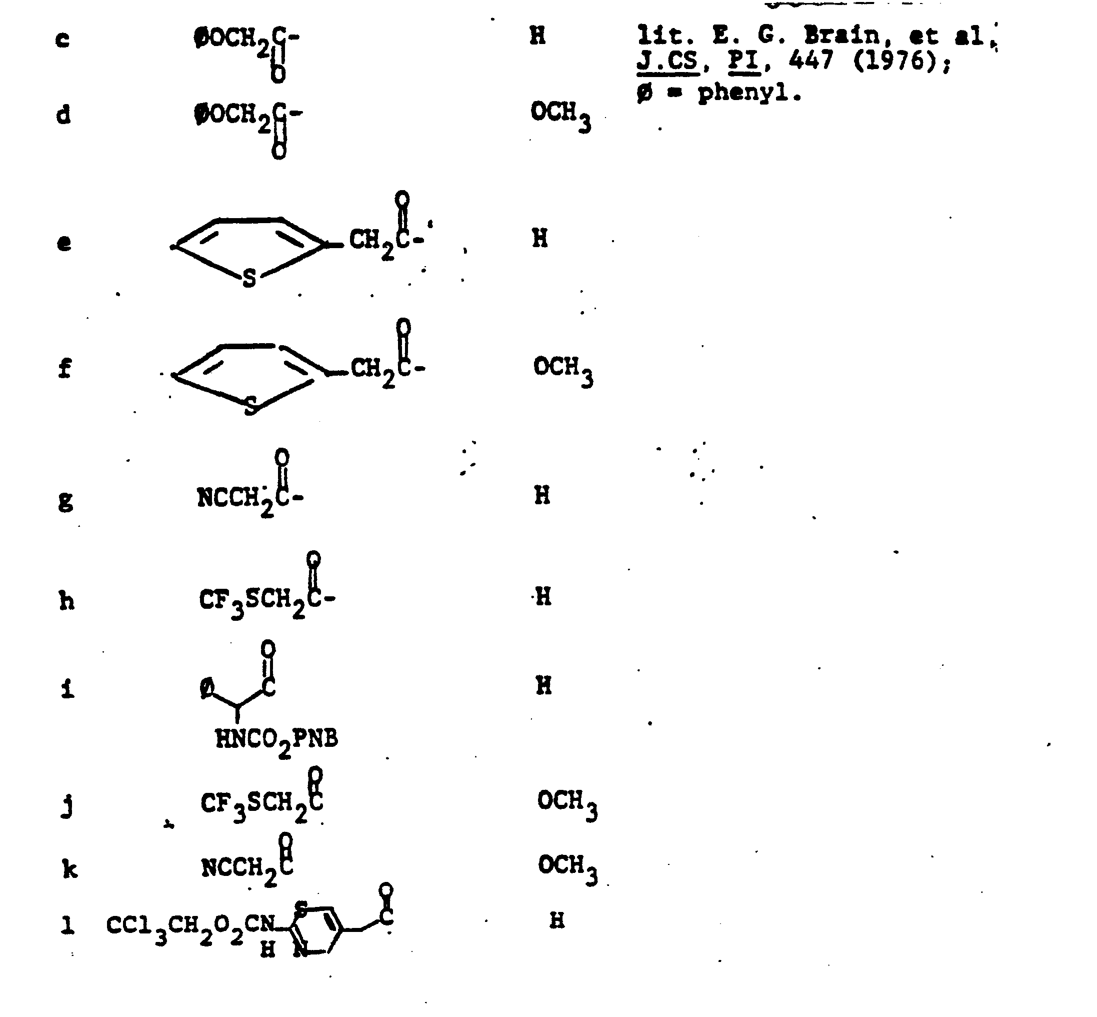

### EXAMPLE 5

### Preparation of 6-phenylacetamido-2-aminoethylthio-pen-2- em-3-carboxylic acid I

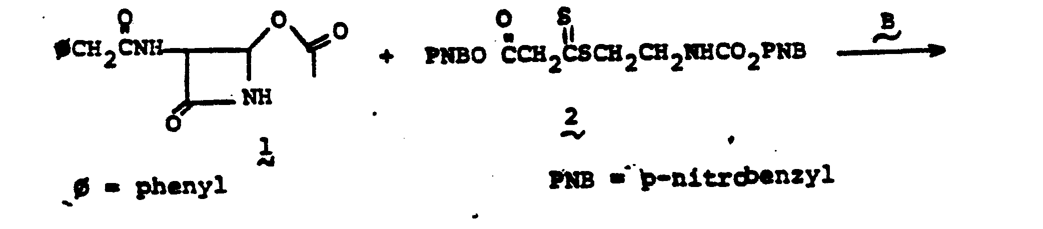

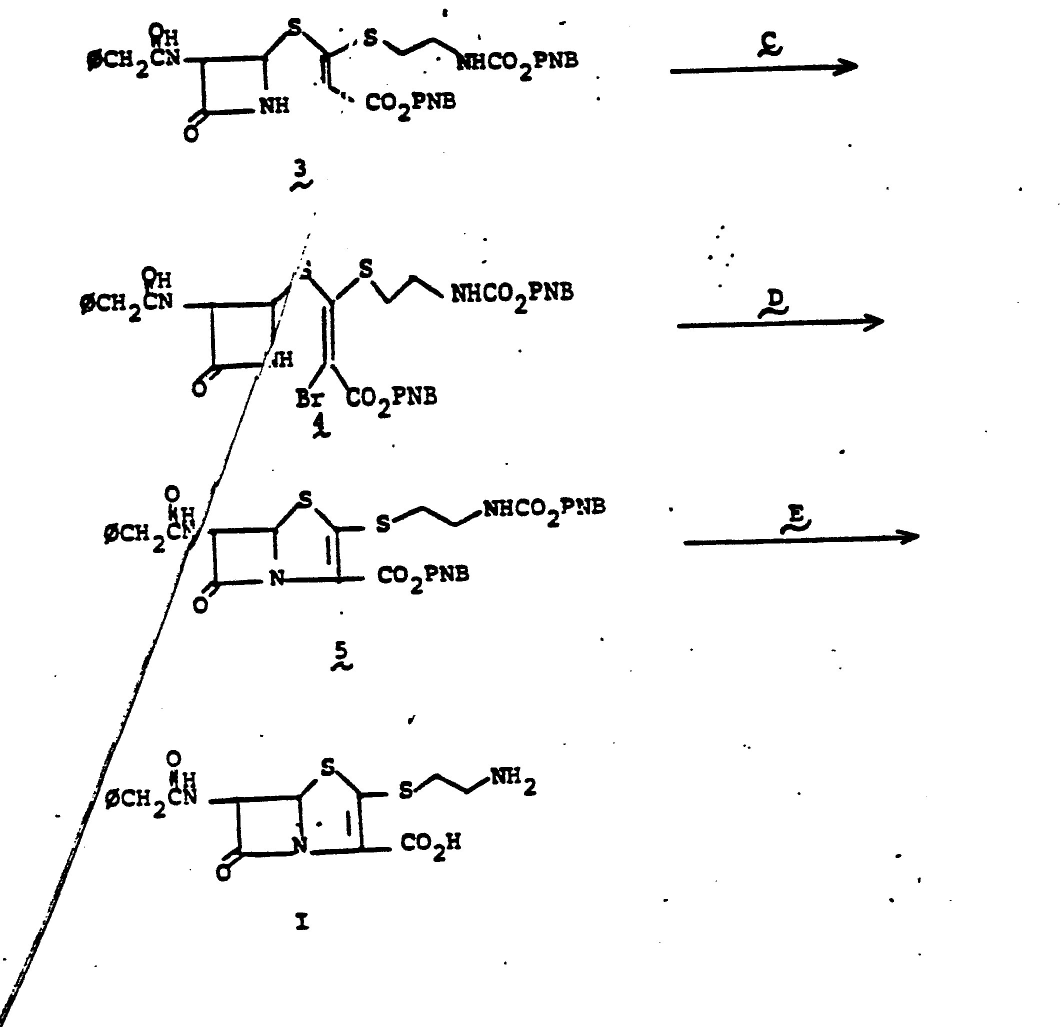

### /

### Preparation of 2

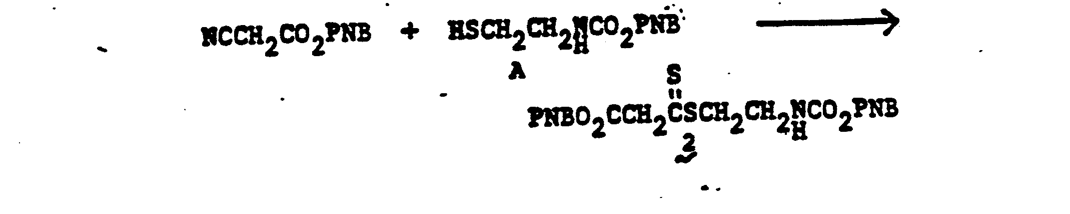

To a stirred solution of 3.77 g (17.2 mmol) of p-nitzobenzylcyanoacetate and 4.0 g (15,6 mmol) of N-p-nitrocbzcysteamine (A) in 40 ml dry benzene at 25°C under a nitrogen atmosphere is introduce a stream of HC1(g) for 7min. The reaction mixture is cooled on an ice-water bath and the introduction of HCl(g) continued for 3 min. The ice-water bath is removed and the mixture is stirred magnetically for 24 hrs. After this time, the solution is removed from the separated solid with the aid.of a filter stick and the solid is washed with dry benzene (2 x 40 ml) in the same manner. The solid is dried in vacuo in the reaction flask, then dissolved in dry dimethylsulfoxide (DMSO) at 25°C. To the stirred solution is added an excess of H₂S(g) under nitrogen atmosphere for 10 min. and the mixture is stirred at 25°C. for 1.0 hr. The resulting orange colored solution containing some suspended material is poured into 200 ml cold H₂0 and 200 ml Et₂0. The organic phase is separated and washed with H₂0 (4x) and saturated NaCl(aq) solution, then dried over MgSO₄, filtered and evaporated to give 3.4 g of residue. Purification is accomplished by chromatography on silica gel eluting with benzene:ethylacetate (4:1) to give 2.1 g (67% based on 59% conversion) of 2 as a yellow-orange oil which slowly solidifies; IR(CHC1₃) 34₀₀, 1720(sh), 171₀, 1601, 1520 cm⁻¹; _{NMR} (CDCl₃)δ: 3.45 (m, 4H)= 4.08 (s, 2H); 5.1 (bs, 1H): 5.17 (s, 2H); 5.27 (s, 2H); 7.42 (d, J=8Hz, 4H): 8.12 (d, J=BHa, 4H); mass spectrum m/e no 493(M⁺) 446, 256, 239, 209, 195, 165, 153, 136, 120.

### Step B:

### Preparation of Seco-Lactam

To a stirred mixture of 47.2 mg (0.18 mmol) of azetidinones 1 and 87.1 mg (0.18 mmaol) of dithioate 2 in 1.5 ml of dry TAF at 25°C is added in one portion 50.5 mg (0.25 mmol) of solid aluminum isoproproxiae [Al(O-)₃]. The mixture is stirred at 25°C under N₂ for 6.0 hr. and is then partitioned between EtOAc and a cold, aqueous solution of dilute HCl-tartaric acid mixture. The EtOAc phase is separated and is washed with saturated NaCl(aq.), dried MgSO₄), filtered, and evaporated. Purification by plate layer chromatography yields 3.

### Step C:

### Preparation of Bromide 4

To a stirred solution of lactam 3 (59.1 mg, 0.085 mmol) and 15.1 mg (0.085 mmol HMPA (hexamethylphoe- phoramide in 2.0 ml dry THF (tetrahydrofuran) at 25°C is added one portion 16.5 mg (0.093 mmol) solid NBS (N-bromosuccinimide). The mixture is stirred at 25°C under N₂ for 0.5 hr. and evaporated. The residue is partitioned between EtOAc and H₂O and the EtOAc phase is separated. It is further washed with H₂O (2x) and saturated NaCl (aq.), then dried (MgSO₄) filtered, and evaporated. Purification by repetitive plate layer chraaaatography yields 4.

### Step D:

### Preparation of penem 5

To a stirred mixture of 27.8 mg (0.04 mmol) of bromide 4 and 28 mg (0.14 mmol) of CuBr·S (CH₃)₂ in 5 ml dry THF at -78°C under N₂ is added 2ml of a cold, freshly prepared solution of lithium diisopropylamide, LiN( 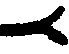 )₂, generated at 0°/20 min. with 5 mg (0.05 mmol) HN( 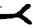 )₂ (diisopropylamine) and 19 µl of 2.4 M buLi (butyllithium). The mixture is stirred over the following temperature ranges for the times indicated: -78° to -74° (40 min.); -74° to -68° (30 min.); -68° to -57° (30 min.); -57° to -41° (20 min.); -41° to -25° (20 min.); -26° to -20° (28 min.); -20° to -13° (38 min.); -15° to -9° (53 min.); -10° to -5° (18 min.); and -5° to 0° (18 min.). The mixture is then treated at 0° with 1 ml of saturated NH₄Cl (aq.) and diluted with Et₂O/H₂O. The organic phase is separated and washed further with aqueous NN₄Cl and saturated NcCl (aq.). The organic layer is dried (MgS0₄), filtered, and evaporated. The residue is purified by plate layer chromatography to yield 5.

### Step E:

### Preparation of Penem I

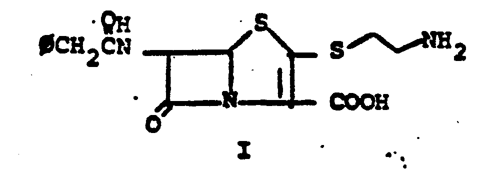

To 10 mg of 5 (Step D, above) is added 0.60 ml dioxane, 0.05 al ethanol, 0.35 ml deionized water and 0.01 ml of 1.0M K₂HPO₄. To the resultant clear solution is added 5 mg of 10% Pd/C. The suspension is flushed with N₂m then 5-6 times alternately with 50 psi H₂ and vacuum. Finally, it is shaken under a 50 psi H₂ atmosphere for 30-40 min. After centrifugation, the Pd/C is washed and centrifuged 2-3X with 0.5 ml portions of deionized water. The combined centrifugates are extracted 5x1-2 ml ether. Residual ether is removed under vacuum and the aqueous solution applied to an XAD-2 column (20 x 140 mm). Fractions of 100 drops (6-7 ml) are collected, with continuous UV monitoring, by elution with deionized water. The chosen fractions are combined and lyophilized to provide I.

### EXAMPLE 6

Preparation of

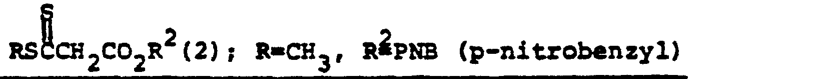

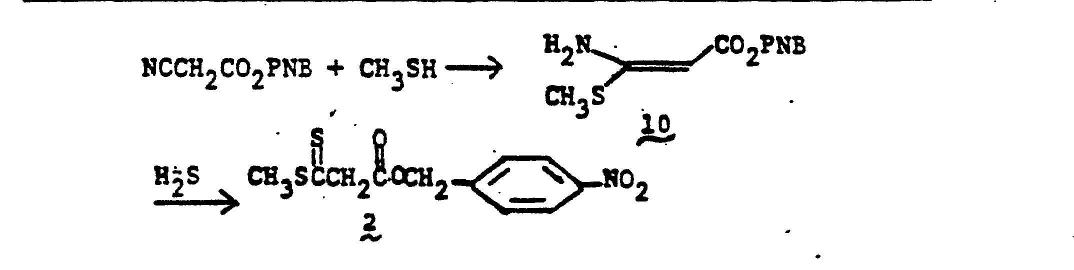

To a stirred mixture of 11.0 g (.05 mol) of p-nitro- benzylcyanoacetate and 3.72 g (.078 mol) methylmercaptan in 200 ml of sodium dried benzene at 25°C is introduced a stream of BCl(g) until 1.85 g (.05 mmol) is added. The mixture is then stirred at 25°C under nitrogen atmosphere for 74.5 hrs. The solution is decanted away from the separated solid and the moist precipitate is dissolved in 50 ml of dry pyridine. (Alternatively the precipitate is dissolved in dry dimethylsulfoxide and treated with H₂S(g) as described below to afford 2 directly.) The pyridine solution is treated with excess H₂S(g) and is stirred at 25°C under nitrogen atmosphere for 2 hrs. The reaction mixture is poured into ice/water and extracted with ether. The separated extract is washed twice with water and twice with brine solution then dried over MgSO₄, filtered, and evaporated to give a crystalline mass. Recrystallization from ether gives 5.0 g of the enamide 10; ir(CHCl₃), 3636, 3322, 1664, 1592, 1517 cm⁻¹; NMR(CDCl₃)δ: 2.4(s, 3H), 4.68 (s, 1H); 5.17 (s, 2H); 6.43 (bs, 2H); 7.43 (d, J=8Hz, 2H); 8.13 (d, J-8Hz, 2H); mass spectrum m/e 268 (M⁺), 221, 175, 136. Purification of the mother liquors from above by column chromatography on silica gel eluting with CHC1₃- pet-ether (1:;) provides 940 mg of 2 as an orange oil: ir (CHC1₃) 1742, 1613, 1522; NMR (CDCl₃)δ 2.67 (s, 3H); 4.13 (s, 2H); 5.23 (s, 2H); 7.47 (d, J=9Hz, 2H); 8.17 (d, J-9Hz, 2H); mass spectrum m/e 285 (M⁺), 238, 136, 106. The enamide 10 is converted to 2 by the following procedure.

The crude enamide 10 7.9 g (29 mmol) is dissolved in 40 ml of dry DMF (dimethylformamide) at 25°C and is treated successively with 3.7 g (32 mmol) of trifluoroacetic acid and excess hydrogen sulfide. The resulting mixture is stirred magnetically at 25°C under CaCl₂ drying tube protection for 5 hrs. The mixture is poured into 75 ml ice-H₂O and extracted with 100 ml Et₂0 (diethylether). The separated etheral extract is washed twice with H₂0 and saturated NaCl(aq) solution, then dried with MgSO₄, filtered, and evaporated. Purification by column chromatography on silica gel eluting with CHCl₃-petroleum ether (1:1) gives 6.55 g (74%) of 2.

### EXAMPLE 7

Preparation of dithioate derivative 2,

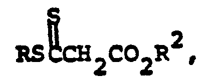

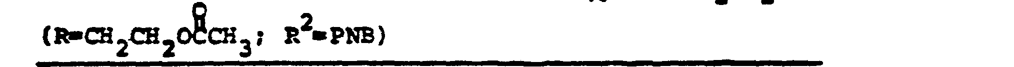

To a stirred mixture of 6.78 g (5.6 mmol) of 2-acetoxyethyl mercaptan and 11.0 g (5 mmol) p-nitro- benzylcyanoacetate in 100 ml of dry benzene is introduced 3.0 g (8.1 mmol) of hydrogen chloride gas. The mixture is stirred under N₂ at RT (25°C) for 42 hours after which time the solution is decanted away from the oily mass. The mass is washed twice with dry benzene by decantation. The oil is dissolved in 20 ml of dry dimethylformamide and is treated with a vigorous stream of hydrogen sulfide (g) for 5 min. The mixture is stirred at 25°C for 5 hours and then is poured into ice cold water and is extracted with ether. The ether extract is washed well with water, then brine, and is dried over MgSO₄, filtered, and evaporated. Purification by column chromatography on silica gel provides the dithioate derivative 2 as an orange solid; ir (CHCl₃) 1770, 1600, 1538 cm⁻¹; nmr (CDCl₃)δ: 2.03, 3H(s); 3.53, 2H(t,J-6Hz); 4.1, 2H(s); 4.27, 2H(t,J=6Hz); 5.25, 2H(s); 7.46, 2H(d,J=9Hz); 8.2, 2H(d,J=9Hz); mass spectrum m/e 357(M⁺), 297, 271, 206, 169, 136.

### EXAMPLE 8

Preparation of Beuzyl-thiobenzoylacetate 2; 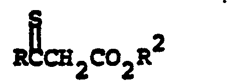 (R = phenyl; R²= benzyl) :

Into a stirred solution of 19.2 g of benzylaceto- acetate in 150 ml of acetonitrile at -60°C is passed a stream of hydrogen sulfide gas for 1.5 hours, followed by dry HCl(g) for 40 min. The reaction solution is then let warm to -20°C and is poured into ice water. The product 2 is extracted into benzene and purified by vacuum distillation to provide the known benzyl-thiobenzoylacetate, bp 09° (0.05 mm).

### EXAMPLE 9

Following the procedure described in the foregoing text and Examples, the following starting reagents 2 necessary for the preparation of the compounds of the present invention are representatively obtained by analogy.

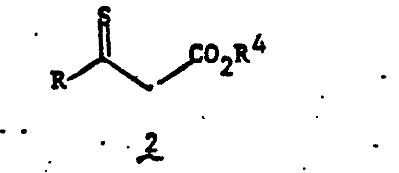

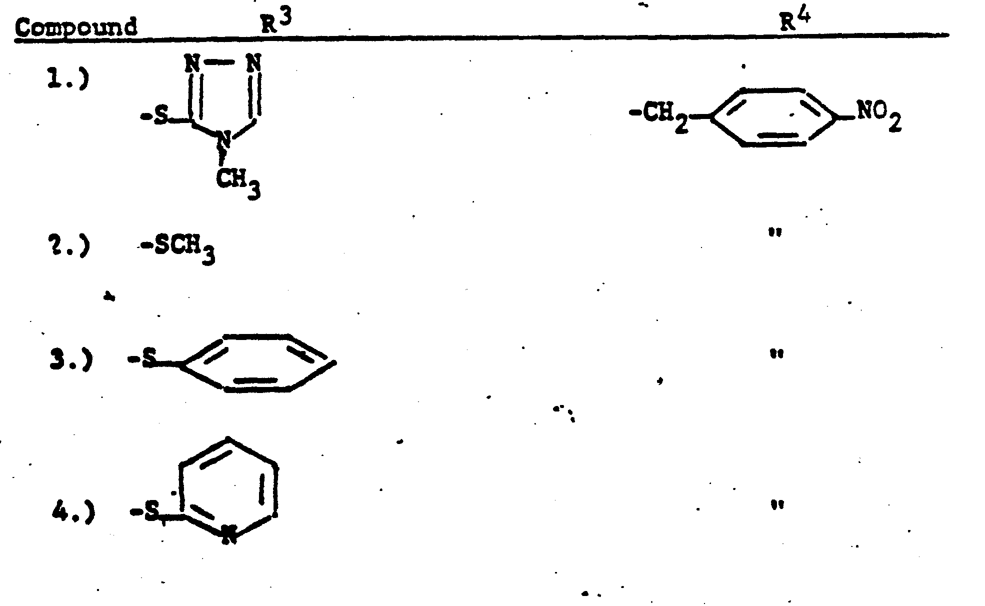

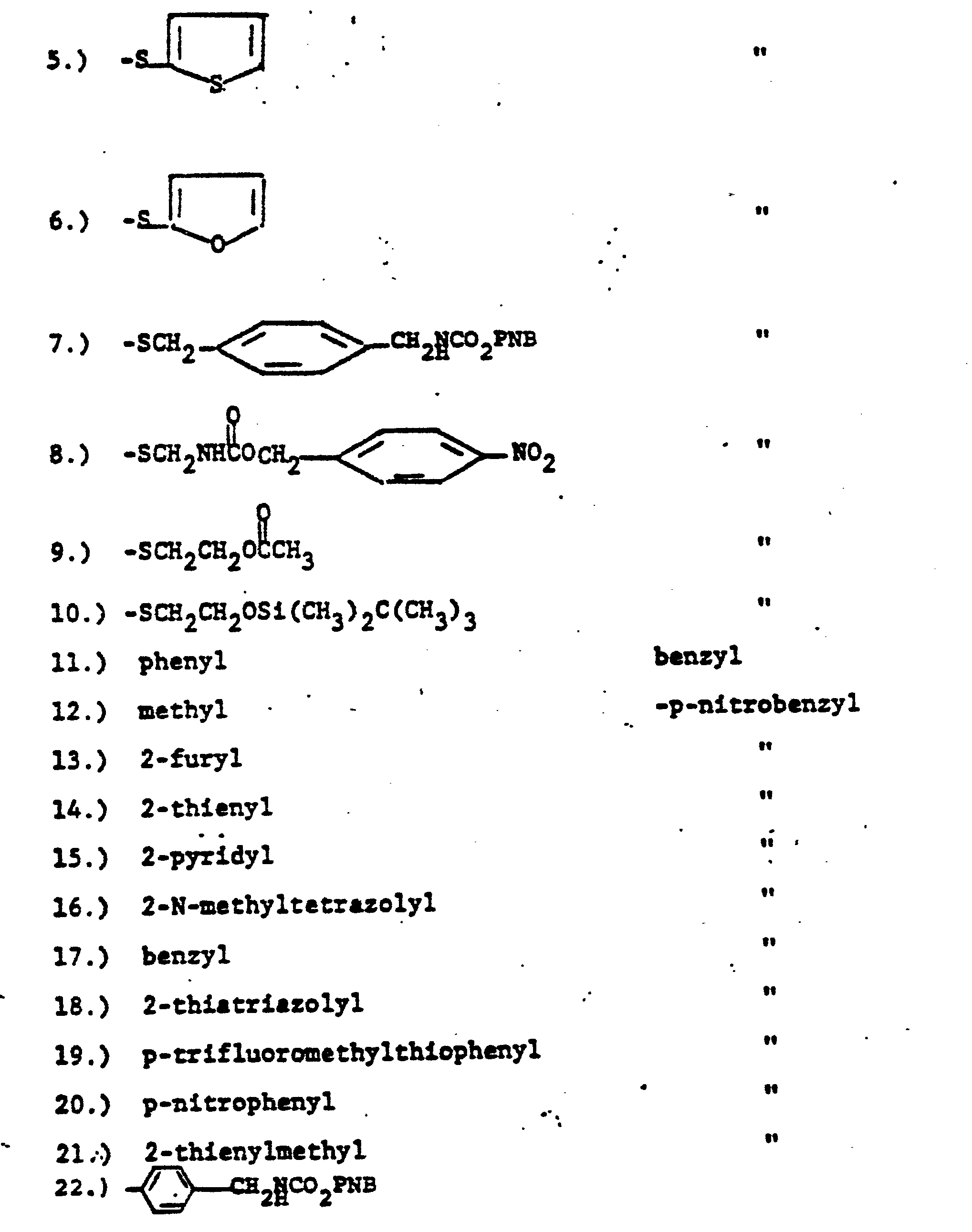

### EXAMPLE 10

### Preparation of Seco-lactam

To a stirred mixture of acetoxyazetidinone (Example 3) (95.1 mg., 0.36 mmol) and 139.2 mg (0.49 mmol) of the dithioate rsagent 2 of Example 6 (compound 2) in 5 ml methanol at 25°C is added 22 mg (0.4 mmol) sodium methoxide. The mixture is stirred under N₂ for 17 hours and an insoluble product is collected by filtration to provide 22 mg (12.5%) of a single trans olefin isomer 3: nmr (d₆-DMSO 2.43, 3H(s); 3.5, 2H(s); 4.7, 1H(m); 5.3, 2H(s); 5.36, lH(d, J=2Hz); 5.83, 1H(s); 7.3, 5H(s); 7.67, 2H(d,J=8Hz); 8.27, 2H(d,J=BHz); 8.9, 1H(d,J=8Hz); 9.03,1H(bs); mass spectrum m/e 470(M⁺-17); 442, 352, 285, 202, 159, 136, 106, 91. The filtrate above is evaporated and the residue is partitioned between EtOAc/brine. The organic is separated, dried (MgSO₄), filtered and evaporated. Purification, by plate layer chromatography [1 development φH-EtOAc(1:1)] provides two separable product fractions: 28.5 mg of more non-polar material as a mixture of isomers: nmr (CDC1₃) 2.37 and 2.43, 38(s); 3.57, 2H(s); 4.77, 1H(m): 5.2, 2H(s); 5.3, 1H(m); 5.63 and 5.83, 1H(s); 6.6, 1H(m); 6.87, 1H(m); 7.23, 5H(s); 7.5, 2H(d,J=8Hz); 8.2, 2H(d,J=8Hz); mass spectrum m/e 470 (M⁺ -17), 442, 352, 285, 202, 159, 136, 91; and 37.4 mg of more polar material as a mixture of isomers: nmr (CDC1₃) 2.27, 38(s); 3.53, 28(s); 5.23, 2H(s); 5.6, 3H(m); 7.07, 2H(m); 7.23, 5H(s); 7.5, 2H(d,J=8Hz); 8.2, 2H(d,J=8Hz). Total yield of isomers 3 is 88 mg (50%).

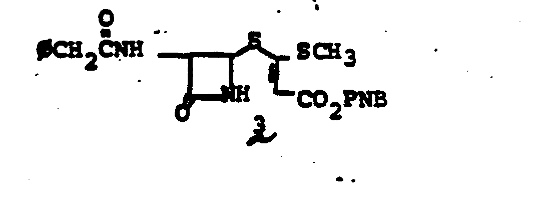

### EXAMPLE 11

### Preparation of Bromide 4

To a stirred solution of 50.5 mg (.104 mmole) of non-polar lactams 3 of Example 10 and 18 mg (0.1 mmole of hexamethylphosphoramide in 2 ml of dry tetrahydrofuran at 25°C is added 17.8 mg (0.1 mmole) of N-bromosuccinimide. The mixture is stirred at 25°C under N₂ for 0.5 hours, after which time the solvent is evaporated. The residue is purified by plate layer chromatography to yield the desired bromide 4.

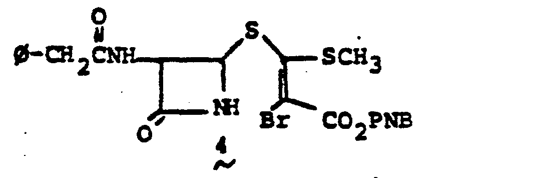

### EXAMPLE 12

### Preparation of p-Nitrobenzyl-6-phenylacetamido-2-thiomethyl pen-2-em-3-carboxylate (5)

To a stirred solution of bromide derivative 4a (56.6 mg, 0.1 mmol) and cuprous bromide-dimethylsulfide complex (67.7 mg, 0.33 mmol) in 10 ml of dry tetrahydrofuran at -78°C under N₂ is added a cold, freshly propared solution of lithium diisopropylamide (0.11 mmol) in 4 ml dry tetrahydrofuran. The mixture is stirred from of 8°C to 0°C over a 6 hour period. At 0°C the mixture is treated with 2 ml of saturated, aqueous ammonium chloride solution..The mixture is then partitioned between ether and water and the organic phase is separated. The organic extract is washed successively with saturated, aqueous ammonium chloride solution. the mixture is then partitioned between ether and water and the organic phase is separated. The organic extract is washed successively with saturated, aqueous ammonium chloride and brine followed by drying (MgSO₄), filtering, and evaporating. The residue so obtained is purified by plate layer chromatography to provide penem derivative 5.

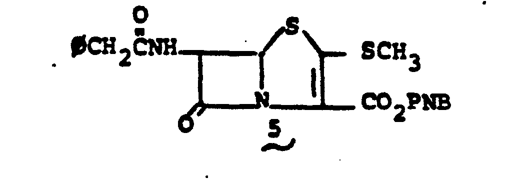

### EXAMPLE 13

### Preparation of Sodio-6-phenylacetamido-2-thio-methyl- pen-2-em-3-carboxylate (I)

A mixture of 20 mg of 10% Pd/C, 10 mg (o.2 mmol) of p-nitro-benzyl-6-phenylacetamido-2-thiomethyl pen-2-em-3-carboxylate (5 of Example 12) and 1.7 mg of sodium bicarbonate in 1 ml of ethylacetate, 0.2 ml isopropanol, and 1 ml of 0.5N pH 7 phosphate buffer is hydrogenated at RT and 40 psi in a Parr shaker for 15 minutes. The catalyst is removed by filtration and is washed thoroughly with deionized water. The filtrate is collected in a cold receiver. The cold filtrate is thoroughly extracted with ethyl acetate and the phases are separated. The aqueous phase is acidified to pH~2.5 with cold 1M ph~2.0 phosphate buffer and is then extracted with cold ethyl acetate. The separated ethyl acetate extract is than shaken with an aqueous solution of sodium bicarbonate (7 mg, 0.02 mmol). The aqueous phase is separated and lyophylized to provide sodio-6-phenyl-acetamido-2-thio- methylpen-2-em-3-carboxylate (I).

In cases where the_{.}above procedure produces zwitterionic species, the procedure is modified as follows: After the filtrate is extracted thoroughly with EtOAc, the cold aqueous phase is brought to a pH 7 and chromatographed on XAD-2 resin to afford the desired penem zwitterionic species.

### EXAMPLE 14

### Preparation of Sodio-6-phenylacetamido-6-Methoxyl-2-phenyl- pen-2-em-3-carboxylate

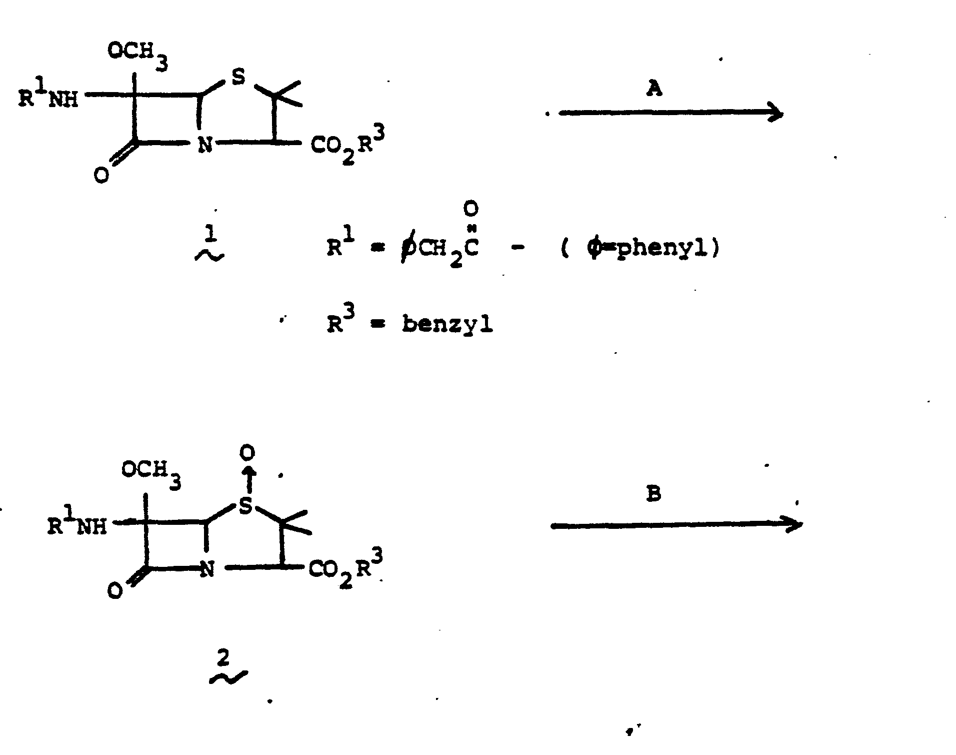

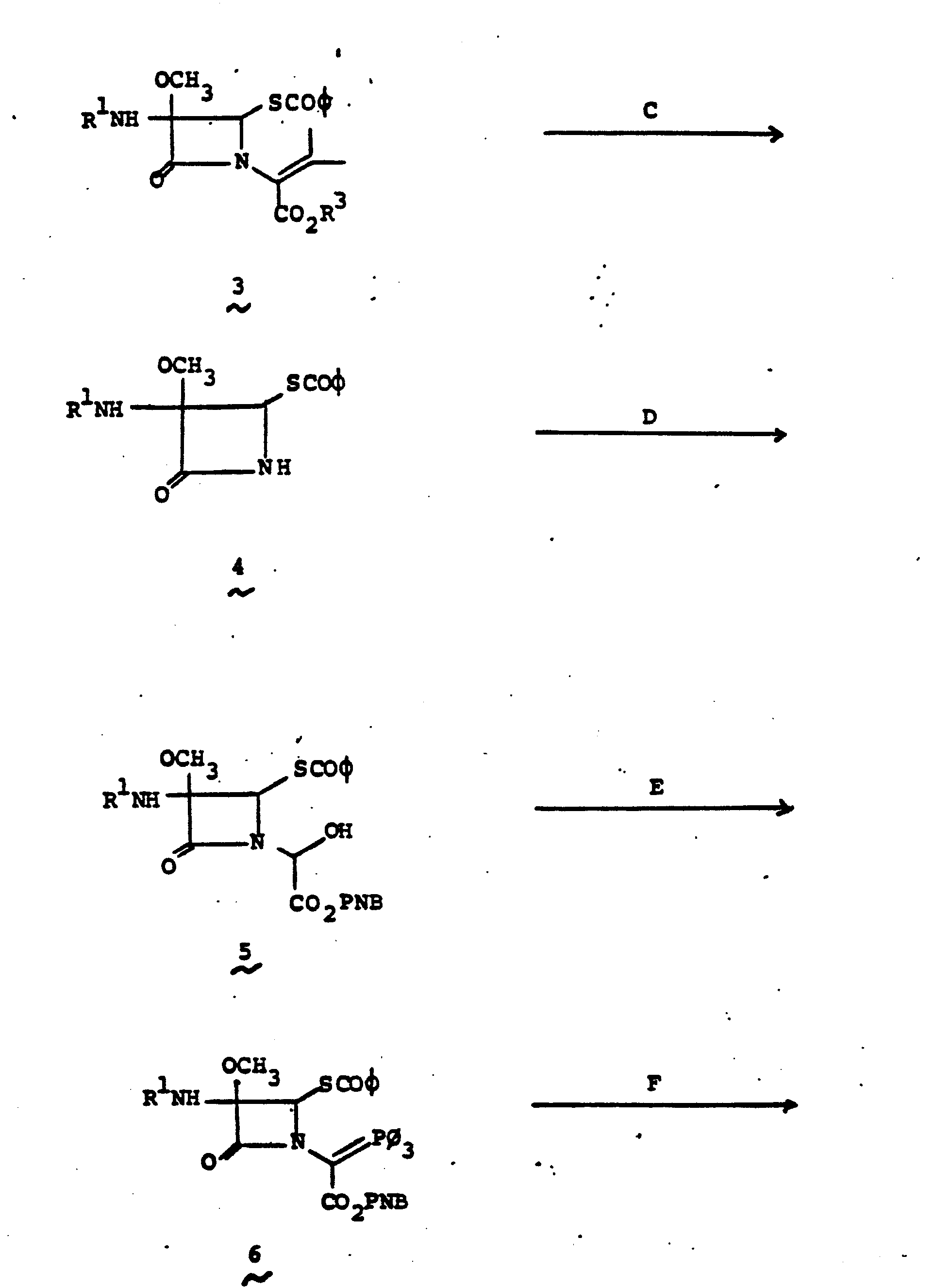

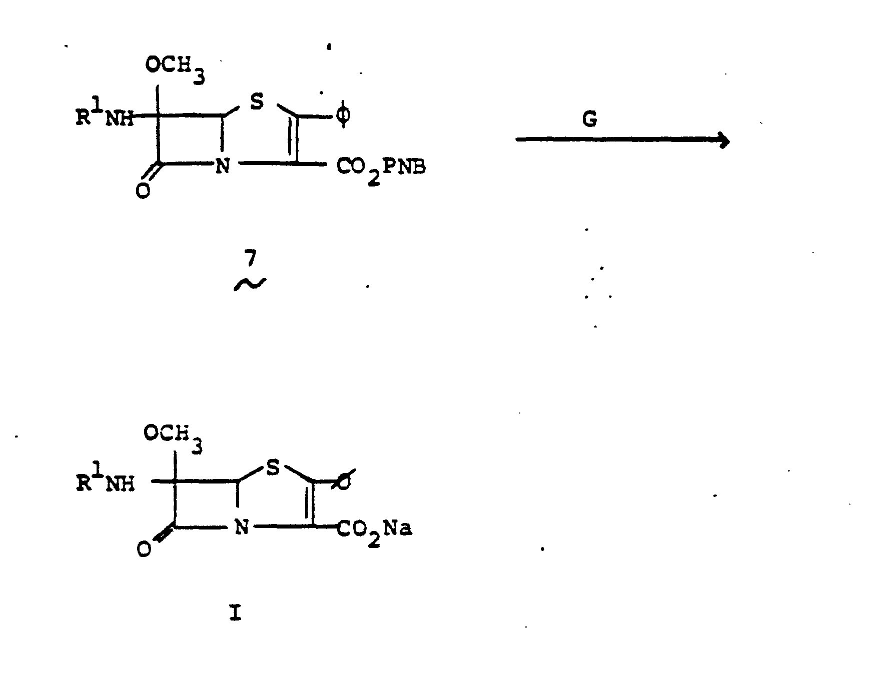

### Step A

To a stirred solution of 567.5 mg (1.25 mmoles) of benzyl-6-methoxy-6-phenyl acetamidope- nicillanate in 15 ml. methylene chloride at 25°C is added all at once 253.8 mg (1.25 mmoles) of 85% m-chloroperbenzoic acid. The mixture is stirred under an atmosphere of nitrogen at 25°C for 15 min. and is then partitioned between ethyl acetate and ice cold, aqueous sodium bicarbonate solution. The organic phase is separated, washed further with saturated, aqueous sodium chloride solution, dried (MgSO₄), filtered, and evaporated. The residue is purified by plate'layer chromatography (PLC)to provide the desired sulfoxide.

### step _{B}

A stirred mixture of 94 mg (0.2 mmoles) of the sulfoxide obtained in Step A, 49 mg (0.24 mmoles) of tri-n-butylphosphine, and 229 mg (1.0 mmoles) of benzoic anhydride in 3.0 ml of benzene is refluxed at 90°C under a nitrogen atmosphere for 7.0 hours. The cooled mixture is partitioned between ethyl acetate and cold, aqueous sodium bicarbonate and the organic phase is separated. The organic phase is washed with saturated, aqueous sodium chloride, dried (MgSO₄), filtered, and evaporated. Purification by PLC provides 3.

### Step C

To a stirred solution of 20.7mg (0.036 mmoles) of azetidinone 3 in 0.75 ml. of 8:1 acetone-water and two drops of 0.1N pH 7 phosphate buffer at 25°C is added all at once 5.7 mg (0.036 mmoles) of potassium permanganate. The mixture is stirred at 25°C under nitrogen for 8 min and then an additional 5.7 mg of potassium permanganate is added. The mixture is stirred further for 50 min. and is then cooled on an ice-water bath, diluted with ethyl acetate, and is treated with 5% aqueous sodium thiosulfate. The mixture is filtered through celite and is washed well with ethyl acetate. The filtrate is partitioned between ethyl acetate and cold, aqueous brine solution and the organic phase is separated, dried (MgSO₄), filtered, and evaporated. The residue is purified by PLC to give 4.

### Step D

A stirred mixture of 10.4 mg (0.027 mmoles) of azetidinone 4 (Step C) and a large excess of p-nitrobenzylglyoxalate hydrate in 1 ml of toluene and 0.5 ml of dimethyl formamide containing 500 mg of ground 3A molecular sieves is heated at 80°C under a nitrogen atmosphere for 5.0 hr. The cooled mixture is diluted with ethyl acetate and is filtered. The filtrate is concentrated on a rotatory evaporator and the concentrate is partitioned between diethyl ether and cold water. The organic phase is separated, washed further with water, dried (MgSO₄), filtered, and evaporated. The residue is purified by PLC to give 5.

### Step E

To a stirred solution of 11.9mg (0.02 mmoles) of lactam 5 from Step D in 1.0 ml anhydrous tetrahydrofuran at 0°C under a nitrogen atmosphere is added in rapid succession 2.4 mg (0.02 mmoles) of neat thionyl chloride and 2.0 mg (0.02 mmoles) of neat triethyl amine. The mixture is stirred at 0°C for 2.0 hours and is then partitioned between diethyl ether and : cold, aqueous brine. The organic phase is separated, washed with saturated, aqueous sodium chloride, dried (MgSO₄), filtered, and evaporated. The residue so obtained is dissolved in 0.5 ml dry tetrahydrofuran and is stirred with 10.0 mg (0.04 mmoles) of triphenyl- phosphine at 54°C under a nitrogen atmosphere for 18.0 hrs. The cooled mixture is partitioned between ethyl acetate and aqueous sodium bicarbonate solution. The organic phase is separated, dried (Na₂SO₄), filtered, and evaporated. Purification of the residue by PLC affords the phosphorane derivative 6.

### Step F

A stirred solution of 83.9 mg (0.1 mmole) of phosphoraneazetidinone (Step E) in 80 ml of toluene , -is heated at 85°C under an atmosphere of nitrogen for 3.5 days. The toluene is removed under reduced pressure and the residue is purified by plate layer chromatography to provide penem 7.

### Step G

A mixture of 56.1mg (0.1 mmoles) of penem 7, 150 mg of 10% palladium-on-charcoal, and dilute, aqueous sodium bicarbonate in 5 ml. of ethyl acetate is hydrogenated at 25°C and atmospheric pressure for 30 minutes. After this time 25 mg of fresh catalyst is added and the mixture is hydrogenated for an additional 5 minutes. The catalyst is removed by filtration and washed with dilute, cold aqueous sodium bicarbonate and ethyl acetate. The organic phase is separated and the aqueous phase acidified with dilute, cold aqueous citric acid. Thorough extraction of the acidified aqueous phase with methylene chloride, followed by drying (Na₂SO₄) and evaporation, provides the penem carboxylic acid 8, which is immediately converted to its dodium salt by treatment with a stoichiometric amount of aqueous sodium bicarbonate in acetone solution, evaporation of acetone, and lyopholization of the aqueous solution.

### EXAMPLE 14A

### Preparation of:

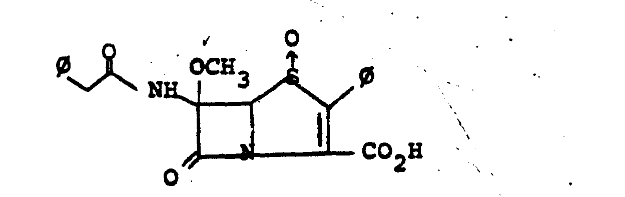

To a stirred solution of 0.1 mmoles of penem 7 from Example 14, Step F, in 2 ml of methylen chloride at 25°C is added 20.3 mg (0.1 mmoles) of 85% m chloroperbenzoic acid. The mixture is stirred at 25°C under a nitrogen atmosphere for 0.5 hours and then is partitioned between methylene chloride and and cold, dilute aqueous sodium bicarbonate. The organic phase is separated, dried (Na₂SO₄), filtered and evaporated to yield the desired sulfoxide.

The above illustrated carboxylic acid is obtained as described in Step G of Example 14 when the indicated substitution is made by the catalytic hydrogenation of the sulfoxide prepared above.

### EXAMPLE 15

Following the procedure of the foregoing Examples and text, the following species of the invention are obtained by analogy:

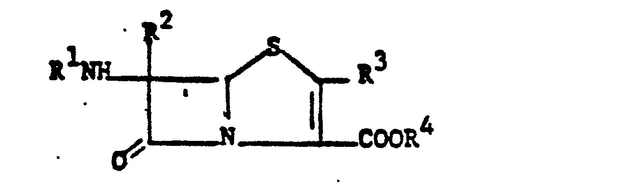

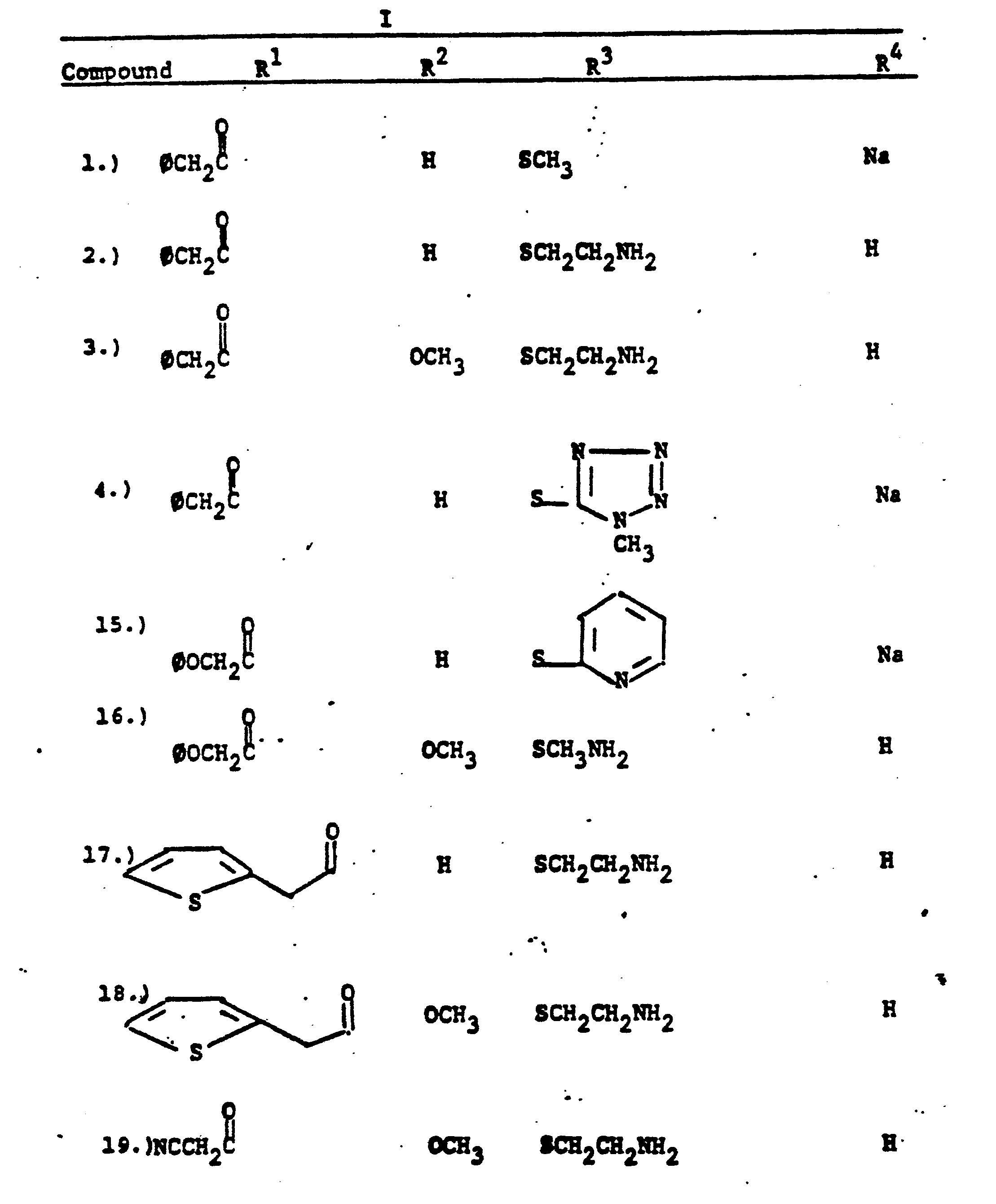

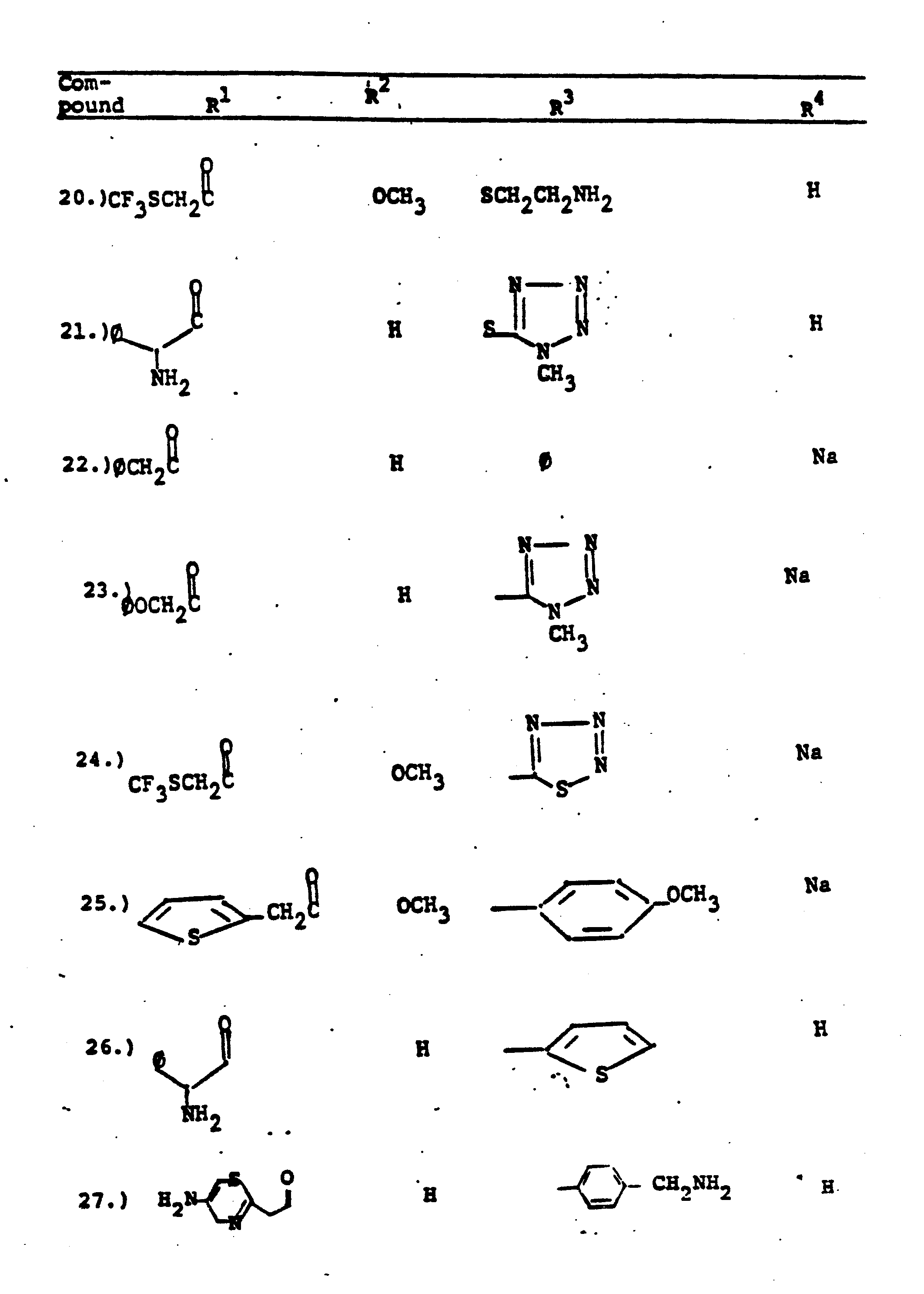

### SECTION D

### EXAMPLE 1

Preparation of Starting Material 6

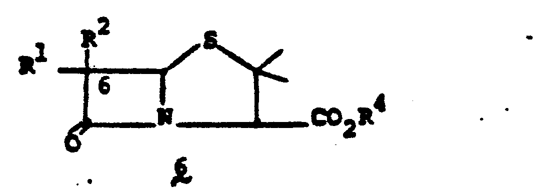

wherein R¹, R² and R⁴ are as defined above. In general, the compounds 6 are prepared according to the method of DiNinno, et al., [F. DiNinno, T.R. Beattie, and B.G. Christensen, J.Org. Chem., 42, 2960 (1977)] which is summarized in Schemes I and II: 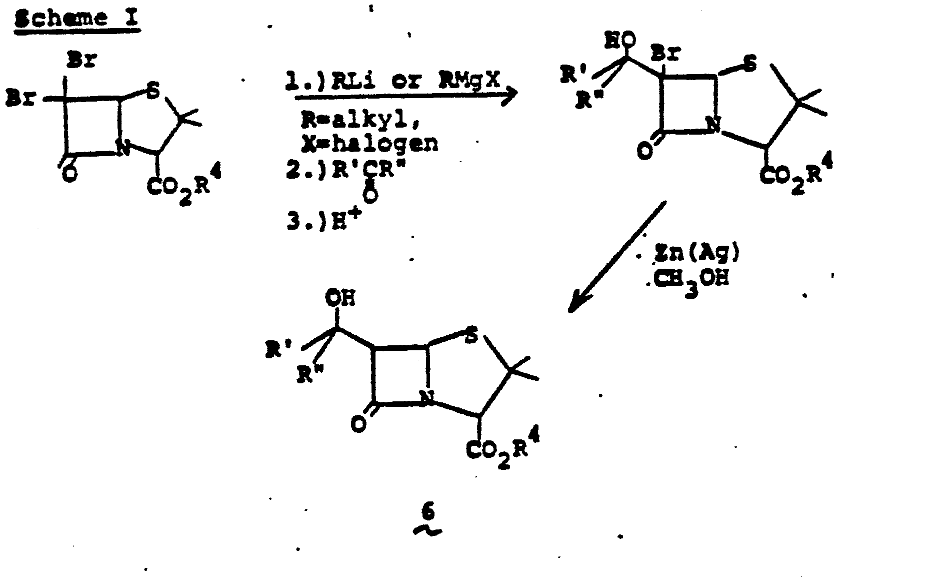 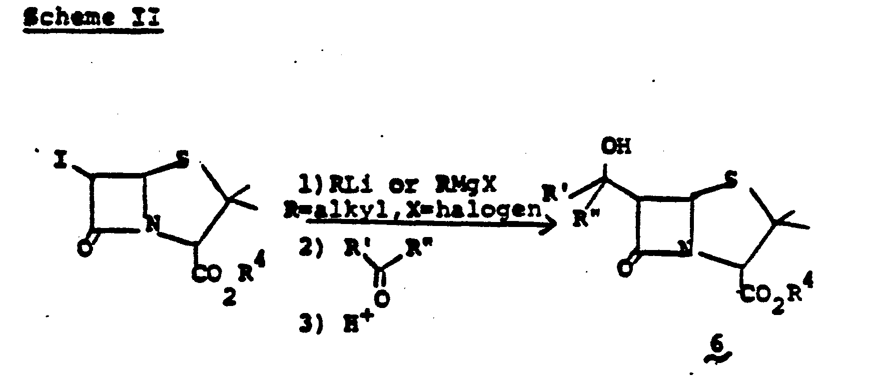 wherein R' and R" may be hydrogen, alkyl, aralkyl, heterocyclyl and heterocyclylalkyl.

The substituents at ring position 6 correspond in this Example I to the generically defined substituent R¹ and R² of Structure I, above. Thus, radicals such as

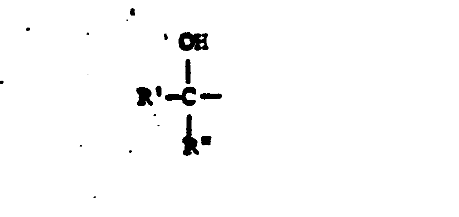

and -OR (below) encountered hers in this Example 1 at position 6 are but convenient specific demonstrations of the generic radicals R¹ and R² as defined above for Structure I, the ultimate compounds of the present invention.

It is to be noted that the above reaction scheme is regio-specific for the 6-position and that there are no criticalities of reaction parameters other than those set forth above and elaborated upon in the following specific examples. It should be further noted that the above-described procedure provides all embodiments of the present invention except those wherein one of the 6- substituents is alkoxyl such as methoxyl, for example; in that event, the above procedure is modified as illustrated in the following scheme:

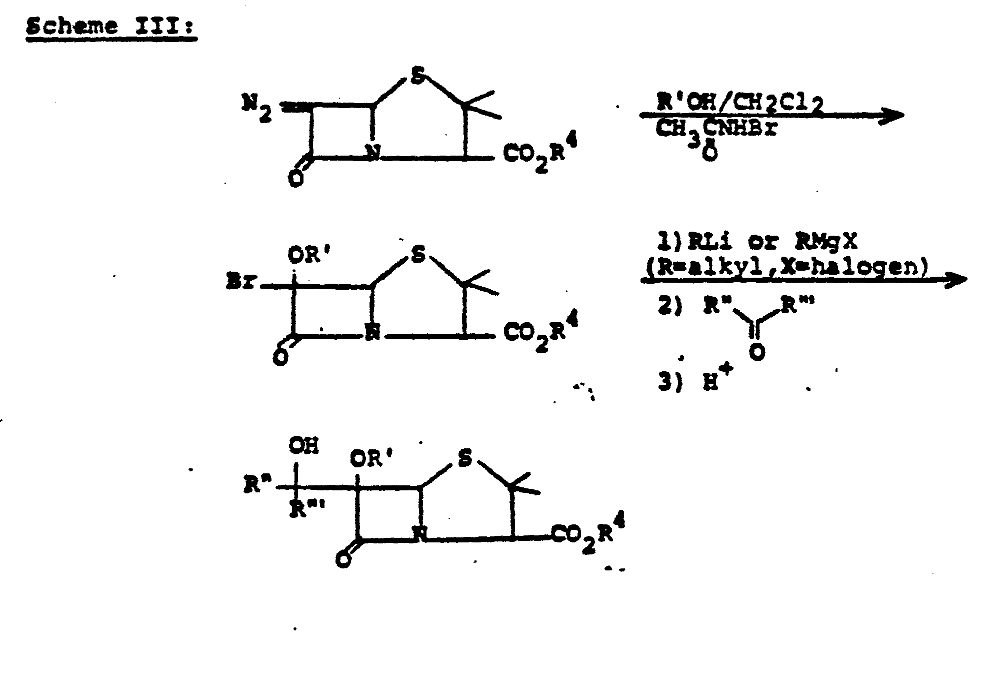

In Scheme III, the diazo starting material is available to the art; see for example: Hauser, et al., Helv, Chem. Acta. 50, 1327 (1967). Typically the first step of the reaction is conducted in a solvent medium such as R'OH or a mixture of R'OH and a solvent such as CH₂Cl₂, acetonitrile, benzene or the like containing 1 to about 3 equivalents of a brominating agent such as N-bromoacetamide, N-bromosuccinimide, or the like; typically the reaction is conducted at from about 0° to about 50°C for from a few minutes to 12 hours. The resulting 6-bromo-6-substituted species are known [Cama, et al., J. Amer. Chem. Soc.,95,1408 (1972)] as is the above described process; the 6-bromo-6-substituted species is then treated with a base (1.0 to 1.5 equivalents) such as an organo-metallic base,. for example, n-butyl lithium, methyl magnesium bromide, or the like in a solvent such as diethyl ether, tetrahydrofuran, dimethoxyethane or the like at a temperature of from -80°C to about 0°C: and thereafter adding, as above-described, the reagent of choice R'CR" to give the desired final product.

### EXAMPLE 2

### Preparation of bienzyl-6-methoxy-6-(1-hydroxyethyl)-penicillanate (6)

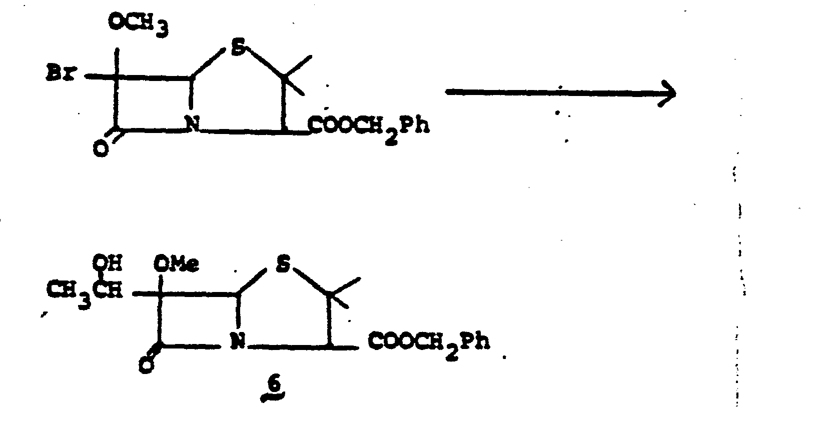

To a stirred solution of 90.8 mg. (0.2 mmol) of benzyl-6-methoxy-6-bromopenicillanate [prepared according to L.D. Cama, W.J. Leanza, T.R. Beattie, and B.G. Christensen, J. Amer. Chem. Soc., 95, 1408 (1972) i 5 ml of dry THF at -78° under a nitrogen atmosphere .s added dropwise 69 µl (o.2 mmol) of 2.9M ethereal mehyl- magnesium bromide. The mixture is stirred at -78° oder nitrogen for 0.5 hours and 57 µl (1 mmol) of neat acetalde- hyde is added. The resulting mixture is stirred at -78° for 0.5 hours and 0.5 ml. of saturated, aqueous NH₄(1 solution is added. The mixture is partitioned between Et₂0 and H₂0 and the organic phase is separated, dried (MgSO₄), filtered, and evaporated. Purification by plate layer chromotography affords benzyl-6-methoxy-6-(1-hydroxyethyl)penicillanate (6)

### EXAMPLE 3

Following the foregoing Examples (I, Ia) and text, the following useful starting materials 6 for the practice of the present invention are obtained by analogy.

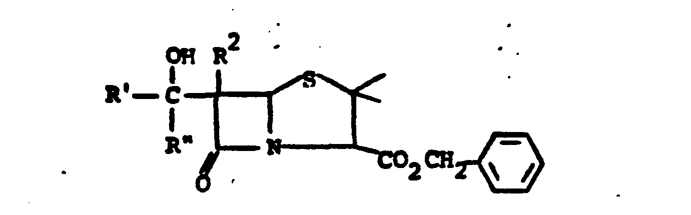

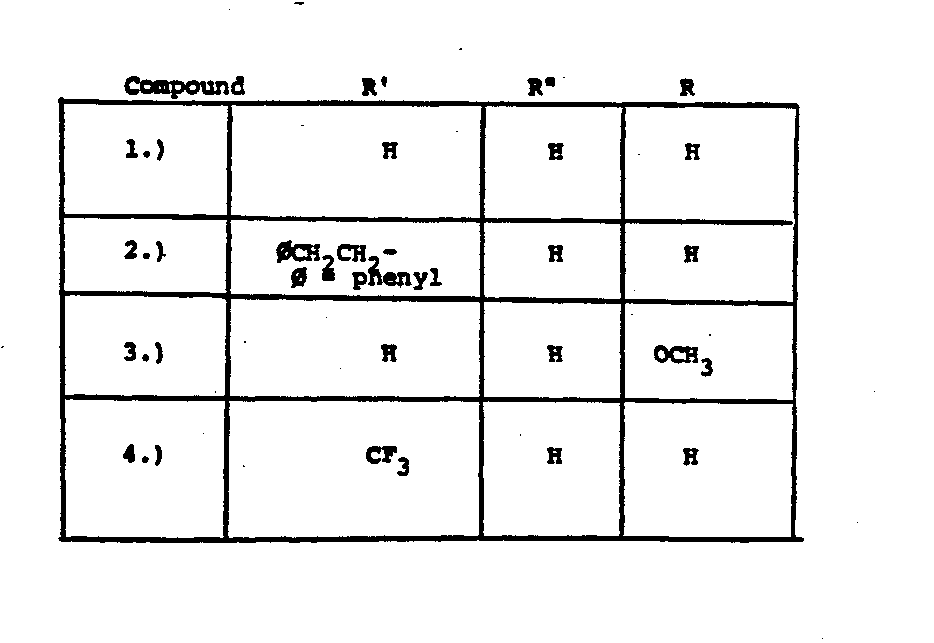

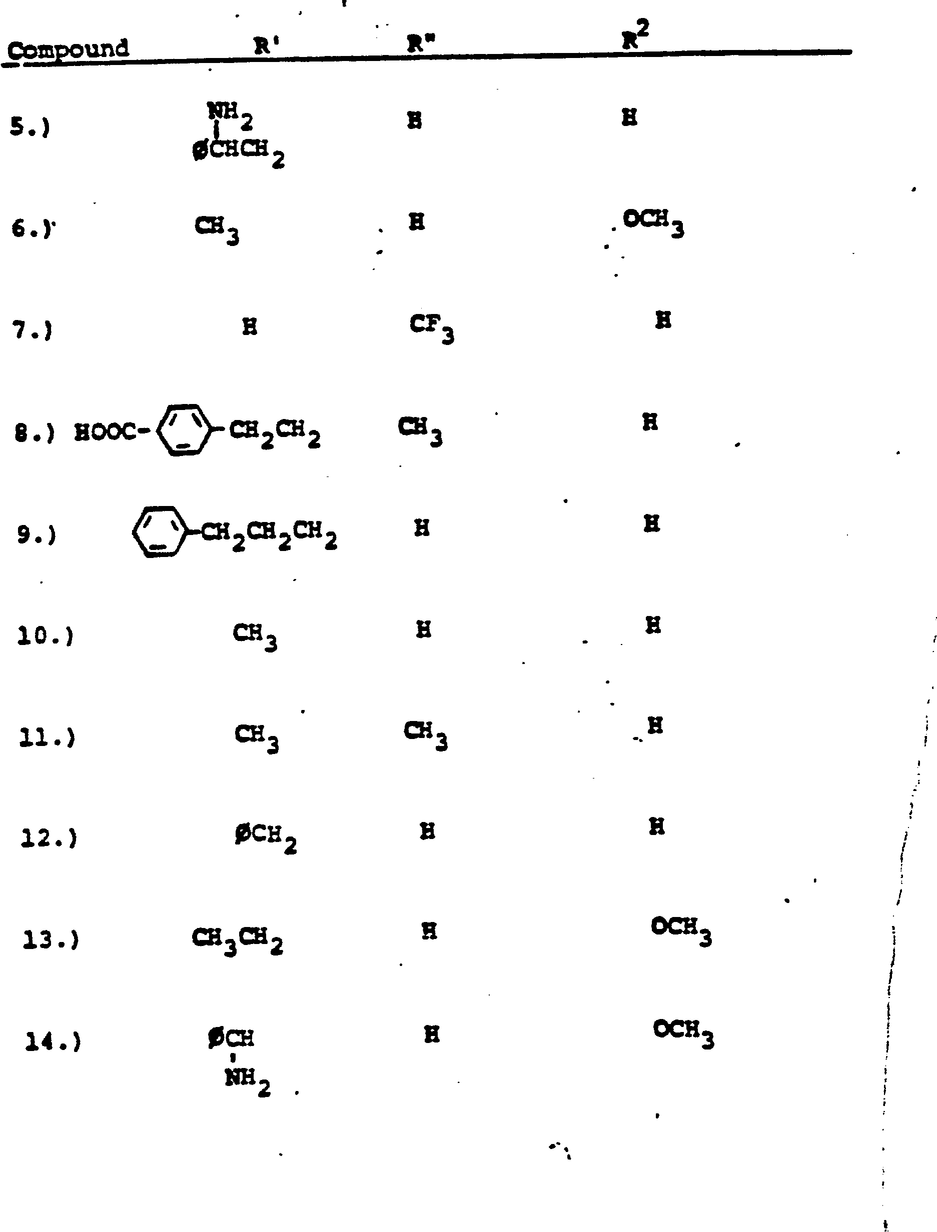

### EXAMPLE 4

### Preparation of Azetidinone 1

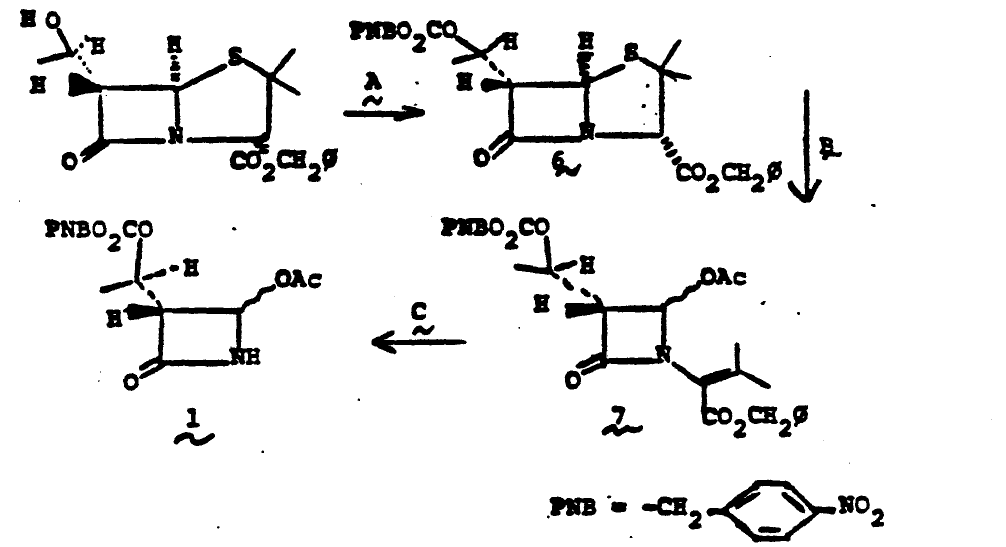

### Step A, Preparation of 6

To a stirred solution of 215 mg. (0.6 mmol) of benzyl-6α-((R)-1'-hydroxyethyl]penicillanate and 276 mg (0.13 mmol p-nitrobenzyl-chloroformate in 5 ml dry methylene chloride at 0°C is added in one portion solid 4-dimethylaminopyridine (156 mg., 0.13 mmole). The mixture is stirred at 0°C under a nitrogen atmosphere for 2 min. and allowed to warm over 28 min. The mixture is poured into ice-H₂O and extracted with CH₂Cl₂. The organic phase is separated and washed successively with cold, dilute, aqueous HCl and saturated NaCl (aq.). After drying with MgSO₄, the filtered solution is evaporated and dried in vacuo to give 420 mg of residue. Purification by plate layer chromatography [one dsvelopment C₆H₆-EtOAc(9:1)] provides 278.2 mg. (84%) of product 6; ir (CHCl₃) 1770, 1740 cm⁻¹; nmr (CDCl₃)δ1.4, 3H(S); 1.48, 3H(d, J=6Hz); 1.61, 3H(S); 3.46, 1H(dd, J-2, 7Hz); 4.5, 1H(S) 5.2, 2H(S); 5.23, 1H(m) ; 5.27, 2H(S); 5.31, lH(d, J=2Hz); 7.37, SH(S); 7.53, 2H(D, J=8Hz); 8.23, 2H(d, J=8HZ).

### Step B

### Preparation of 7

A stirred mixture of 186.8 mg (0.36 mmol) of 6 and 232 mg. (0.73 mmol) Hg(OAc)₂ (mercuric acetate) in 7 ml glacial HOAc (acetic acid) is heated at 90°C. for 1.5 hour under a nitrogen atmosphere. The cooled mixture is filtered through supercel, washing thoroughly with CH₂Cl₂. The filtrate is diluted with H₂0 and is neutralized with solid NaHC0₃. The mixture is extracted thoroughly with CH₂Cl₂ and the combined extracts are washed successively with saturated NaHCO₃ (aq.) and saturated NaCl (aq.). After drying with KgSO₄, the filtered solution is evaporated and the residue so obtained is purified by plate layer chromatography [one development CHCl₃-EtOAc(20:1)) to provide 138.6 mg (71%) of approximately a 1:1 mixture of cis and trans- acetoxyazetidinones 7; ir (CHCl₃) 1770, 1750, 1730cm⁻¹; nmr(CDCl₃)δ1.4 1.53 3H(d's, J's=6HZ 7Hz); 1.86, 1.96 & 2.0, 6H(S); 2.23, 3H(S); 3.4 & 3.58, 1H(dd's, J's=2,7Hz 4.10Hz); 5.2, 1H(m); 5.2, 2H(S); 5.23, 2H(S); 6.23 & 6.3, 1H(d's, J's=2 & 4Hz); 7.3, 5H(S); 7.53, 2H(d, J=8Hz); 8.2, 2H(J=8Hz); mass spectrum m/e 540(M⁺), 498, 390, 346, 301, 222, 210, 193, 136, 91.

### step C

### Preparation of 1

To a stirred solution of 138.6 mg (0.26 mmol) of azetidinones 7 in 3.5 ml of 8:1 (CH₃)₂CO-H₂O and 1 drop of pH 7 O.1N phosphate buffer at room temperature (25°C) is added 40.6 mg (0.26 mmol) of solid KMnO₄. The mixture is stirred under a nitrogen atmosphere at 25°C for 8 min. After this time, 40.6 mg (0.26 mmol) of additional KMno₄ is added and the mixture is stirred further for 45 min. The reaction mixture is diluted with EtOAc (ethylacetate) and treated with cold, aqueous Na₂S₂O₃ until the violet coloration of KMnO₄ is no longer apparent. The mixture is filtered through celite and is washed well with EtOAc. The filtrate is washed with saturated NaCl (aq.), dried (MgSO₄), filtered, and evaporated. Purification of the residue by plate layer chromatography [one development CHCl₃-EtDAC(3:1)] gives 65.5 mg. (72%) of the azetidinone mixture 1; ir (CHCl₃) 3300, 1785, 1750cm⁻¹; nmr (CDCl₃)δ,1.5 & 1.53, 3H(d's, J's-7Hz) 1.98 & 2.12, 3H(S); 2.43, (dd, _{J}-2, 6_{H}z) & 2.65, (dq,J=1.5, 4.0, 9Hz) [1H]; 5.28, 2H(S); 5.3, 1H(M); 5.88 & 5.95, 1H(d's, J's=1.5 & 4.0Hz); 6.93, 1H(bs); 7.57, 2H(d, J=8Hz)= 8.25, 2H(d, J=8Hz); mass spectrum m/e 309, 292, 249, 181, 154, 136.

### EXAMPLE 5

Following the procedure of Example 4 the following 3-and 3,3-disubstituted-4-acetoxy azetidinones 1 are prepared by analogy.

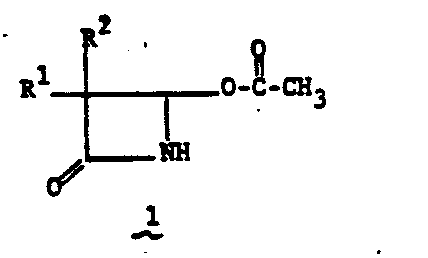

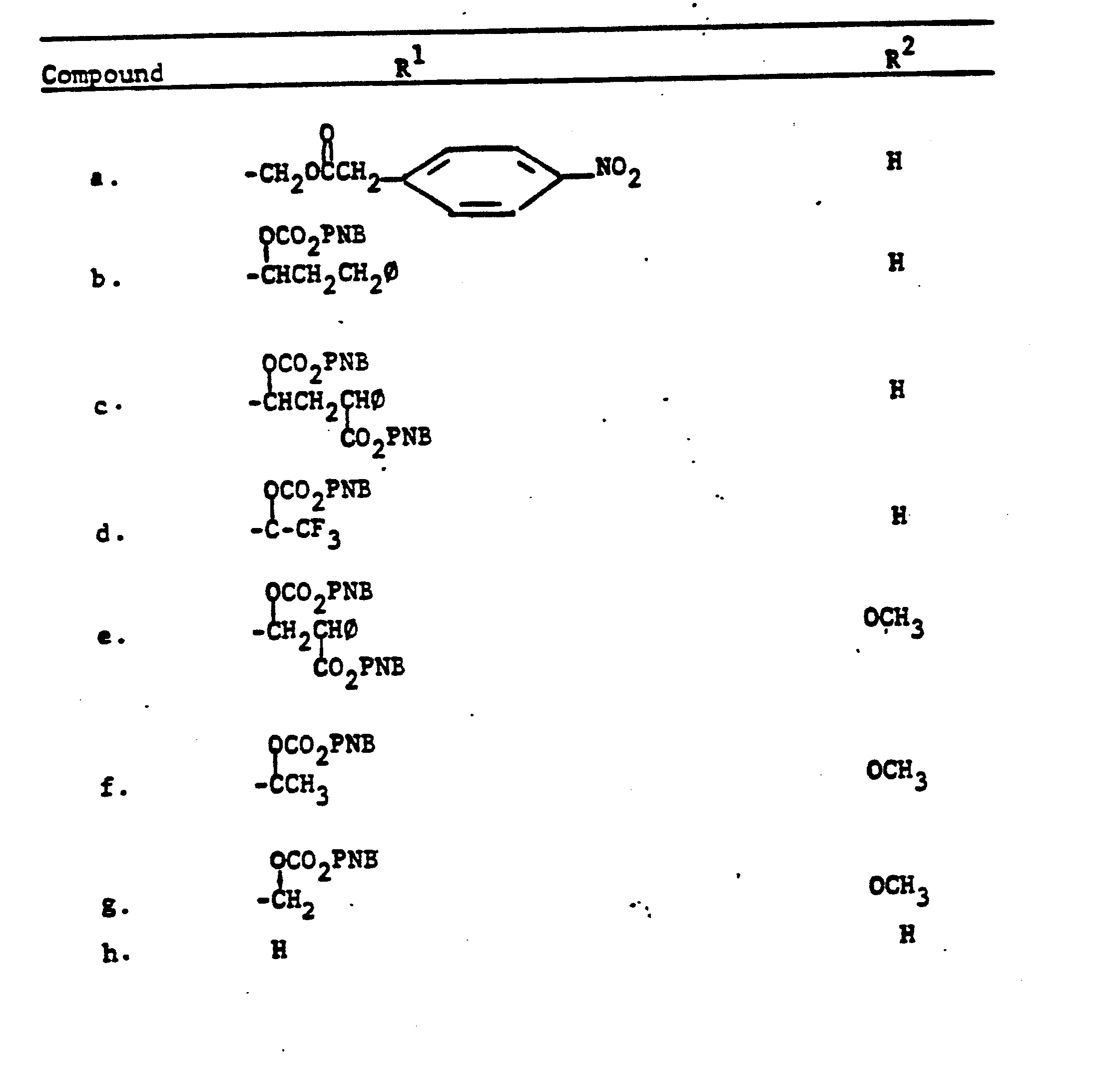

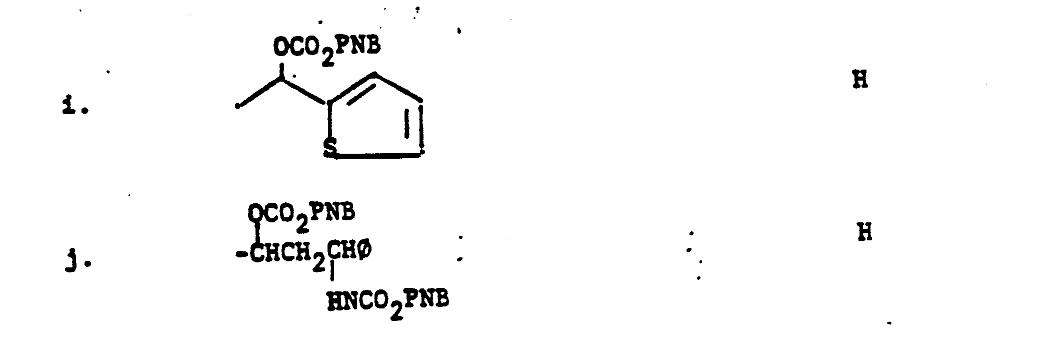

PNB = p-nitrobenzyl φ = phenyl

### EXAMPLE 6

### Preparation of 6-α-[(R)-l'hydroxyethyl]-2-aminoethylthio-Pen-2-em-3'-carboxylic acid (I)

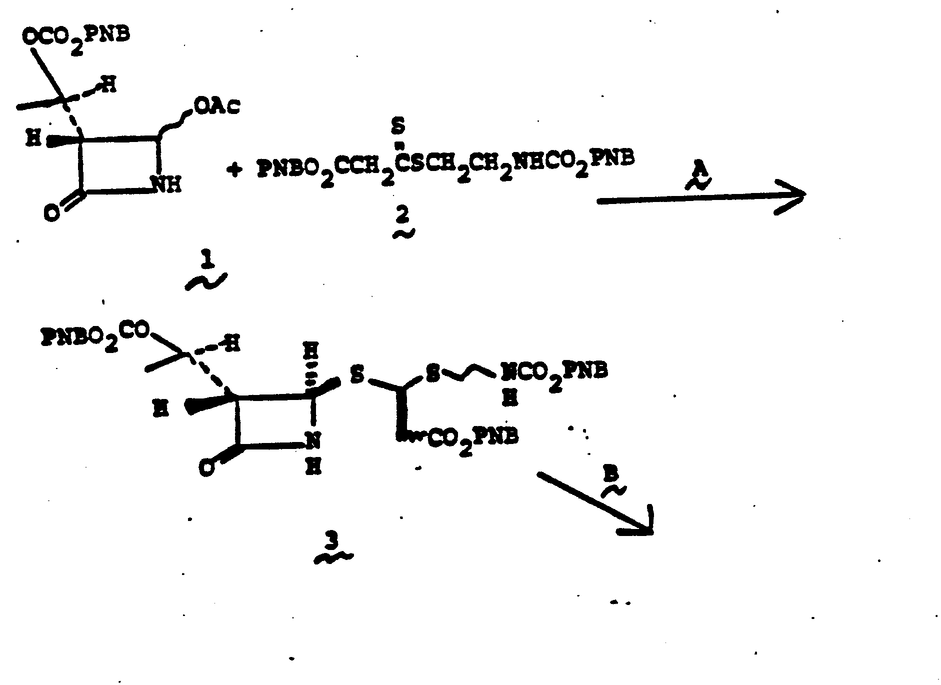

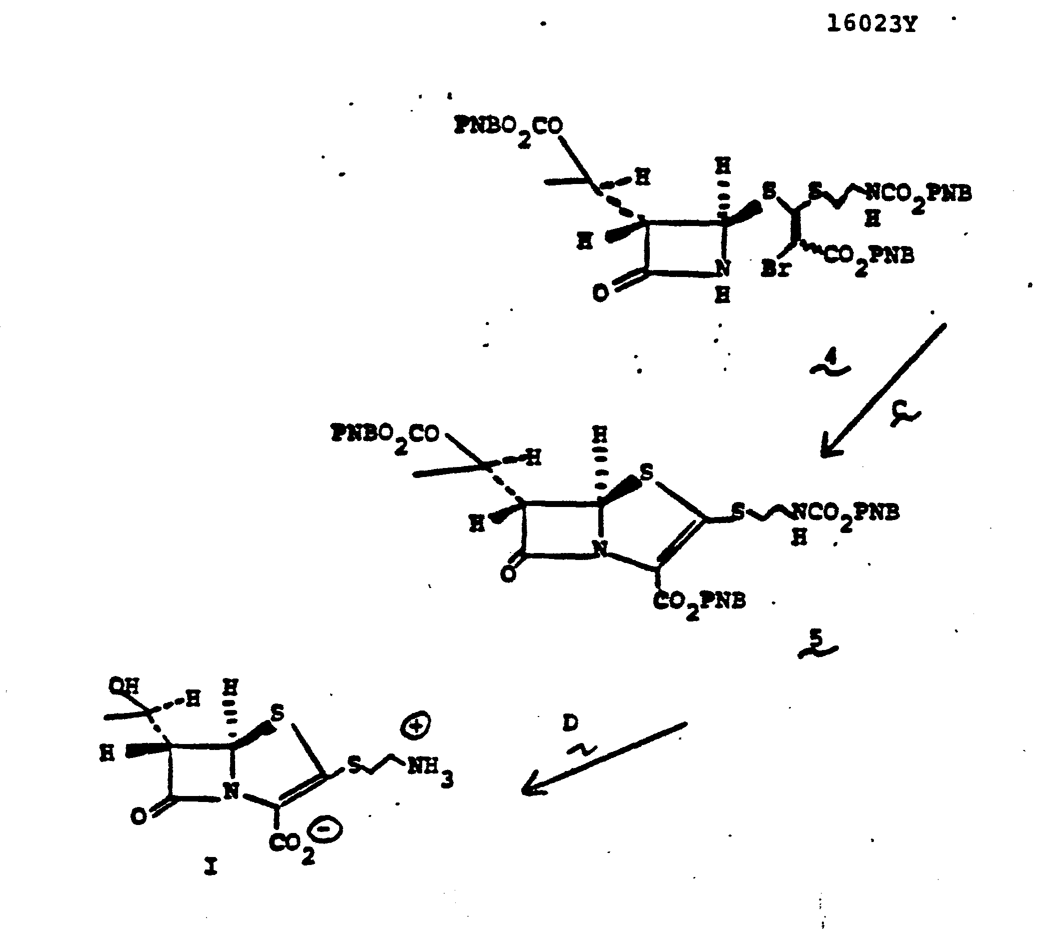

### Step A:

### Preparation of 2

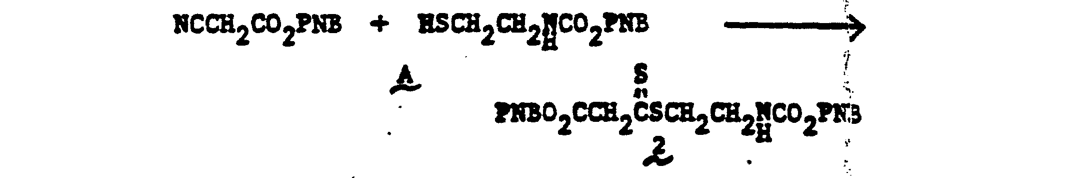

To a stirred solution of 3.77 g(17.2 mmol) of p-nitrobenzylcyanoacetate and 4.0 g (15.6 mmol) of N-p-nitro-Cbz-cysteamine (A) in 40 ml dry benzene at 25°C under a nitrogen atmosphere is introduced a stream of BCl(g) for 7 min. The reaction mixture is cooled on an ice water bath and the introduction of HCl(g) continued for 3 min. The ice water bath is removed and the mixture is stirred magnetically for 24 hrs. After this time, the solution is removed from the separated solid with the aid of a filter stick and the solid is washed with dry benzene (2 x 40 ml) in the same manner. The solid is dried in vacuo in the reaction flask, then dissolved in dry dimethylsulfoxide (DMSO) at 25°C. To the stirred solution is added an excess of H₂S(g) under nitrogen atmosphere for 10 min. and the mixture is stirred at 25°C. for 1.0 hr. The resulting orange colored solution containing some suspended material is poured into 200 ml cold H₂0 and 200 ml Et₂O. The organic phase is separated and washed with H₂0 (4x) and saturated NaCl(aq) solution, then dried over MgSO₄, filtered and evaporated to give 3.4 g of residue. Purification is accomplished by chromatography on silica gel eluting with benzene:ethylacetate (4:1) to give 2.1 g (67% based on 59% conversion) of 2 as a yellow-orange oil which slowly solidifies; IR(CHCl₃) 3400, 1720(sh), 1710, 16₀₁, 1₅₂₀ cm⁻¹; NMR (CDCl₃)δ: 3.45 (m, 4H); 4.08 (s, 2H); 5.1 (bs, 1H): 5.17 (s, 2H); 5.27 (s, 2H); 7.42 (d, J=8Hz, 4H); 8.12 (d, J=8Hz, 4H) mass spectrum m/e no 493(M⁺) 446, 256, 239, 209, 195, 165, 153, 136, 120.

### Step

### Preparation of Seco-Lactam 3

To a stirred mixture of 62.2 mg (0.18 mmol) of azetidinones 1 and 87.1 mg (0.18 mmol) of dithioate 2 in 1.5 ml of dry THF at 25°C is added in one portion 50.5 mg (0.25 mmol) If solid aluminum isoproproxide [Al(O 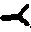 )₃]. The mixture is stirred at 25°C under N₂ for 6.0 hr. and is then partitioned between EtGAc and a cold, aqueous solution of dilute HCl-tartaric acid mixture. The EtOAc phase is separated and is washed with saturated NaCl(aq.), dried (MgSO₄), filtered, and evaporated. Purification by plate layer chromatography [2 developments CHCl₃-EtOAc (3:1)] provides 66.4 mg. (48%) of white foam 3, ir (CHCl₃) 1770, 1725, 1690, 1600 cm⁻¹; nmr (CDCl₃)δ1.48, 3H (d, J=6Hz); 3.2, 2H(m); 3.5, 3H(m); 5.24, 8H(m) : 6.1, 1H(s); 6.83, 1H(bs); 7.56, 6H(m); 8.24, 6H (apparent doublet, J=8Hz); 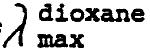 263 nm (E% = 427);α_{D} = +49.7°.

### Step C

### Preparation of Bromide 4

To a stirred solution of lactam 3 (66.4 mg, 0.085 mmol) and 15.1 mg (0.085 mmol) HMPA (hexamethylphosphoramide in 2.0 ml dry THF (tetrahydrofuran) at 25°C is added in one portion 16.5 mg (0.093 mmol) solid NBS (N-bromosuccinimide). The mixture is stirred at 25°C under N₂ for 0.5 hr. and evaporated. The residue is partitioned between EtOAc and H₂O and the EtOAc phase is separated. It is further washed with H₂0 (2x) and saturated NaCl (aq.), then dried (MgSO₄), filtered, and evaporated. Purification by repetitive plate layer chromatography [2 developments CHCl₃-EtOAc (3:1)] affords 54.7 mg (75%) of material after the first chromatogram and 35.7 mg (49%) of white foam 4 sfter the second chromatogram: ir (CHCl₃) 3375, 1775, 1720, 1600 cm⁻¹; nmr (CDCl₃)δ1.48, 3H (d, J=6Hz); 2.96, 2H(m); 3.37, 2H(m); 3.46, 1H(dd, J=2.5, 7Hz); 5.18, 2H(s) 5.1-5.4, 2H(m); 5.24, 2H(s), 5.38, 2H(s); 5.7, 1H(m); 6.72, 1H(bs); 7.56, 6H(m); 8.25, 6H(m);α_{D} = +48.5°; 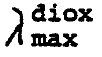 264 nm (E% = 394).

### Step D

### Preparation of penem 5

To a stirred mixture of 35.7 mg (0.04 mmol) of bromide 4 and 28 mg (0.14 mmol) of CuBr·S(CH₃)₂ in 5 ml dry THF at -78°C under N₂ is added 2 ml of a cold, freshly prepared solution of lithium diisopropylamide, LiN( 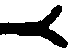 )₂, [generated at 0°/20 min. with 5 mg (0.05 mmol) HN( 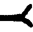 )₂ (diisopropylamide) and 19 pl of 2.4 M buLi (butyllithium]. The mixture is stirred over the following temperature ranges for the times indicated: -78° to -74° (40 min.); -74° to -68° (30 min.); -68° to -57° (30 min.); -57° to -41° (20 min.); -41° to -26° (20 min.); -26° to -20° (28 min.); -20° to -13° (38 min.); -15° to -9° (53 min.); -10° to -5° (18 min.); and -5° to 0° (18 min.). The mixture is then treated at 0° with 1 ml of saturated NH₄Cl (aq.) and diluted with Et₂O/H₂O. The organic phase is separated and washed further with aqueous NH₄Cl and saturated NaCl (aq.). The organic layer is dried (MgSO₄), filtered, and evaporated. The residue is purified by plate layer chromatography [two developments in CHCl₃-EtOAc (3:1)] to afford 18.6 mg (58%) of penem 5 as an off-white foam; ir (CHC1₃) 3430, 1799, 1730, 1704, 1601, 1520 cm⁻¹; nmr(CDCl₃)δ1.40, 3H(d, J=5.5Hz); 3.09, 2H(m); 3.54, 2H(m); 3.63, 1H(dd, J-2.5, 6.0 Hz); 5.22, 9H(m); 7.58, 6H(m) 8.26, 6H(m); 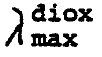 265 nm (E% 292), 315 nm (E% 91.5);α_{D} = -41.3°.

### Step

### Preparation of Penem I

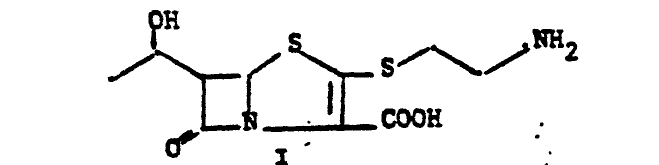

To 5.2 mg 5 (Step D, above) is added 0.60 ml dioxane, 0.05 ml ethanol, 0.35 ml deionized water and 0.01 ml of 1.OM R₂HPO₄. To the resultant clear solution is added 5 mg of 10% Pd/C. The suspension is flushed with N₂, then 5-6 times alternately with 50 psi H₂ and vacuum. Finally, it is shaken under a 50 psi H₂ atmosphere for 30-40 min. After centrifugation, the Pd/C is washed and centrifuged 2-3X with 0.5 ml portions of deionized water. The combined centrifugates are extracted 5xl - 2 ml ether. Residual ether is removed under vacuum and the aqueous solution applied to an XAD-2 column (20 x 140 mm). Fractions of 100 drops (6-7 ml) are collected, with continuous UV monitoring, by elution with deionized water. The chosen fractions are combined and lyophilized to provide I.

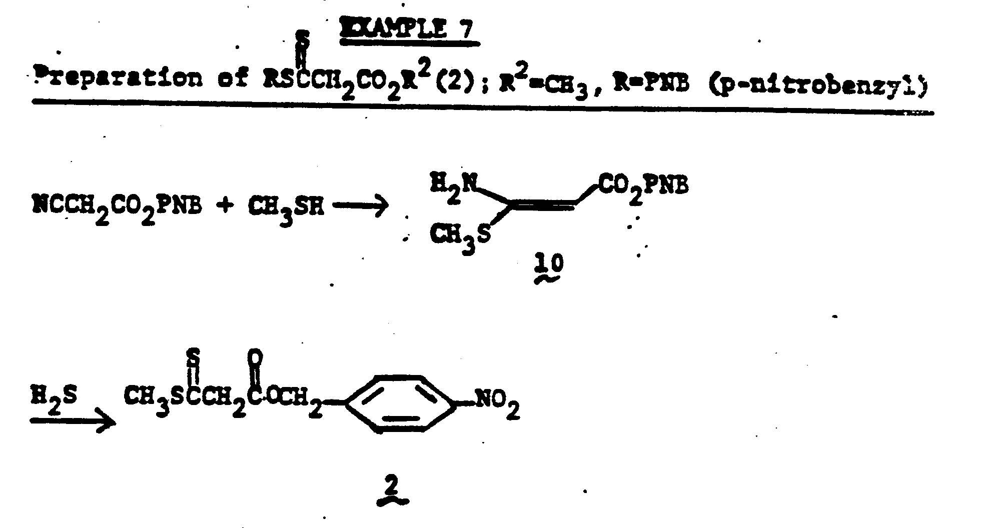

To a stirred mixture of 11.0 g (.05 mol) of p-nitro- benzylcyanoacetate and 3.72 g (.078 mol) methylmercaptan in 200 ml of sodium dried benzene at 25°C is introduced a stream of HCl(g) until 1.85 (.05 mmol) is added. The mixture is then stirred at 25°C under nitrogen atmosphere for 74.5 hrs. The solution is decanted away from the separated solid and the moist precipitate is dissolved in 50 ml of dry pyridine. (Alternatively the precipitate is dissolved in dry dimethylsulfoxide and treated with H₂S(g) as described below to afford 2 directly.) The pyridine solution is treated with excess H₂S(g) and is stirred at 25°C under nitrogen atmosphere for 2 hrs. The reaction mixture is poured into ice/water and extracted with ether. The separated extract is washed twice with water and twice with brine solution then dried over MgSO₄, filtered, and evaporated to give a crystalline mass. Recrystallization from ether gives 5.0 g of the enamide 10: ir(CHCl₃), 3636, 3322, 1664, 1592, 1517 cm⁻¹; NMR(CDCl₃)δ: 2.4(s, 3H), 4.63 (s, 1H); 5.17 (s, 2H); 6.43 (bs, 2H); 7.43 (d, J=8Hz, 2H); 8.13 (d, J=8Hz, 2H) mass spectrum m/e.268 (M⁺), 221, 175, 136. Purification of the mother liquors from above by column chromatography on silica gel eluting with CHCl₃- pet-ether (1:1) provides 940 mg of 2 as an orange oil: ir (CHC1₃) 1742, 1613, 1522; NMR (CDCl3)δ2.67 (s, 3H); 4.13 (s, 2H): 5.23 (s, 2H); 7.47 (d, J=9Hz, 2H); 8.17 (d, J=9Hz, 2H); mass spectrum m/e 285 (M⁺), 238, 136, 106. The enamide 10 is converted to 2 by the following procedure.

The crude enamide 10 7.9 g (29 mmol) is dissolved in 40 ml of dry DMF (dimethylformamide) at 25°C and is treated successively with 3.7 g (32 mmol) of trifluoroacetic acid and excess hydrogen sulfide. The resulting mixture is stirred magnetically at 25°C under CaCl2drying tube protection for 5 hrs. The mixture is poured into 75 ml ice-H₂0 and extracted with 100 ml Et₂0 (diethylether). The separated etheral extract is washed twice with H₂O and saturated NaCl(aq) solution, then dried with MgSO₄, filtered, and evaporated. Purification by column chromatography on silica gel eluting with CHC1₃-petroleum ether (1:1) gives 6.'55 g (74%) of. 2.

### EXAMPLE 8

Preparation of dithioate derivative,

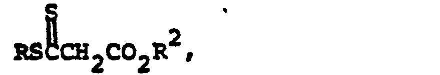

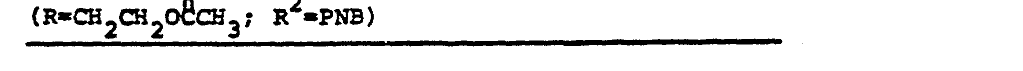

To a stirred mixture of 6.78 g (5.6 mmol) of 2-acetoxyethyl mercaptan and 11.0 g (5 mmol) p-nitrobenzyl- cyanoacetate in 100 ml of dry benzene is introduced 3.0 g (8.1 mmol) of hydrogen chloride gas. The mixture is stirred under N₂ at RT (25°C) for 42 hours after which time the solution is decanted away from the oily mass. The mass is washed twice with dry benzene by decantation. The oil is dissolved in 20 ml of dry dimethylformamide and is treated with a vigorous stream of hydrogen sulfide (g) for 5 min. The mixture is stirred at 25°C for 5 hours and then is poured into ice cold water and is extracted with ether. The ether extract is washed well with water, then brine, and is dried over MgSO₄, filtered, and evaporated. Purification by column chromatography on silica gel provides the dithioate derivative as an orange solid; ir (CHC1₃) 1770, 1600, 1538.cm⁻¹; nmr (CDCl₃) δ: 2.03, 3H(s); 3.53, 2H(t,J-6Hz); 4.1, 2H(s); 4.27, 2H(t,J-6Hz); 5.25, 2H(s); 7.46, 2H(d,J-9Hz); 8.2, 2H(d,J-9Hz); mass spectrum m/e 357(M⁺), 297, 271, 206, 169, 136.

### EXAMPLE 9

Preparation of Benzyl-thiobenzoylacetate 2, 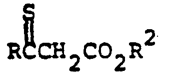 2 (R = phenyl; R²= benzyl):

Into a stirred solution of 19.2 g of benzylaceto- acetate in 150 ml of acetonitrile at -60°C is passed a stream of hydrogen sulfide gas for 1.5 hours, followed by dry HCl(g) for 40 min. The reaction solution is then let warm to -20°C and is poured into ice water. The product 2 is extracted into benzene and purified by vacuum distillation to provide the known benzyl-thiobenzoylacetate, bp 98° (0.05 mm).

### EXAMPLE 10

Following the procedure described in the foregoing text and Examples, the following starting reagents 2 necessary for the preparation of the compounds of the present invention are representatively obtained by analogy.

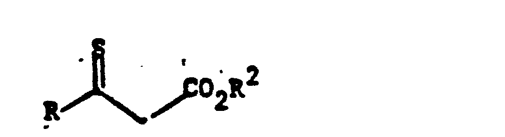

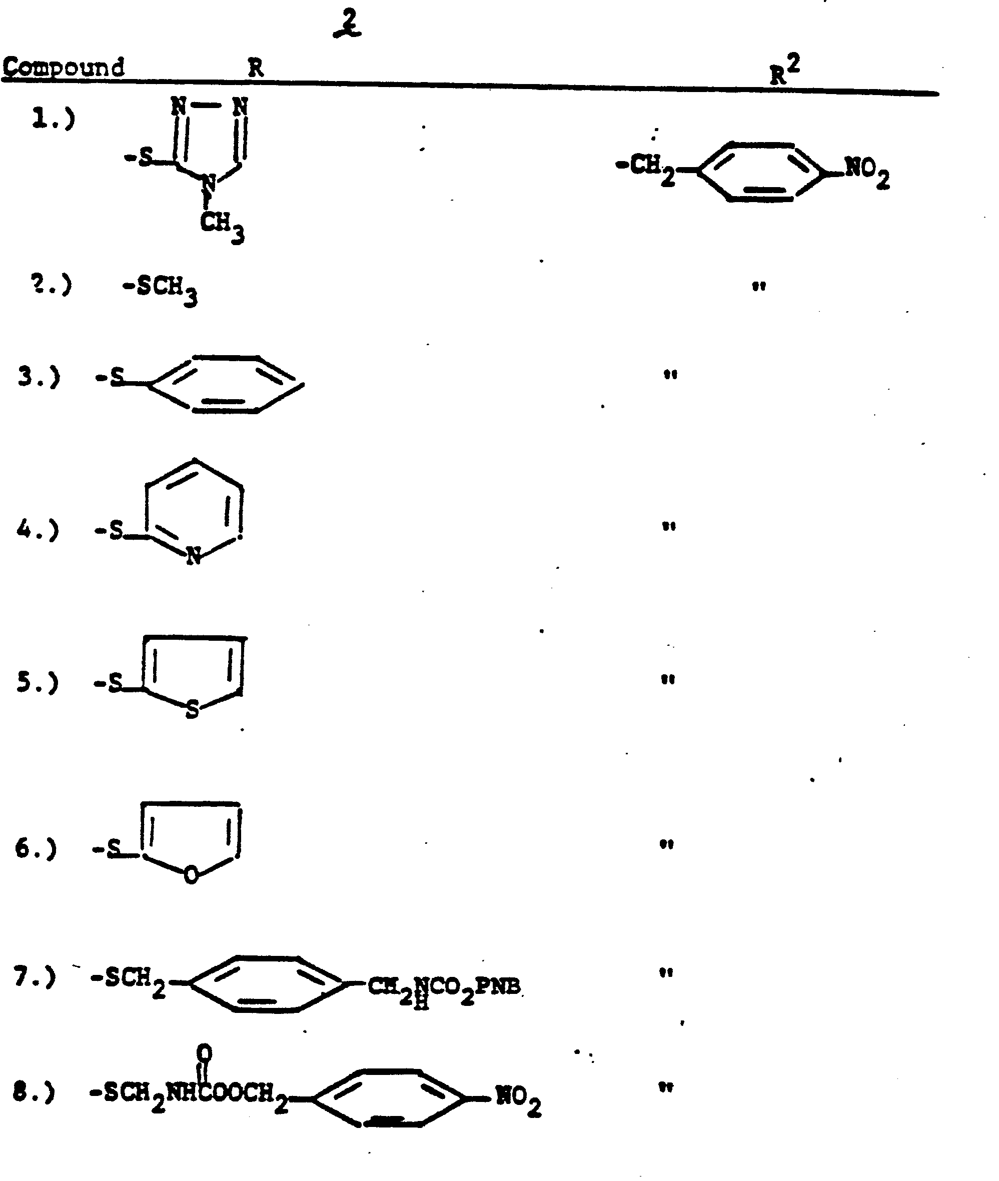

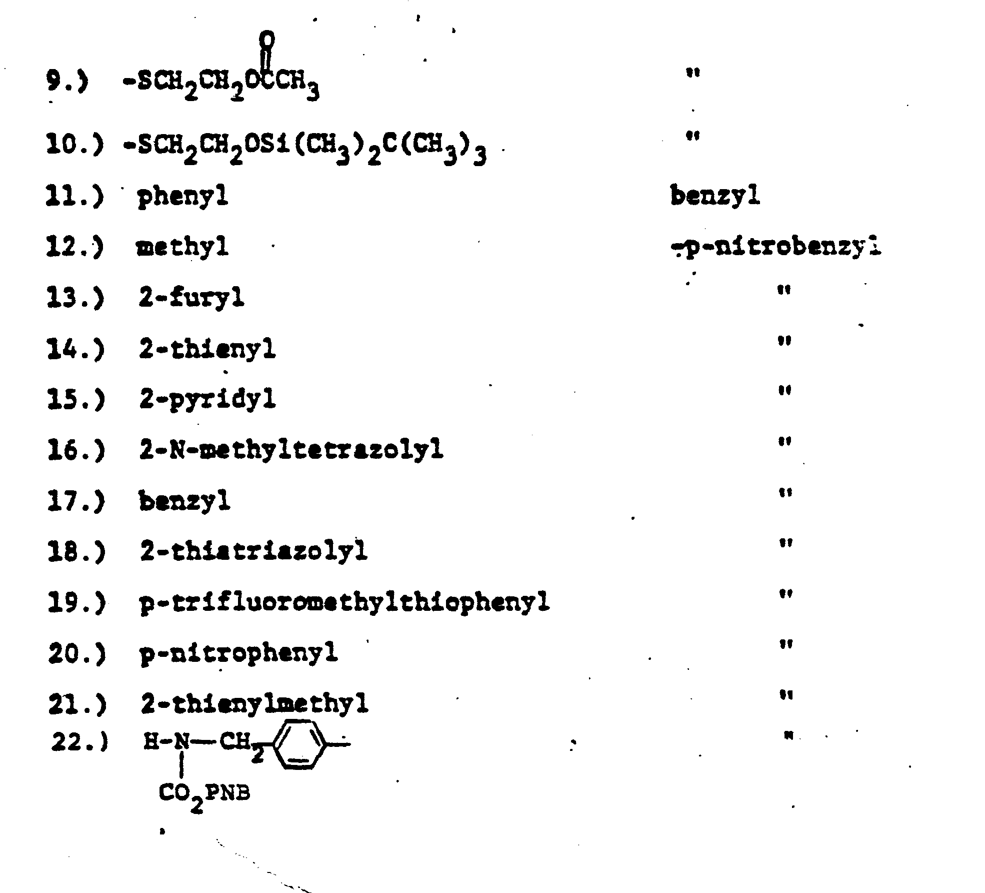

### EXAMPLE 11

Preparation of Seco-lactam 3: R₁R₂=H, R₃=SCH₃'

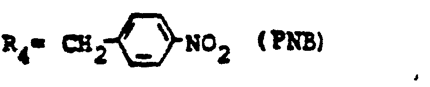

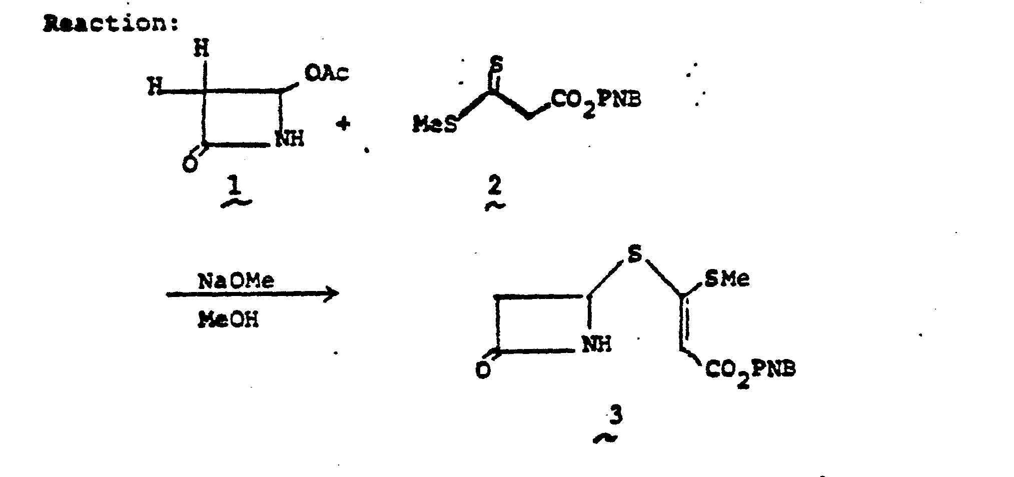

A mixture of 504 mg (3.9 mmol) of 4-acetoxyazetidinone and 940 mg (3.3 mmol) of dithioate 2 in 25 ml methanol is cooled briefly on an ice/H₂O bath under Argon atmosphere and 184 mg (3.4 mmol) of sodium methoxide is added. The mixture is stirred cold for 10 minutes.and the ice/H₂O bath is then removed. The mixture is stirred further for 67 hrs. The reaction is diluted with EtOAc and H₂O and the organic phase is separated, dried over MgS0₄, filtered, and evaporated to give a solid residue. The residue is treated with methanol and the insoluble product 3 is collected by filtration to give 865 mg (74%); recrystallization from hot MeCN/EtOAc gives 680 mg; mp 146-155°, IR (CHCl₃) 1₇₇₉, 1695, ₁₆₀₀, ₁515 cm⁻¹; NMR (d₆-DMSO)δ: 2.45 2.53 (singlets, 3H); 2.8, 3.03, 3.37, 3.67 (multiplets, 2H): 5.27 (s, 2H); 5.33 (m, IH); 5.78; 5.82 (singlets, 1H); 7.63 (d, J=9Hz, 2H); 8.2 (d, J=9Fz, 2H); 8.63 & 8.9 (broad singlet, 1H). Anal. Calcd. for C₁₄B₁₄N₂O₅S₂: C, 47.⁴⁵; H, 3.98; N, 7.90; S, 18.09. Found: c, 47,30; H, 4.01; N, 7.93, S, 17.95;

### EXAMPLE 12

### Preparation of secolactam 3

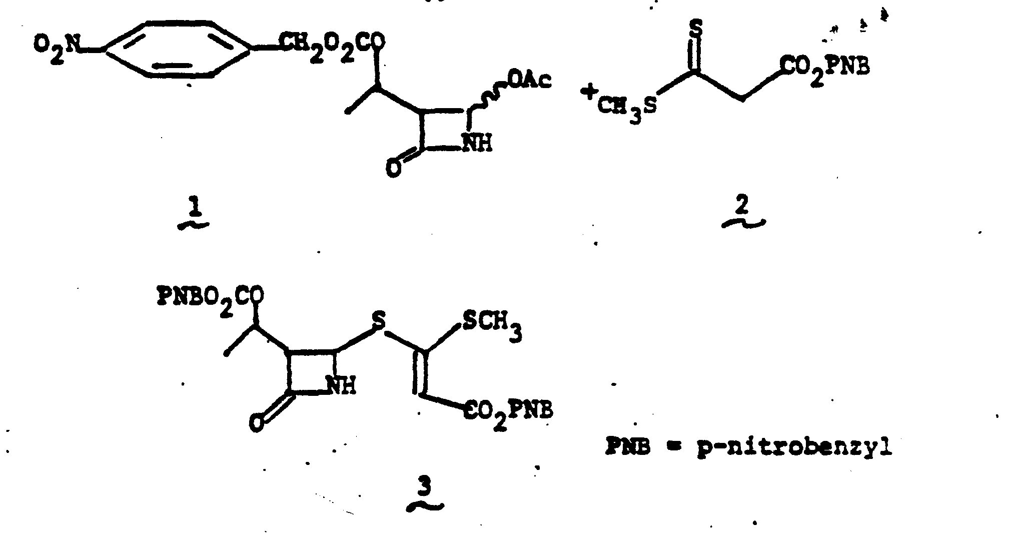

To a stirred mixture of 70.4mg (0.2 mmol) of azetidinones 1 and 57.0 mg (0.2 mmol) of dithiomalonate derivative 2 in 2 ml of dry tetrahydrofuran (THF) at 25°C is added 57.2 mg (0.28 mmol) If solid aluminum isopropoxide. The mixture is stirred at 25°c under nitroge atmosphere for 24 hours and is then partitioned betwetn ehtylacetate and cold, dilute aqueous HCl. The EtOAc phase is separated, washed with brine, dried with MgSO₄, filteret and evaporated Purification by plate layer chromatography provides seco- lactam 3.

### EXAMPLE 13

Following the procedures described in the foregoing Examples and text, the following secolactams 3 are prepared by analogy. Note that for each entry in the table, R and R² are defined by compounds a-j o£ Example 5, above.

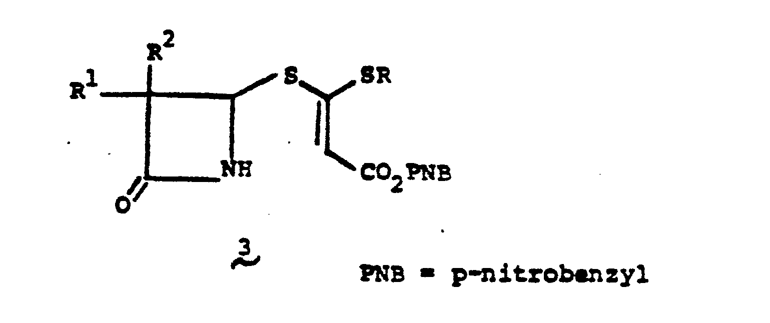

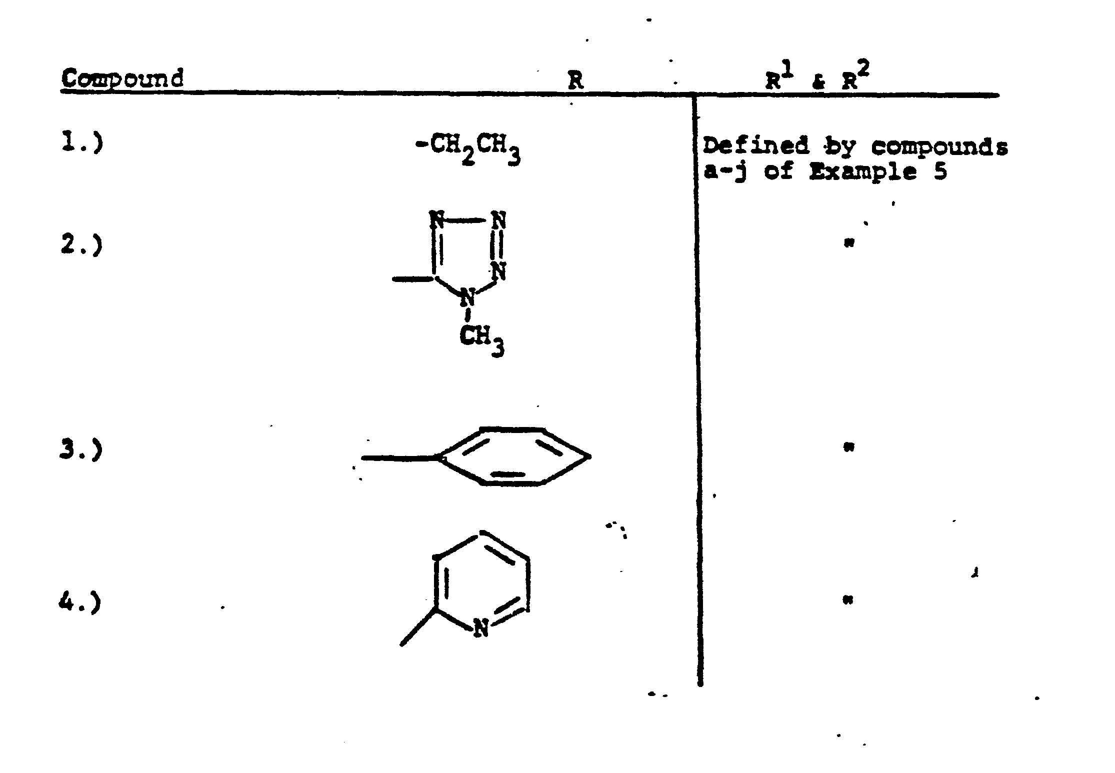

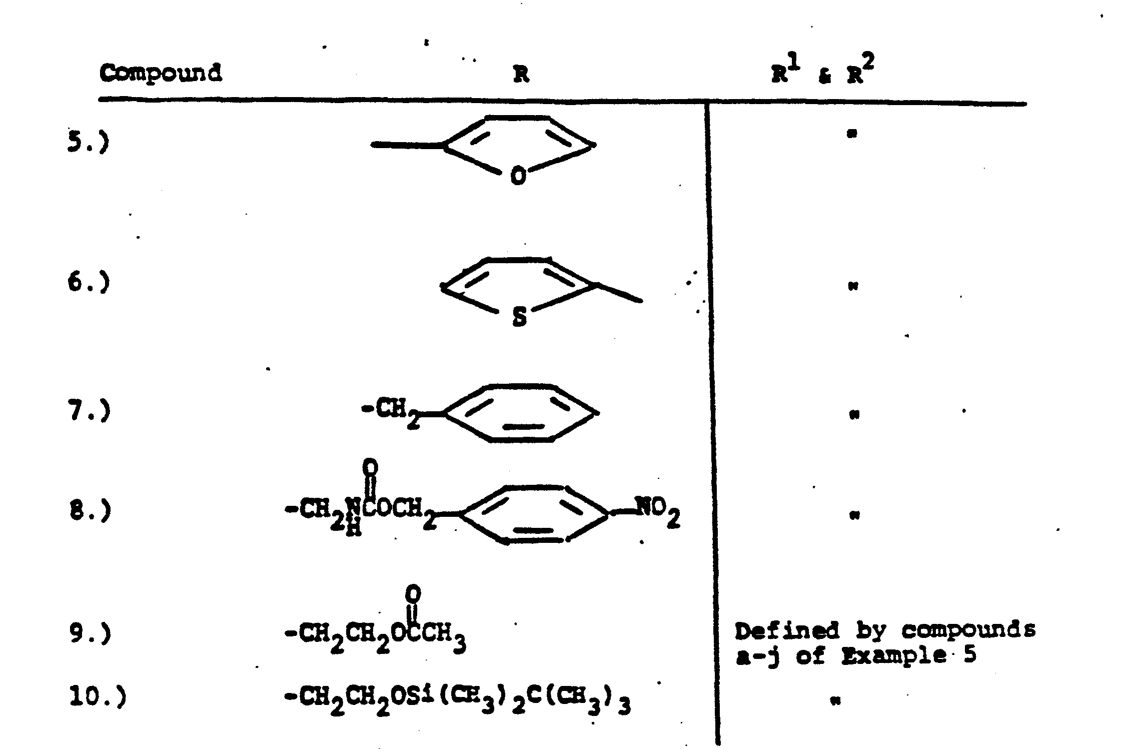

### EXAMPLE 14

Preparation of secolactam 3

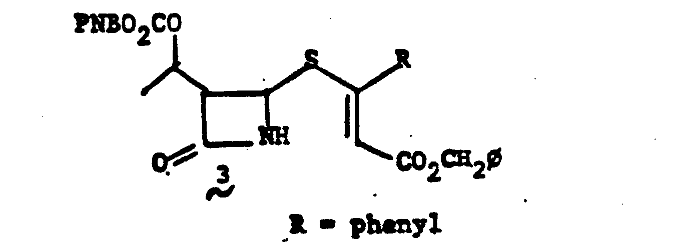

To a stirred mixture of 35.2 mg (0.1 mnol) of azetidinones 1 and 27 mg (0.1 mnol) of benzylthiobenzoyl- acetate in 2.0 ml methanol at 25°c is added 59.4 mg (0.11 maaol) of sodium methoxide. The mixture is stirred at 25°C under a nitrogen atmosphere for 24 hours. The solvent is removed by evaporation and the residue is partitioned between ethyl acetate and cold water. The EtOAc phase is separated, washed with brine, dried with MgSO₄, filtered, and evaporated. The secolactam 3 is obtained by plate layer chromatography.

### EXAMPLE 15

Employing the procedures described in the foregoing text and Examples, the following azetidinones 3 are prepared by analogy: Note that for each entry in the table, _{R}¹ and _{R}² are defined by compounds a-j of Example 5, above.

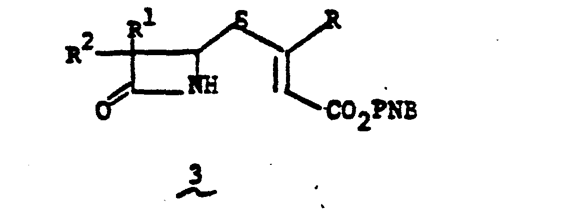

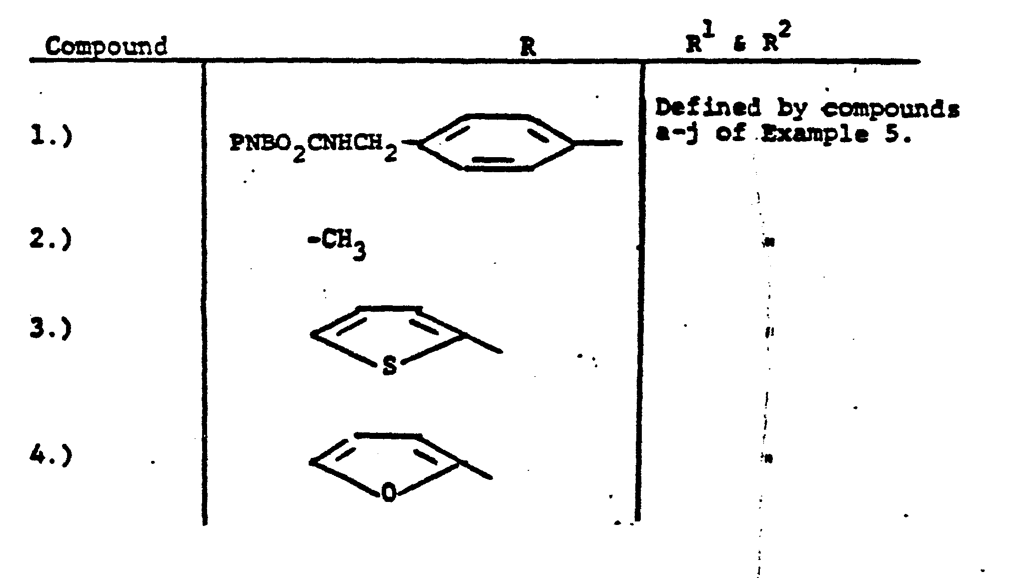

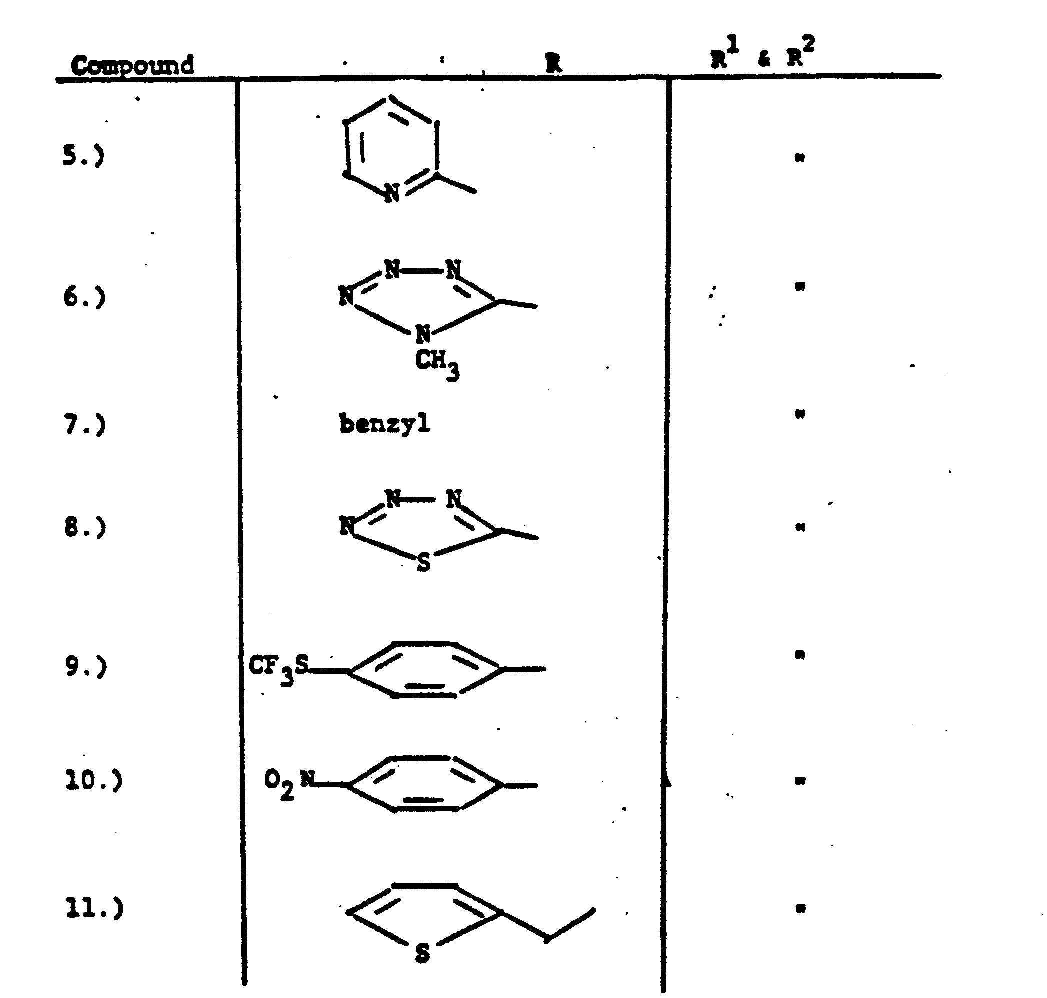

### EXAMPLE 16

preparation of 4 R₁= R₂= H, R₃=SCH_{3'}

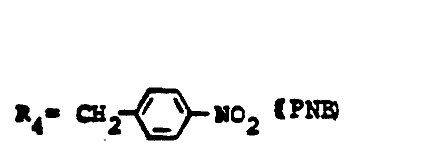

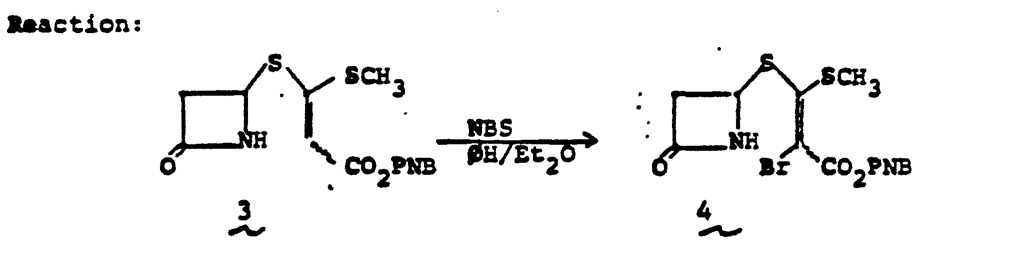

To a stirred mixture of 64.5 mg (0.18 mmol) of 3 in 5 ml benzene and 3 ml Et₂0 at 60° is added 35.7 mg (0.2 mmol) of solid N-bromosuccinimide. The mixture is stirred at 60° for 7 min. and then let cool under an Argon atmosphere for 1 hr. The mixture is evaporated and quickly purified by plate layer chromatography [one development benzeneethylacetate (2:1)] to provide 41 mg (52%) of 4; ir (CHC1₃) 1779, 1727, 1605, 1517; NMR (CDCl₃)δ: 2.38 2.47 (singlets, 3H); 2.67-3.73 (multiplets, 2H); 4.93 5.2 (doublets of doublets, J-2, 4Hz, lH); 5.33 (s, 2H); 6.73 (bs, 1H); 7.53 (dd, J-8Hz, 2H); 8.2 (dd, J=8Hz, 2H); mass spectrum m/e 434, 432(M⁺), 387, 385, 353, 311, 284, 281, 279, 136.

Further purification of mixture 4 results in the decomposition of the E-isomer and the isolation of pure Z-isomer: 15 mg: IR (CHC1₃) 3534, 1783, 1733, 1613, 1522; NMR (CDCl₃)δ: 2.4(s, 3H); 3.04(doublet of quartets, J=1.0, 2.5 & 15Hz, 1H) 3.53(doublet of quartets, J-2.0Hz, S.OHz, 15Hz); 5.17(dd, J=2.5, 5Hz, lH); 5.3(s, 2H); 7.1(bs, 1H); 7.48(d, J=8Hz); 8.13(d, J=SHz).

### EXAMPLE 17

Preparation of bromide 4

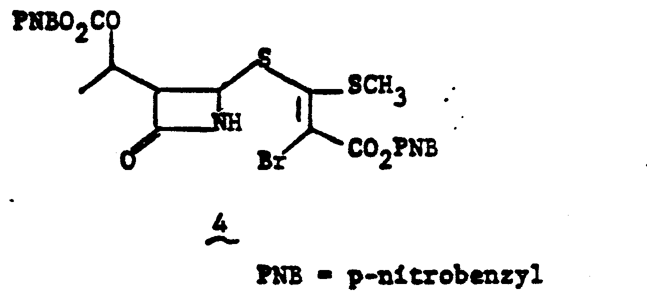

To a stirred solution of the secolactam 3 prepared in Example 12 (57.7 mg 0.1 mmol) and 17.9 mg (0.1 mmol) of hexamethylphosphoramide in 2 ml dry tetrahydrofuran at 25°C is added 17.8 mg (0.1 mmol) of N-bromosuccinimide. The mixture is stirred at 25°C under N₂for 0.5 hr. and the solvent is removed under reduced pressure. Purification by repetitive plate layer chromatography affords bromide 4.

### EXAMPLE 18

Following the procedures described in the foregoing text and Examples, the following intermediate species 4 are representatively prepared by analogy. Note that R and R² are defined for each entry in the table by compounds a-j of Example 5, above.

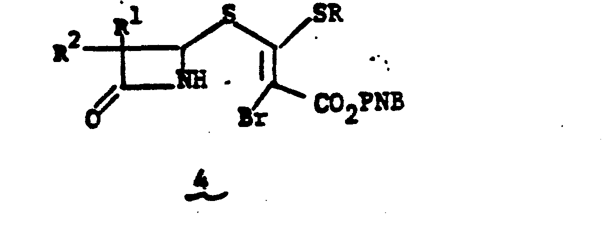

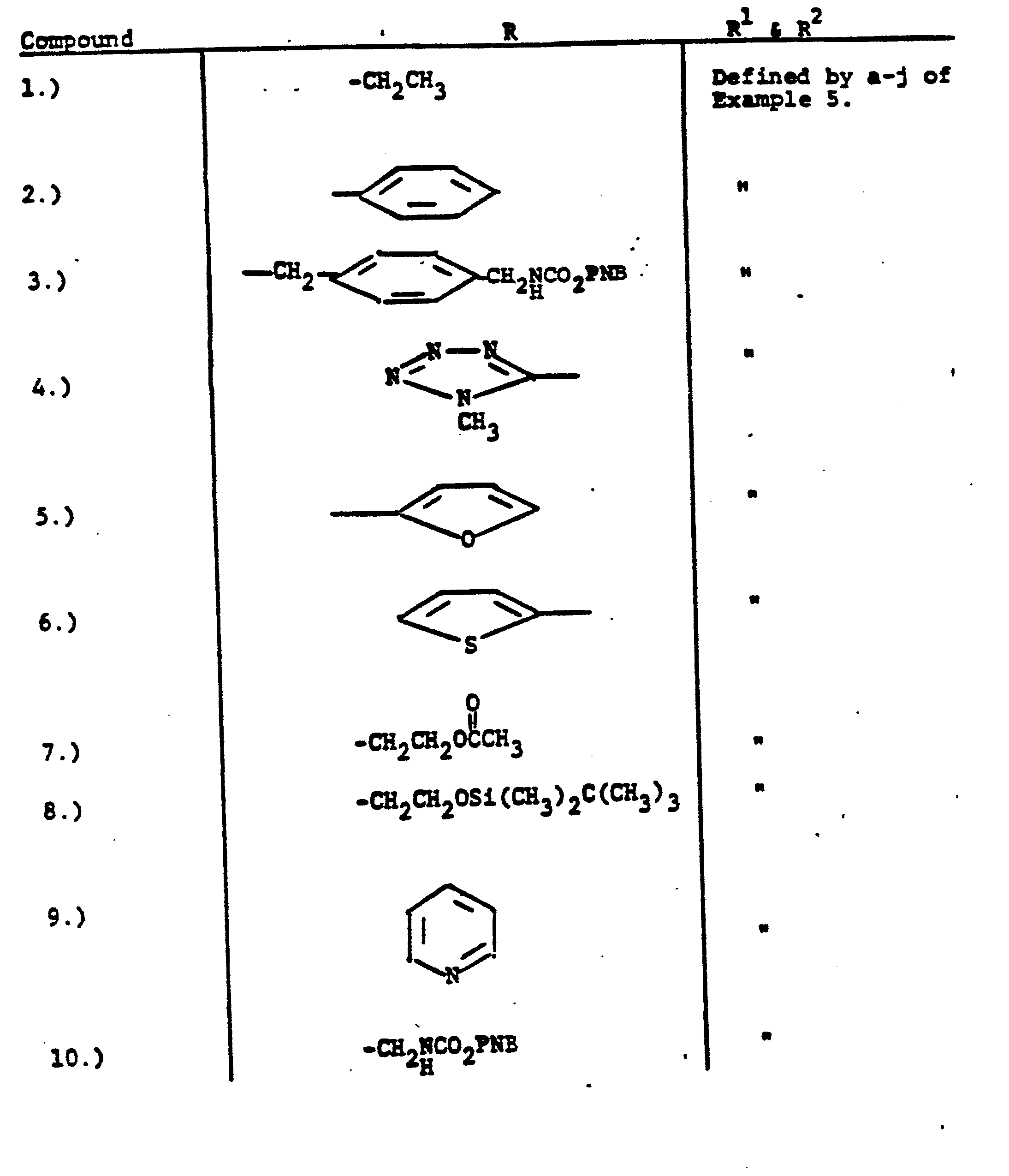

### EXAMPLE 19

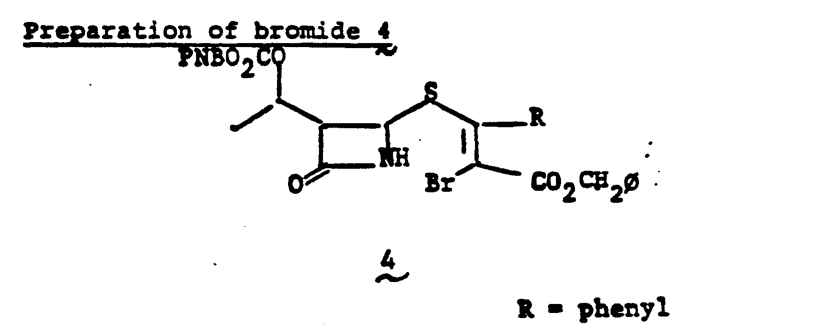

R = phenyl To a stirred mixture of the secolactam derivative 3 of Example 14 (56.2 mg, 0.1 mmol) and 17.9 mg (0.1 mmol) of hexamethylphosphoramide in 2 ml dry tetrahydrofuran at 25°C is added 17.8 mg (0.1 mmol) of N-bromosuccinimide. The mixture is stirred at 25°C under N₂ for 1.0 hour and the solvent removed under reduced pressure. Purification by repetitive plate layer chromatography provides bromide 4.

### EXAMPLE 20

Following the procedure described in the foregoing text and Examples, the following bromides 4 are representatively obtained. Note that R^{l} and R² in the table are defined by each entry by Compounds a-j of Example 5, above.

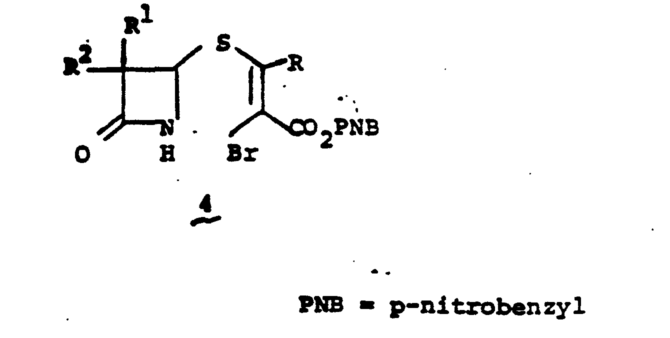

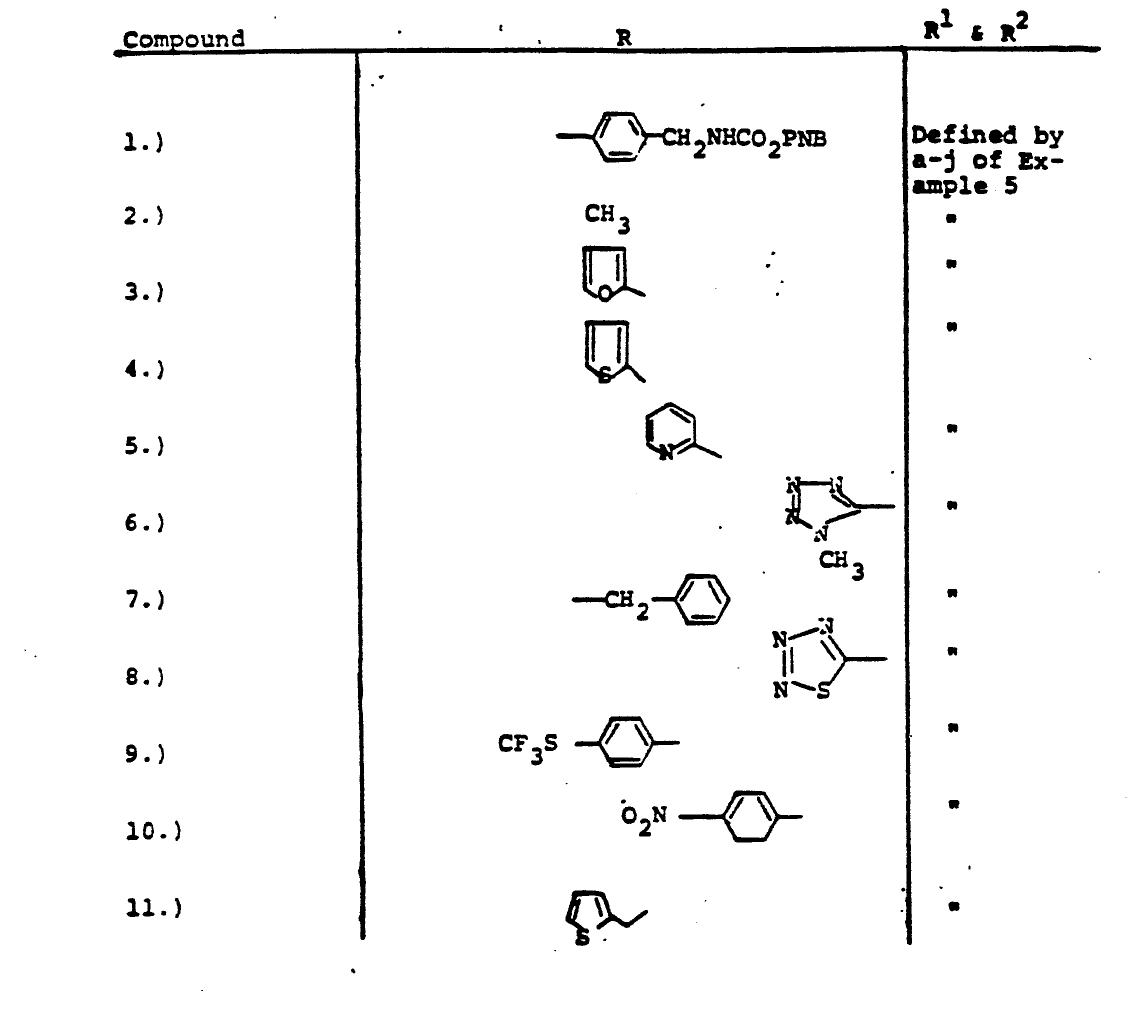

### EXAMPLE 21

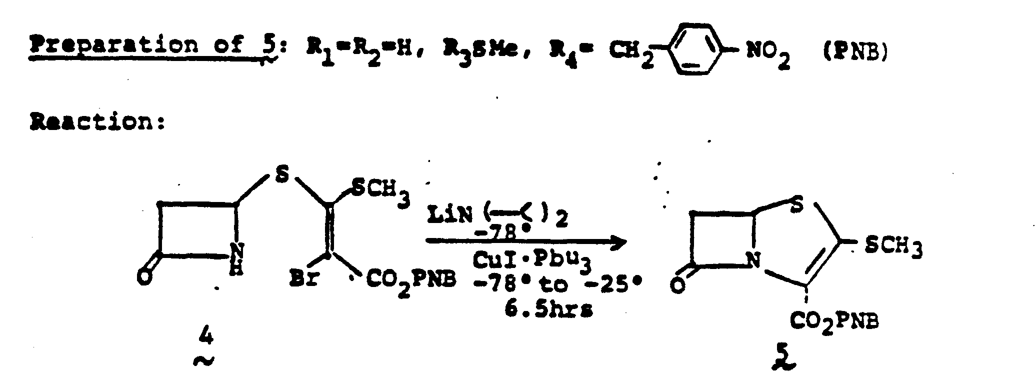

To a stirred solution of pure Z-bromolactam 4 (50.7 mg, 0.12 mmol) and 46.0 mg (0.12 mmol) of cuprous iodide-tri-n-butylphosphine complex in 15 ml dry THF at -78° under Argon atmosphere is added dropwise a cold solution of freshly prepared lithium diisopropylamide (0.12 mmol) in 6.6 ml dry THF. The resulting greenish solution is stirred from -78°C to-25°C under Argon atmosphere over a period of 6.5 hrs. The reaction is quenched at -25°C with 1.0 ml saturated NH₄Cl (aq) solution and is then partitioned between Et₂O/H₂O. The organic phase is separated ant washed successively with aqueous NH₄Cl solution and brine, then dried MgSO₄, filtered, and evaporated. The residue is purified by plate layer chromatography [2 developments CHCl₃] to give 17.7 mg (43%) of 5: IR (CHC1₃) 1795, 1689, 1608, 1517 cm⁻¹; NMR (CDCl₃)δ: 2.47(s, 3H); 3.2(dd, J=_{2.}5_{H}z, 16Hz, IH); 3.8(dd, J-5, 16Hz, IH); 5.17(dd, J=2.SHz, 5.OHz, IH); 5.27(s, 2H); 7.47(d, J=9Hz, 2H); 8.2(d, 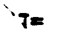 9Hz, 2H); mass spectrum m/e 352(M⁺); 310, 174, 158, 144

### EXAMPLE 22

Preparation of Penem 5 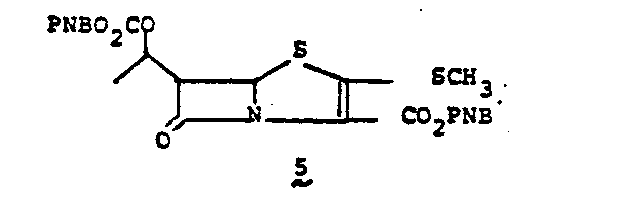 To a stirred mixture of the seco-lactam bromide 4 of Example 15 (65.6 mg, 0.1 mmol) and cuprous bromide-dimethylsulfide complex (67.7 mg, 0.33 mmol) in 10 ml dry tetrahydrofuran at -78°C under a nitrogen atmosphere is added a cold, freshly prepared solution of lithium diisopropylamide (0.11 mmol) in 4 ml dry tetrahydrofuran. The mixture is stirred at -78°C for 40 minutes and is allowed to warm to 0°C over a period of 5 hours. At 0°C, 2 ml of saturated ammonium chloride (aq.) solution is added and the mixture is partitioned between ether and water. The organic phase is separated and washed further with saturated ammonium chloride solution and brine. The organic phase is separated, dried over MgSO₄, filtered, and evaporated. Purification is accomplished by plate layer chromatography to yield penem 5.

### EXAMPLE 23

Following the procedure described in the foregoing Examples and text, there is obtained the following representative penems 5 by analogy: Note that R¹ and _{R}² in the table are defined for each entry by compounds a-j of Example 5, above.

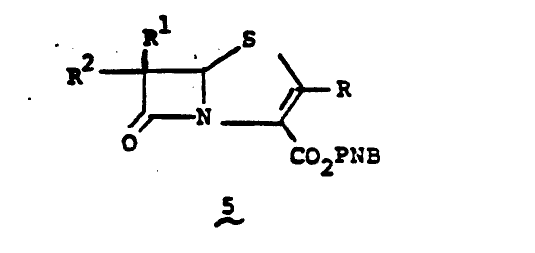

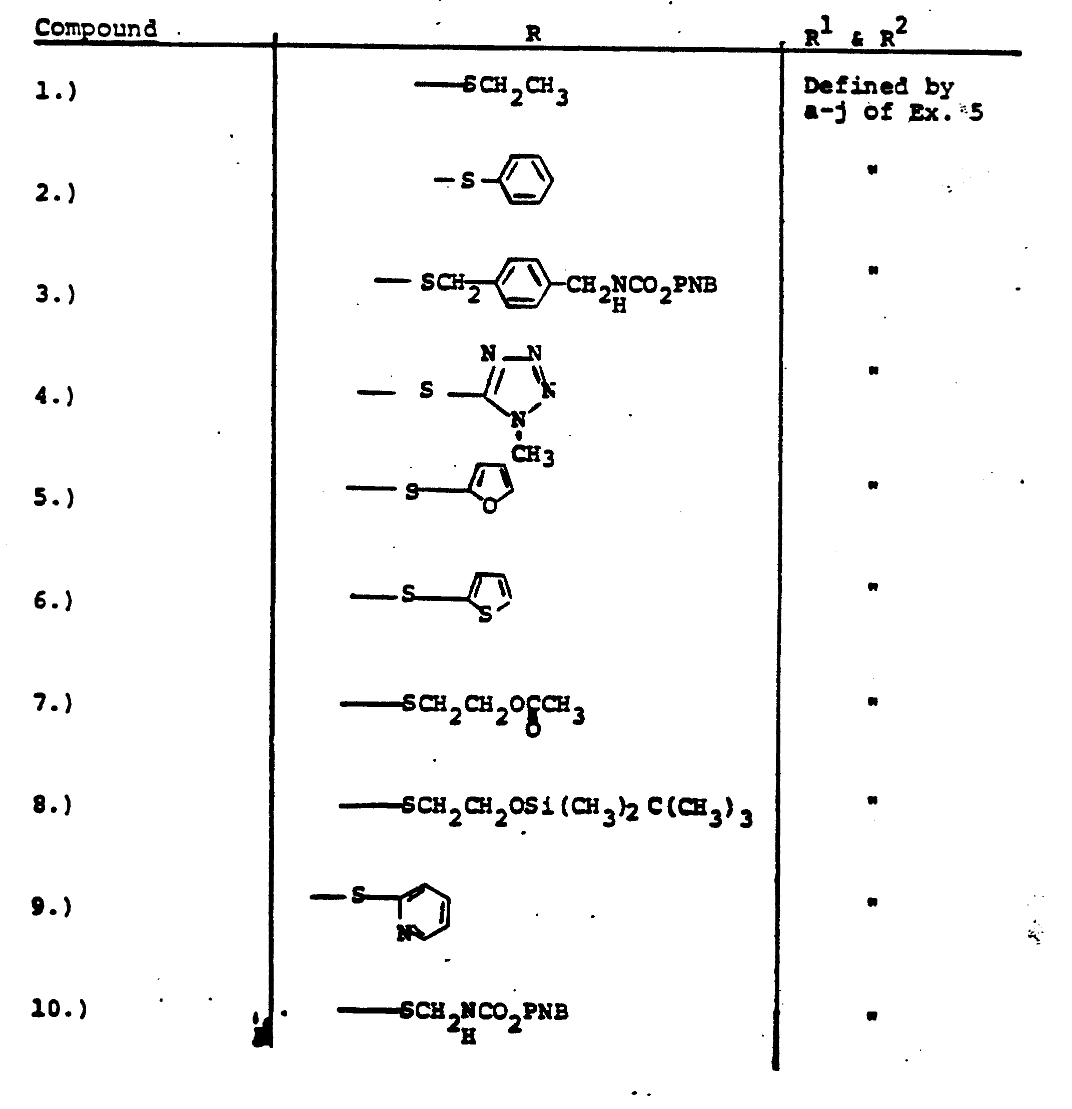

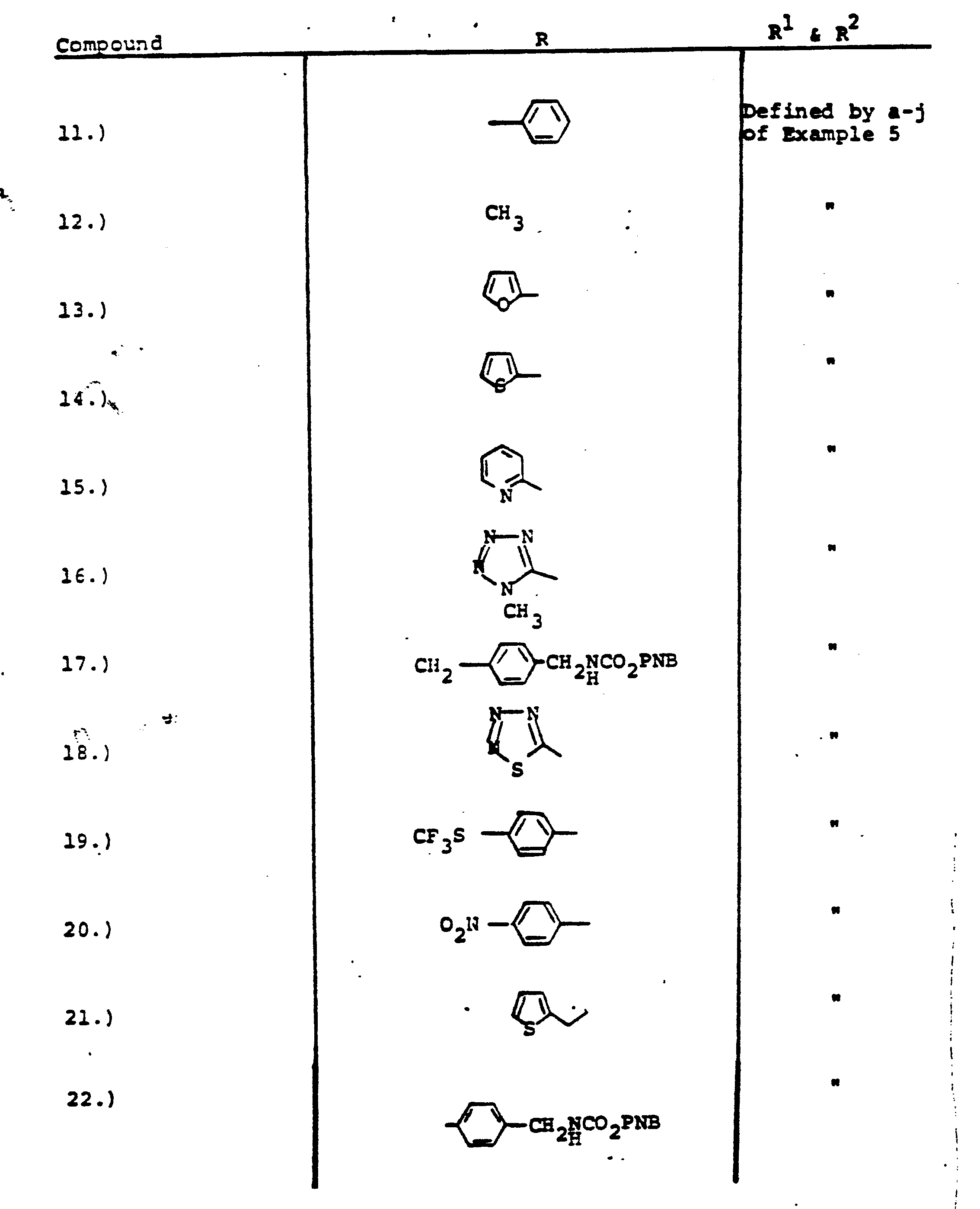

### EXAMPLE 24

Conversion of 5(R₁= R₂=H, R₃SCH₃' 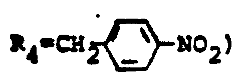 to the rree acid ana its sodium salt, I

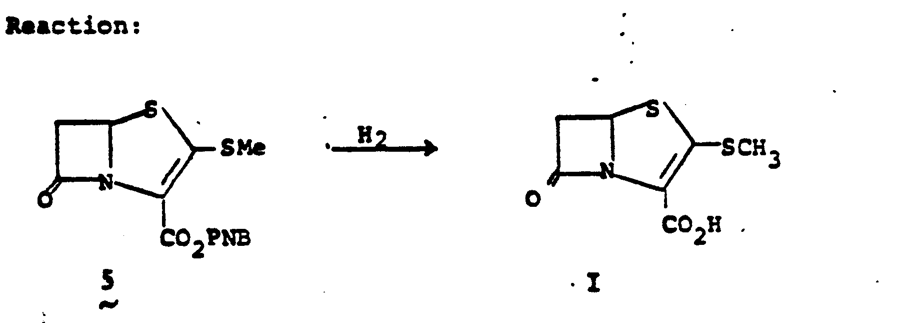

A mixture of 7.8 mg (0.02 mmol) of penem 5 and 7.8 mg 10% Pd/C in 1.0 ml dioxane, 1.0 ml THF, 0.5 ml H₂), and 0.5 ml 0.5N pH 7 phosphate buffer is hydrogenated at rt and 40 psi fer 25 min. The catalyst is removed by filtration through SOLKA-FLOC washing with water. The filtrate is extracted with an equal volume of EtOAc and the aqueous layer ts then assayed for antibacterial activity. The aqueous sorition is then cooled on an ice/H₂O bath and is acidified to pH 3.3 with 1.OM pH 2 phosphate buffer. The solution is extracted twice with EtOAc and the extract is dried over MgSO_{4'} filtered, and evaporated. The residue is dissolved in CHCl₃ quickly and the IR of the free acid recorded: 1785,1718, 1667 cm⁻¹. The chloroform solution is quickly eveporated and is redissolved in a minimum amount of aceton and is then treated with an equivalent amount of NaHCO₃ in 0.5 ml H₂O. The Me₂CO is removed under reduced pressure and the aqueous phase is lyophilized to give a quantitative yield of sodium salt.

### EXAMPLE 25

Preparation of penem I 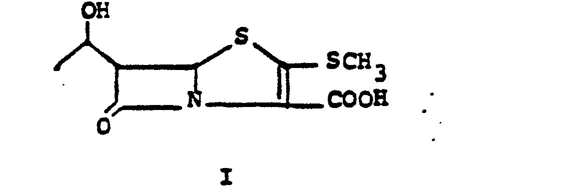 A mixture of 10 mg of 10% palladium on carbon, 5 mg. (0.0086 mmol) of the penem of Example 20 and 1 mg. sodium bicarbonate in 0.8 ml ethylacetate, 0.2 ml isopropanol, and 0.5 ml of 0.5M pH 7 phosphate buffer is hydrogenated at 40 psi at 25°C in a Parr shaker for 48 minutes. The catalyst is removed by filtration through supercel and is washed thoroughly with deionized water. The cold filtrate is extracted with ethylacetate and the separated aqueous phase is acidified at 0°C with O.1M pH 2 phosphate buffer to pH 2.5. The aqueous phase is extracted thoroughly with EtOAc. The extracts are combined, dried over anhydrous sodium sulfate, filtered and evaporated to provide penem I.

The free acid is converted to the corresponding sodium salt by treatment with an equivalent amount of an aqueous acetone solution of sodium bicarbonate, followed by removal of the acetone under reduced pressure and lyophilization of the aqueous solution.

In cases where the deblocking procedure results in the formation of zwitterionic penems I, the above acidification is performed as described but is stopped at pH 7. The zwitterionic product is then obtained after chromatography on X-AD-2 resin.

### EXAMPLE 26

Preparation of sodio-6-[1'-hydroxymethyl]-2- phenyl-pen-2-3m-3-carboxylate.

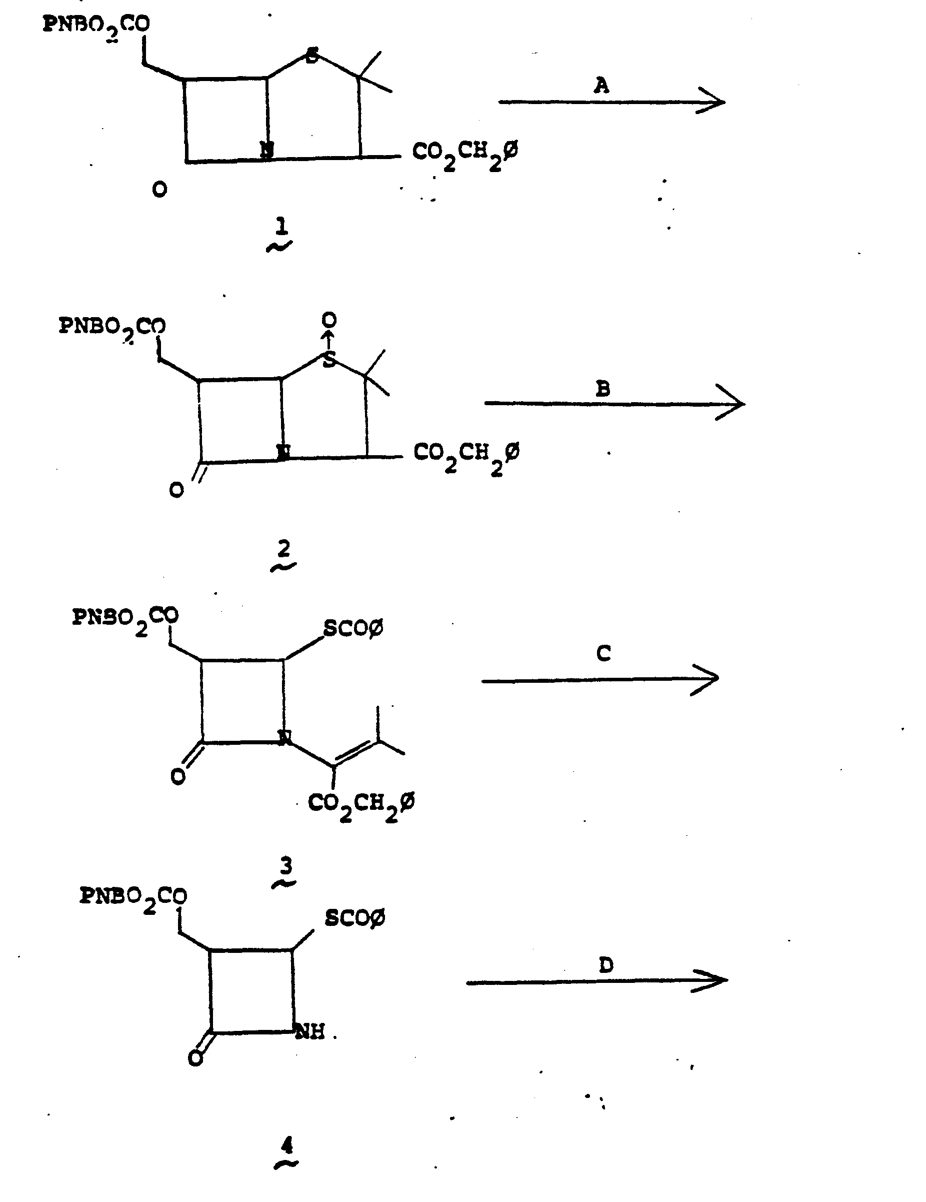

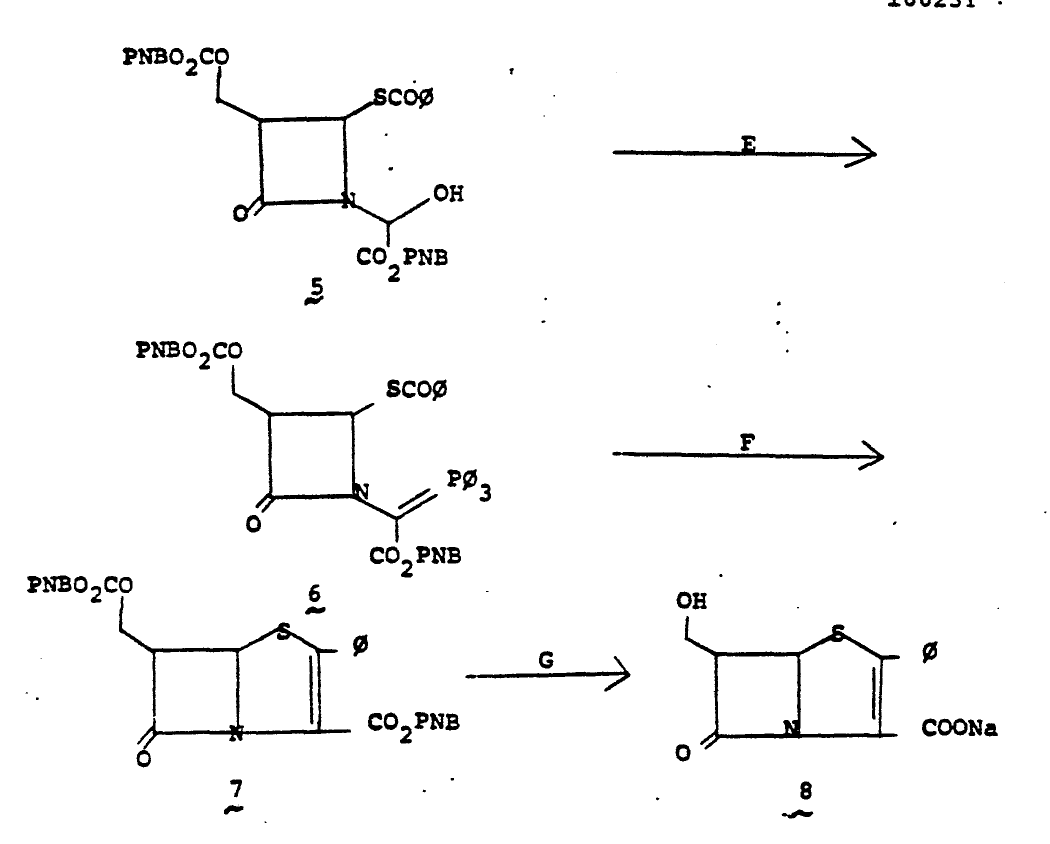

### Step A:

To a stirred solution of benzyl-6a-[l'-p -nitrobenzyloxy- carbonyloxmethyl]penicillanate 625 mg (1.25 mmoles) in 15 ml. methylene chloride at 25°C is added all at once 253.8 mg (1.25 mmoles) of 85% m-chloroperbenzoic acid. The mixture is stirred under an atmosphere of nitrogen at 25°C for 15 min. and is then partitioned between ethyl acetate and ice cold, aqueous sodium bicarbonate solution. The organic phase is separated, washed further with saturated, aqueous sodium chloride solution, dried (MgSO₄)_{'} filtered, and evaporated.

The residue is purified by plate layer chromatography (PLC)

### Step B:

A stirred mixture of 103.2 mg (0.2 mmoles) of the sulfoxide obtained in Step A, 49 mg (0.24 mmoles) of tri-n-butyphosphine, and 229 mg (1.0 mmoles) of benzoic anhydride in 3.0 ml of benzene is refluxed at 90°c under a nitrogen atmosphere for 7.0 kours. The cooled mixture is partitioned between ethyl acetate and cold, aqueous sodium bicarbonate and the organic phase is separated. The organic phase is washed with saturated, aqueous sodium chloride, dried (MgSO₄), filtered, and evaporated.Purification by PLC yields seco-lactam 3

### Step C:

To a stirred solution of 22.3 mg (0.036 mmoles) of azetidinone 3 in 0.75 ml of 8:1 acetone-water arid two drops of O.1N pH 7 phosphate buffer at 25°C is added all at once 5.7 mg (0.036 mmoles) of potassium permanganate. The mixture is stirred at 25°C under nitrogen for 8 min. and then an additional 5.7 mg of potassium permanganate is added. The mixture is stirred further for 50 min. and is then cooled on an ice-water bath, diluted with ethyl acetate, and is treated with 5% aqueous sodium thiosulfate. The mixture is filtered through celite and is washed well with ethyl acetate. The filtrate is partitioned between ethyl acetate and cold, aqueous brine . solution and the organic phase is separated, dried (MgSO₄), filtered, and evaporated. The residue is purified by PLC to provide 4

### Step D:

A stirred mixture of 43.2 mg (0.1 mmoles) of azetidinone 4 (Step C) and a large excess of p-nitrobenzylglyoxalate hydrate in 1 ml of toluene and 0.5 ml of dimethyl formamide containing 500 mg of ground 3A molecular sieves is heated at 80°C under a nitrogen atmosphere for 5.0 hr. The cooled mixture is diluted with ethyl acetate and is filtered. The filtrate is concentrated on a rotatory evaporator and the concentrate is partitioned between diethyl ether and cold water. The organic phase is separated, washed further with water, dried (MgSO₄), filtered, and evaporated. The residue is purified by PLC to give 5.

### Step E:

To a stirred solution of 6.41 mg (0.01 mmoles) of lactam 5 from Step D in 1.0 ml anhydrous tetrahydrofuran at 0°C under a nitrogen atmosphere is added in rapid succession 2.4 mg (0.02 mmoles) of neat thionyl chloride and 2.0 mg (0.02 mmoles) of neat triethyl amine. The mixture is stirred at 0°C for 2.0 hours and is then partitioned between diethyl ether and cold, aqueous brine. The organic phase is separated, washed with saturated, aqueous sodium chloride, dried (MgSO₄), filtered, and evaporated. The residue so obtained is dissolved in 0.5 ml dry tetrahydrofuran and is stirred with 10.0 mg (0.04 mmoles) of triphenylphosphine at 50°C under a nitrogen atmosphere for 18.0 hours. The cooled mixture is partitioned between ethyl acetate and aqueous sodium bicarbonate solution. The organic phase is separated, dried (Na₂SO₄), filtered and evaporated. Purification of the residue by PLC affords the phosphorane derivative 6.

### Step F:

A stirred solution of 88.5 mg (.1 mmole) of I phosphoraneazetidinone (Step E) in 80 ml of toluene is heated at 85°C under an atmosphere of nitrogen for 3.5 days. The toluene is removed under reduced perssure and the residue is purified by plate layer chromatography to provide penem 7:

### Step G:

A mixture of 60.5 mg (0.1 mmoles) of penem 7, 150 mg of 10 percent palladium-on-charcoal, and dilute, aqueous sodium bicarbonate in 5 ml of ethyl acetate is hydrogenated at 25°C and atmospheric pressure for 30 minutes. After this time 25 mg of fresh catalyst is added and the mixture is hydrogenated for an additional 5 minutes. The catalyst is removed by filtration and washed with dilute, cold aqueous sodium bicarbonate and ethyl acetate. The organic phase is separated and the aqueous phase acidified with dilute, cold aqueous citric acid. Thorough extraction of the acidified aqueous phase with methylene chloride, followed by drying (Na₂SO₄) and evaporation, provides the penem carboxylic acid 8, which is immediately converted to its sodium salt by treatment with a stoichiometric amount of aqueous sodium bicarbonate in acetone solution, evaporation of acetone, and lyopholization of the aqueous solution.

### EXAMPLE 26A

### Preparations of:

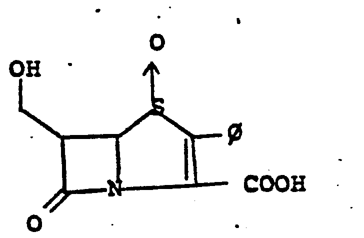

To a stirred solution of 0.1 mmoles of penem 7 from Example 26, Step F in 2 ml of methylene chloride at 25°C is added 20.3 mg (0.1 mmoles) of 85% m-chloroperbenzoic acid. The mixture is stirred at 25°C under a nitrogen atmosphere for 0.5 hours and is then partitioned between methylene chloride and cold, dilute aqueous sodium bicarbonate. The organic phase is separated, dried (Na₂SO₄), filtered and evaporated to yield the desired sulfoxide.

The above illustrated carboxylic acid is obtained as described in Step G of Example 26 when the indicated substitution is made by catalyst hydrogenation of the sulfoxide prepared above.

### EXAMPLE 27

Following the procedures set out in the foregoing Examples and text, the following species of the present invention are obtained by analogy. In the table M=methyl, and φ=phenyl.

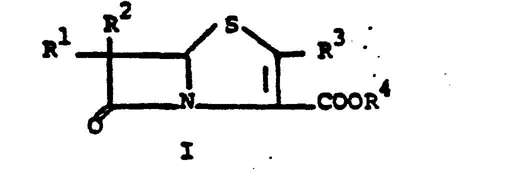

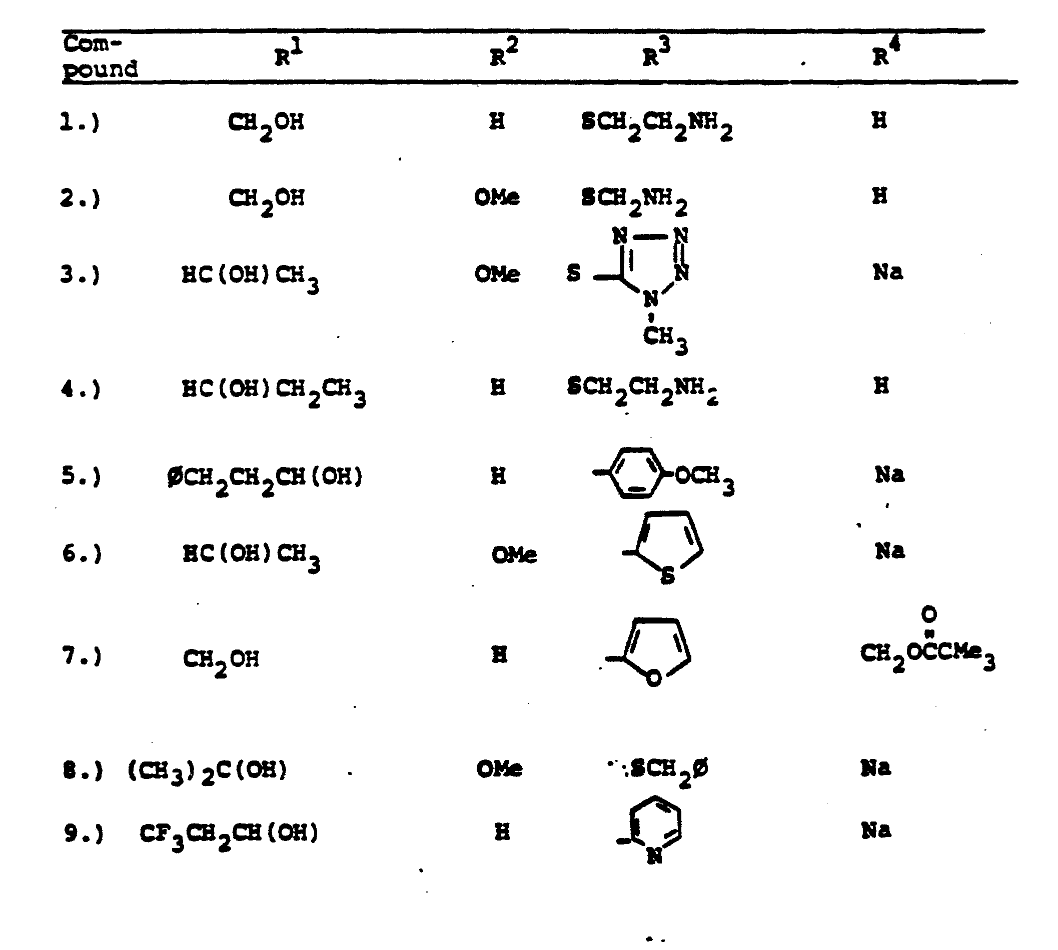

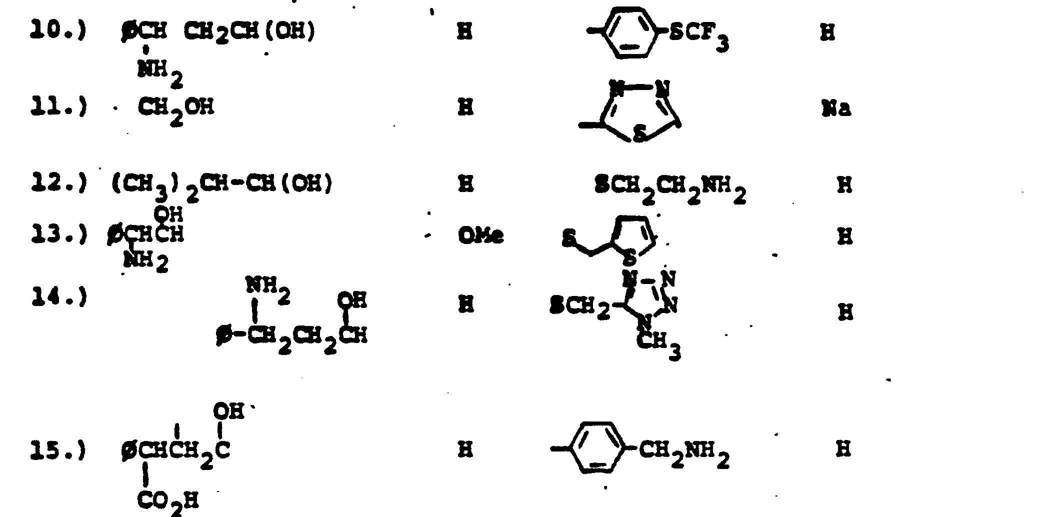

### EXAMPLE 28

### Preparation of Pharmaceutical Compositions

One such unit dosage form is prepared by mixinq 120 mg. of 6-hydroxyethyl-2-aminoethylthio-Pen-2-em-3-carboxylic acid with 20 mg. of lactose and 5 mg of mgnesium stearate and placing the 145 mg. mixture into a No. 3 gelatin capsule. Similarly, by employing more of the active ingredient and less lactose, other dosage forms can be put up in No. 3 gelatin capsulen and should it be necessary to mix more than 145 mg. of ingredients together, larger capsules such as compressed tablets and pills can also be prepared. The following examples are illustrative of the preparation of pharmaceutical farmulations:

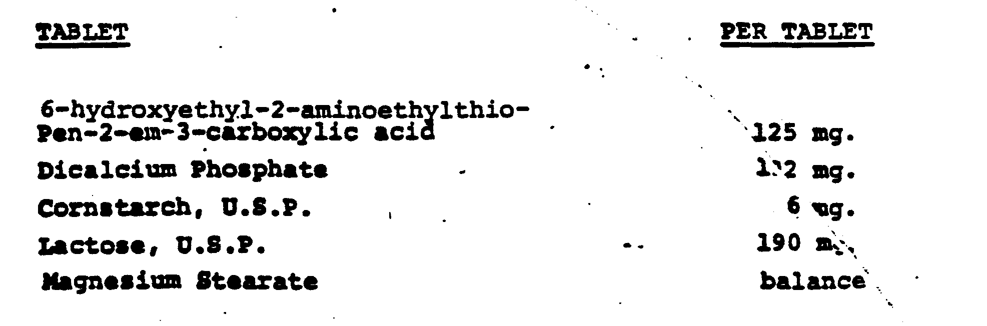

The active ingredient is blended with the dicalcium phosphate, lactose and about half of the cornstarch. The mixture is then grsnulated with 15% cornstarch paste (6 mg) and rough-screened. It is dried at 45°C. and screened again through No. 16 screens. The balance of the cornstarch and the magnesium stearate is added and the mixture is compressed into tablets, approximately 0.5 inch in diameter each weighing 800 mg.

### PARENTERAL SOLUTION

### Ampoule:

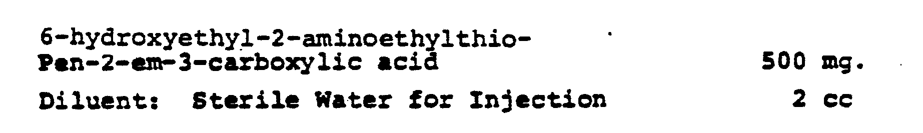

### OPHTHALMIC SOLUTION

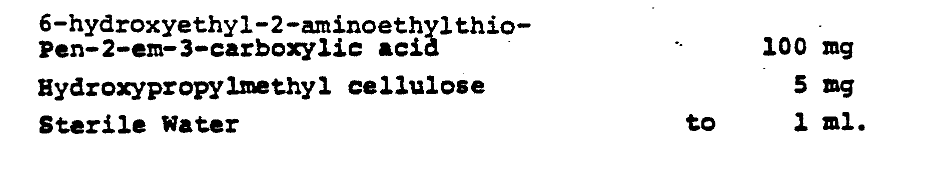

### OTIC SOLUTION

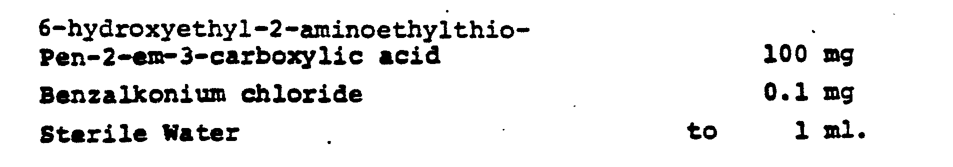

### TOPICAL OINTMENT

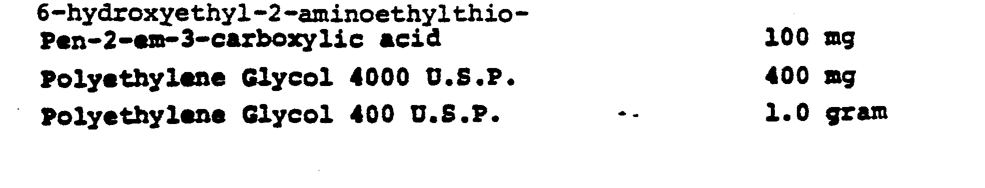

The active ingredient in the above formulations may be administered alone or in combination with other biologically active ingredients as, for example, with other antibacterial agents such as lincomycin, a penicillin, streptomycin, novobiocin, gentamicin, neomycin, colistin and kanamycin, or with other therapeutic agents such as probenecid.

## Claims

1. A compound having the structural formula: 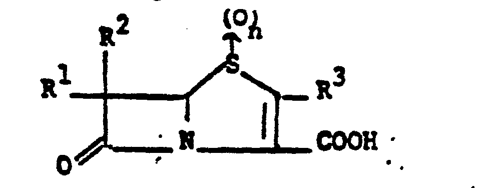
and the pharmaceutically acceptable salts and esters thereof, wherein: n-O or 1;
R¹ and R ² are independsntly selected from the group consisting of hydrogen, -NHR¹ (R^{1'} = H or acyl), substituted and unsubstituted: alkyl, alkenyl, and alkynyl, having from 1-10 carbon atoms; cycloalkyl, cycloalkylalkyl, and alkylcycloalkyl, having 3-4 carbon atoms in the cycloalkyl ring and 1-6 carbon atoms in the alkyl moieties; phenyl; aralkyl, aralkenyl, and aralkynyl wherein the aryl moiety is phenyl and the chain has 1-6 carbon atoms; heferoaryl, heteroaralkyl, heterocyclyl and heterocyclylalkyl wherein the substituent or substituents relative to the above-named radicals are selected from the group consisting of: amino, mono, di-and trialkylamino, hydroxyl, alkoxyl, mercapto, alkylthio, phenylthio, sulfamoyl, amidino, guanidino, nitro, chloro, bromo, fluoro, cyano and carboxy, and wherein the hetero atom or atoms in the ebove-named heterocyclic moieties are selected from the group consisting of 1-4 oxygen, nitrogen or sulphur atoms; and wherein the alkyl moieties of the above-recited snbstitusnts have 1-6 carbon atom; and wherein
_{R}³ is hydrogen; substituted and unsubstituted: -R, -OR, -SR, -NR₂(when n=1, R³ is not -SR); and
R is alkyl having 1-6 carbon atoms, phenyl, phenalkyl having 7-12 carbon atoms, heterocyclyl and heterocyclylelkyl wherein the alkyl has 1-3 carbon atons and the heterocycle has 1-4 hetero atoms selected from O, N and S; and wherein the chain or nuclear substituent on R is selected from amino, mono-, di- and trialkylamino (each alkyl having 1-6 carbon atoms), hydroxyl, alkoxyl, mercapto, alkylthio having 1-3 carbon atoms, phenylthio, sulfamoyl, amidino, guanidino, nitro, chloro, bromo, fluoro, cyano, and carboxyl.

2. A compound according to Claim 1, wherein:
R¹ and R² are independently selected from: hydrogen, hydroxymethyl, 1-hydroxyethyl, 1-hydroxy-3-phenyl-propyl, 1-hydroxy-3-phenyl-3-carboxypropyl, 1-hydroxy-2,2,2-trifluoroethyl, methoxyl, phenyl, methyl, 2-thienyl, 2-pyridyl, benzyl, p-methoxyphenyl; and
R³ is

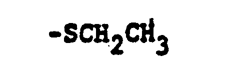

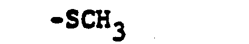

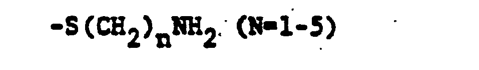

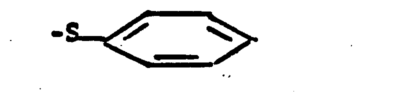

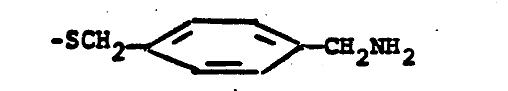

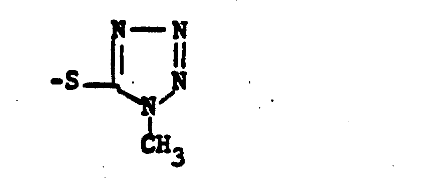

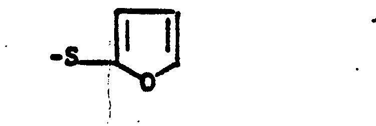

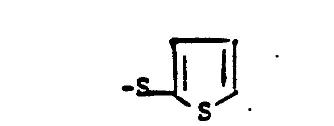

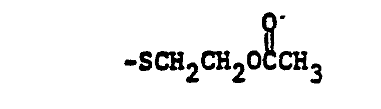

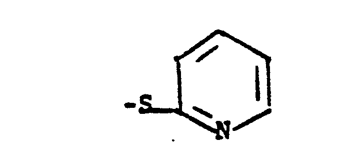

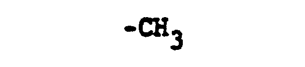

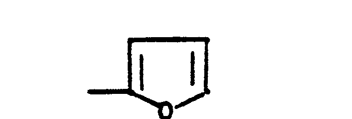

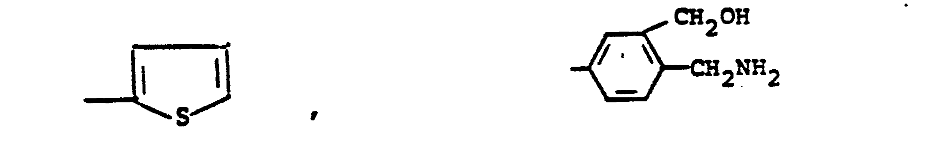

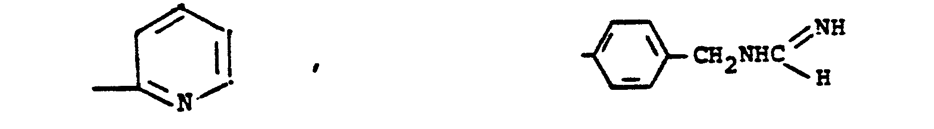

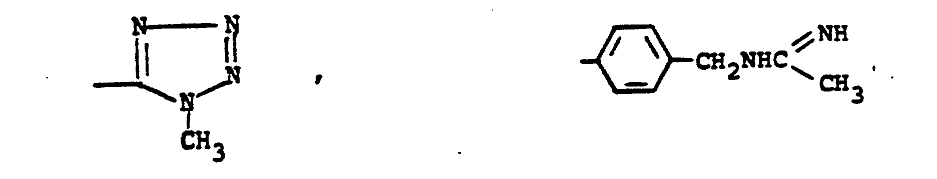

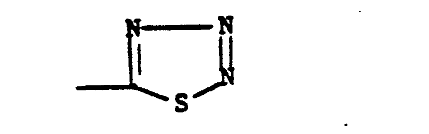

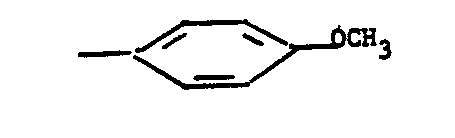

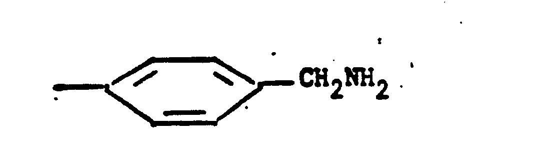

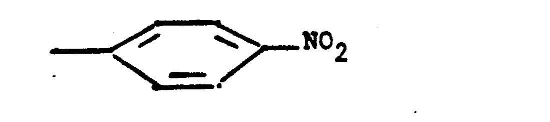

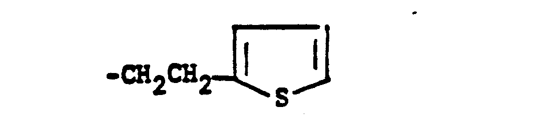

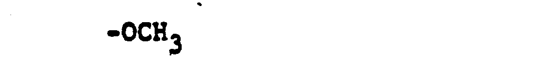

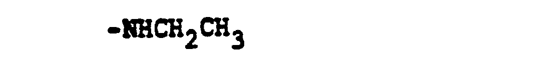

3. A compound according to Claim 2 wherein R² is hydrogen.

4. A process for preparing a compound according to Claim 1 comprising cycliziag: 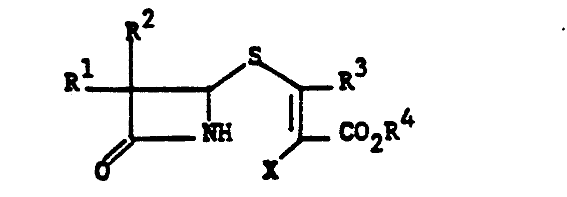 in the presence of base; wherein R is a protecting group or a pharmaceutically acceptable ester moiety and X is halo.

5. A process for preparing a compound according to Claim 1 comprising cyclizing: 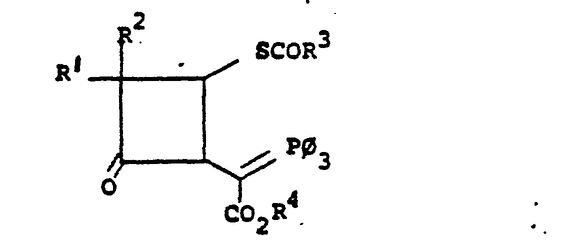 by heading; wherein R is a protecting group or a pharmaceutically acceptable ester moiety; φ is phenyl; and R³ is H or _{R}.

6. A pharmaceutical composition comprising a compound according to Claim 1 and a pharmaceutically acceptable carrier therefor.

7. A compound according to claim 1 having the structural formula: 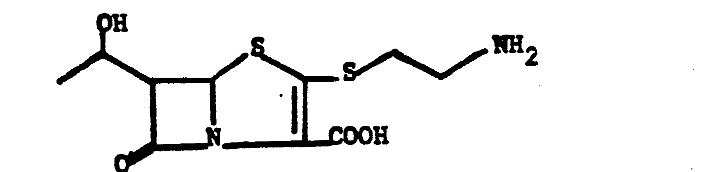 and the pharmaceutically acceptable salts and esters thereof.

8. A process for preparing a compound according to Claim 1 comprising cyclizing 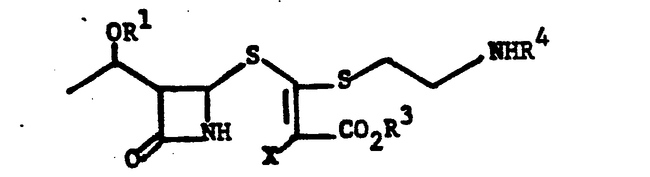 in the presence of base; wherein R and R are protecting groups and R³ is a protecting group or pharmaceutically acceptable ester moiety.

9. A compound according to Claim 1 wherein: n=0; R¹ is phenylacetyl, phenoxyacetyl,3-broaoophsnylacetyl, p-aminomethylphenylacetyl, 4-carboxylmethylphenylacetyl, 4-car- boxamidomethylphenylacetyl, 2-furylacetyl, 5-nitrofuryl- acetyl, 3-furylacetyl, 2-thisnylacstyl, 5-chlorothienyl-- acetyl, 5-methoxythienylacetyl, α-guanidino-2-thienylacetyl, 3-thienylacetyl, 4-methylthienylacetyl, 3-isothia- solylacetyl, 4-methoxyiaothiazolylacetyl, 4-isothiazolyl- acetyl, 3-methylisothiazolylacetyl, 5-isothiasolylacetyl, 3-chloroisothiasolylacstyl, 3-methyl-1,2,5-oxadiazolylace- tyl, 1,2,5-thiadiazolyl-4-aoetyl, 3-asthyl-1,2,5-thiadia solyl-4-acstyl, 3-cbloro-1,2,5-thiadiasolyl-4-acetyl, 3-methoxy-1,2,5-thiadiazolyl-4-acetyl, phenylthioacetyl, 4-pyridylthioacetyl, cyanoacetyl, tetrazolylacetyl, a-fluorophenylacetyl, D-phenylqlycyl, 3-hydroxy-D-phenylglycyl, 2-thienylglycyl, 3-thienylglycyl, phenylmalonyl, 3-chloro- phenylmalonyl, 2-thienylmalonyl, 3-thienylmalonyl, a-phosphonophenylacetyl, a-sulfaminophenylacetyl, a-hydroxyphenylacetyl, a-tetrazolylphenylacetyl and α-sulfophenyl- acetyl; and wherein _{R}³ is:

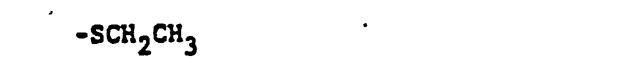

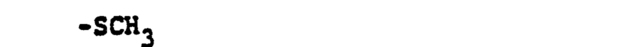

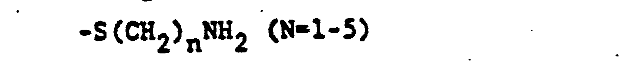

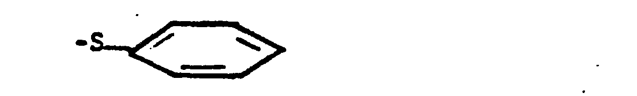

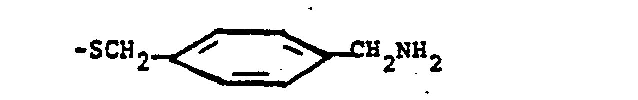

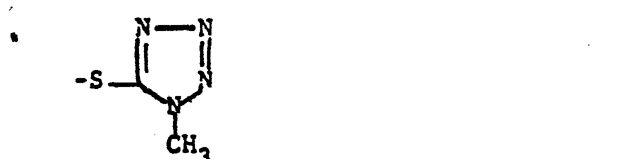

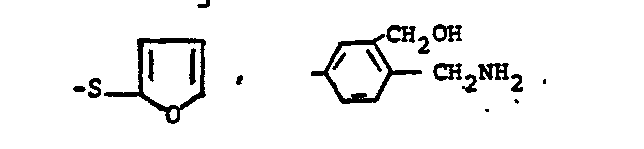

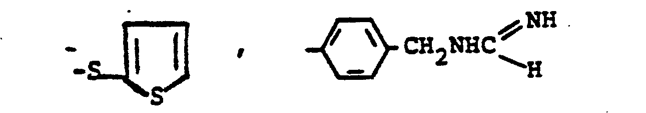

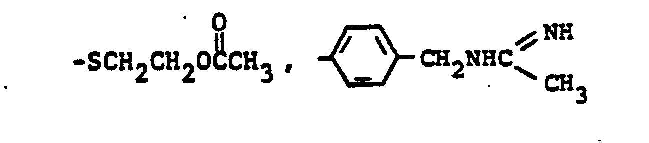

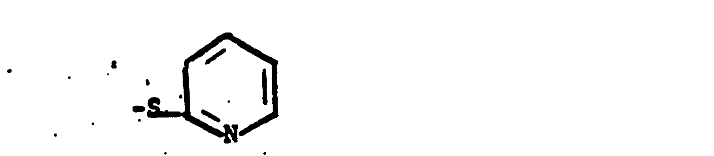

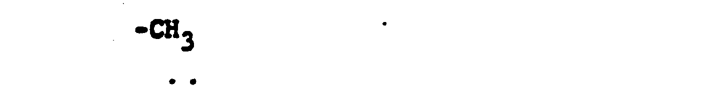

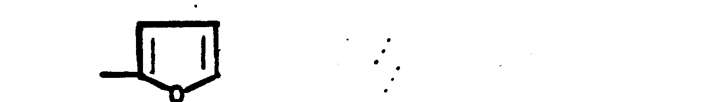

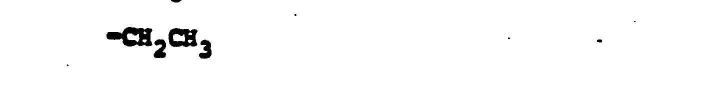

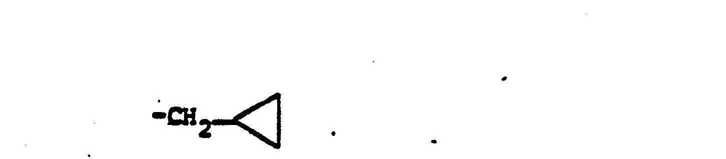

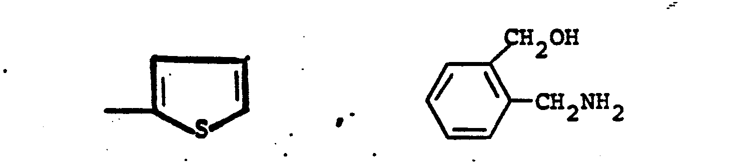

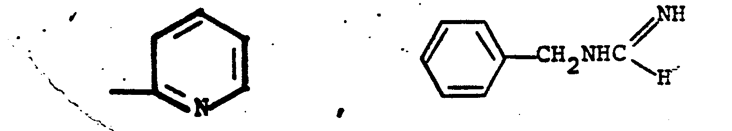

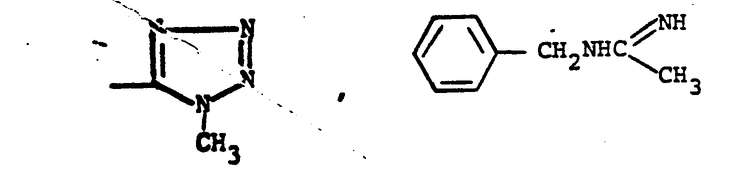

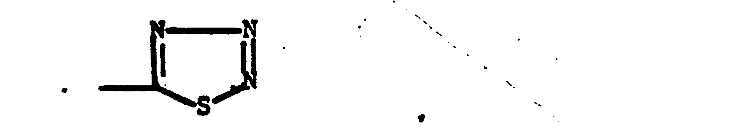

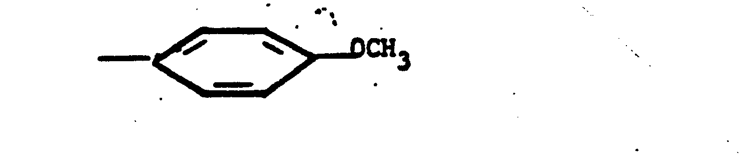

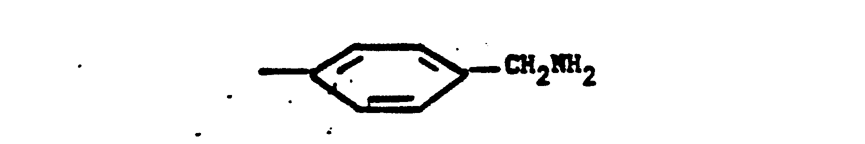

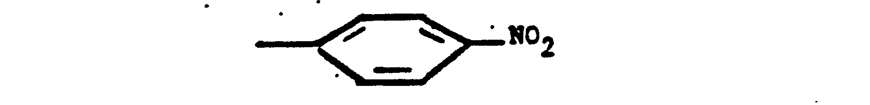

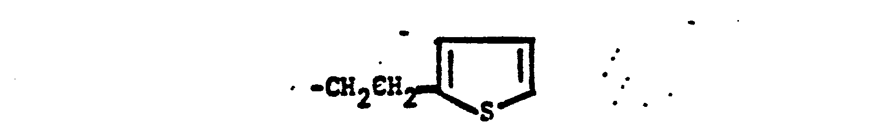

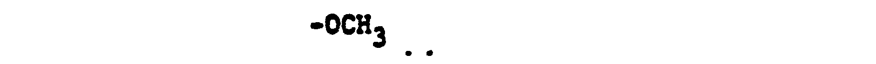

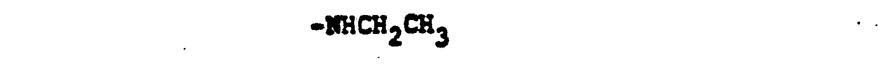

10.A compound according to Claim 9 wherein R³ is :
